(19) Europäisches Patentamt / European Patent Office / Office européen des brevets

(11) EP 2 116 527 A1

(12) EUROPEAN PATENT APPLICATION
published in accordance with Art. 153(4) EPC

(43) Date of publication:
**11.11.2009 Bulletin 2009/46**

(21) Application number: **07791783.9**

(22) Date of filing: **01.08.2007**

(51) Int Cl.:
*C07C 251/66* (2006.01) *C07D 307/82* (2006.01)
*C07D 307/92* (2006.01) *C07D 311/92* (2006.01)
*C07D 333/66* (2006.01) *C07D 335/06* (2006.01)
*G03F 7/031* (2006.01)

(86) International application number:
**PCT/JP2007/065103**

(87) International publication number:
**WO 2008/090640 (31.07.2008 Gazette 2008/31)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **23.01.2007 JP 2007013117**
**29.03.2007 JP 2007088814**

(71) Applicant: **FUJIFILM Corporation**
**Minato-ku**
**Tokyo 106-8620 (JP)**

(72) Inventors:
- **FUJITA, Akinori**
**Fujinomiya-shi**
**Shizuoka 418-8666 (JP)**
- **TAMURA, Takashi**
**Ashigarakami-gun**
**Kanagawa 258-8538 (JP)**
- **IKEDA, Kimi**
**Fujinomiya-shi**
**Shizuoka 418-8666 (JP)**
- **KASHIWAGI, Daisuke**
**Fujinomiya-shi**
**Shizuoka 418-8666 (JP)**

(74) Representative: **HOFFMANN EITLE**
**Patent- und Rechtsanwälte**
**Arabellastrasse 4**
**81925 München (DE)**

# (54) OXIME COMPOUND, PHOTOSENSITIVE COMPOSITION, COLOR FILTER, METHOD FOR PRODUCTION OF THE COLOR FILTER, AND LIQUID CRYSTAL DISPLAY ELEMENT

(57) Provided are an oxime compound represented by General Formula (1), a photosensitive composition containing the oxime compound as a photopolymerization initiator, a production method for a color filter using the photosensitive composition, and a color filter obtained by the production method:

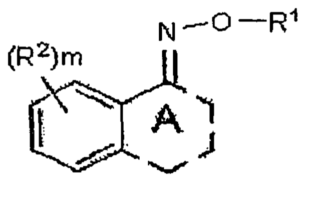

General Formula (1)

in General Formula (1), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group; m is an integer of 0 to 4; when m is an integer of 2 or more, $R^2$s may be linked together to form a ring; and A represents a 4-, 5-, 6- or 7-membered ring.

EP 2 116 527 A1

## Description

### Technical Field

The present invention relates to an oxime compound; a photosensitive composition containing the oxime compound as a photopolymerization initiator; a production method for a color filter using the photosensitive composition; a color filter obtained by the production method; and a liquid crystal display element which contains the color filter.

### Background Art

Conventionally, some oxime ester derivatives are known to serve as a photopolymerization initiator, which generates an active radical by the action of light to initiate polymerization of monomers (see Patent Literatures 1 to 4 and 8).
The recent photopolymerization techniques have increasingly required such a photopolymerization initiator that exhibits high photopolymerizing reactivity and allows easy handling. Furthermore, in order to obtain high-quality products, increased demand has arisen for a photopolymerization initiator which exhibits high sensitivity as well as which causes no reaction by light and/or heat during storage; i.e., which is excellent in other properties such as storage stability and heat stability. Also, exposing light sources have advanced and exposing can be performed at various wavelengths. Thus, there is a need to obtain a photosensitive composition which exhibits high polymerization sensitivity to lights with various wavelengths, which causes no reaction during storage; i.e., is excellent in storage stability. In addition, there is a need to obtain a photopolymerization initiator having higher sensitivity than that of any one of the compounds disclosed in Patent Literatures 1 to 4.
Meanwhile, as electronic devices have recently become downsized and highly-functional, more and more requirements are placed on photocurable resin compositions to be used in such electronic devices. For example, improvement in reliability, formation of high-density circuit pattern, and improvement in pattern accuracy are required for solder resist using a photocurable resin composition, interlayer insulating material, plating resist, anti-sandblasting ink, polymer optical guide path, members for flat panel display (FPD), coating protective film, protective film for color filter, insulating spacer (touch panel).
The photocurable resin composition is generally cured with an ultraviolet (UV) light source, and is widely used for anastatic printing, relief image formation and phtoresist. Such photocurable resin composition contains an organic component which mainly forms an image, and a photopolymerization initiator which changes to a polymerization active species upon irradiation of light such as UV rays.
Also, in order to improve pattern accuracy while considering the recent environmental problems, there is widely employed a photolithgraphic method in which a pattern is formed from an alkali-developable photocurable resin composition through exposure and development. One such alkali-developable photocurable resin composition is a composition containing, as a base polymer, a photosensitive prepolymer which is produced by adding a polybasic acid anhydride to a product obtained between a novolac type epoxy resin and an unsaturated monocarboxylic acid (see Patent Literatures 5 to 7).
Furthermore, in recent years, as a photolithgraphic method which is sensitive to environmental problems such as saving of resources and energy, laser direct imaging is put into practice which uses laser light as a light source. Laser direct imaging is suitable for production of high-multilayer substrates of various kinds with quick delivery and small lot, since a production step of a mask is omitted and scaling can be performed on individual substrates.
The light source and the wavelength of the light used in laser direct imaging depend on the application of the photocurable resin composition. As a light source, some cases employ a gas ion laser which generally uses argon gas, and other cases employ a solid state laser which generally uses a semiconductor or YAG laser. Also, the light source is classified in terms of the wavelength of laser beam into those emitting beams of the ultraviolet region and those emitting beams of the visible region. In general, a laser beam of 365 nm, 405 nm or 488 nm is employed.
The wavelength of the laser is changing from the visible region to the ultraviolet region, in consideration of the working environment and the cost of resist. The light source is changing from the gas laser to the solid state laser in terms of running cost. The semiconductor laser, one of the solid state lasers, is widely used in the fields of communication and photoelectronics. In particular, this laser has advantageous features as compared with the other lasers, in terms of downsizing, high efficiency, low voltage, low power consumption and long service life and thus, is used as a light source for laser direct imaging.
However, existing photosensitive compositions, used in pattern formation for resist, insulating material, color filter, etc. in electronic materials, are still not satisfactory in terms of sensitivity during curing and storage stability of the sensitizer.
Thus, at present, there has not been provided a satisfactory, high sensitive photosensitive composition whose polymerizable compounds can be efficiently polymerized in short time, which is excellent in storage stability and allows easy handling, and which contains, as an essential photopolymerization initiator (radical generator), a highly sensitive,

novel compound being excellent in storage stability and generating active radicals upon irradiation of light to initiate polymerization reaction.

Meanwhile, a color filter is an essential constituent part of a liquid crystal panel. The liquid crystal panel is compact-sized as compared to CRTs and has performance equivalent to or exceeding them. Thus, the liquid crystal panel is replacing CRTs as TV screens, PC screens or other display devices.

In recent years, liquid crystal display devices have increasingly become larger in screen size, and been required to be reduced in cost and increased in image quality.

In order to reduce the cost for the liquid crystal display device, the constituent components (e.g., a color filter and a glass substrate) are required to be reduced in cost. When the cost for the color filter is reduced, it is necessary to reduce the coating amount of the photosensitive composition.

However, when the photosensitive composition is applied onto a large substrate or when the coating amount of the coating liquid is reduced, problems arise that uneven coating occurs. In order to overcome such uneven coating, the coating liquid must be increased in flowability, preventing uneven coating even using a small amount of the coating liquid.

In general, means for increasing the flowability of the coating liquid is decreasing the viscosity thereof by decreasing the molecular weight of a polymer, and increasing dissolution capability of a polymer in a solvent. But, simply by decreasing the viscosity of the coating liquid, uneven coating is likely to occur in the substrate between the center portion and peripheral portions.

Also, the photosensitive composition is required to have a wide range of appropriate developing time in development after exposure; i.e., a wide development latitude (excellent suitability for various processes). When the substrate has a large surface, the difference in developing time occurs since the time for which its top portion is immersed in a developer is considerably different from that for which its end portion is done. This causes, for example, an unfavorable phenomenon in which the formed patterns are different in size from one another due to increasing of uneven development.

Conventionally, spin coating is advantageously used for applying a photosensitive composition onto a substrate, since it can uniformly form a 1 μm- to 3 μm-thick thin film with high accuracy. Thus, spin coating is widely used for production of a color filter.

In recent years, in order to further increase production efficiency and reduce production cost in consideration of mass production of large-scale crystal display devices, slit coating is employed for production of a color filter. The slit coating is suitable for applying a coating liquid onto a wide, large substrate.

Slit coating is a coating method in which a coating liquid is applied onto a substrate from slits in a predetermined discharge amount, using a coating apparatus which has a coating head having a several tens micrometer-wide slit (space) in the tip portion and having a length corresponding to the coating width of a rectangular substrate, while the coating head is being moved relatively to the substrate at a certain speed with the clearance (space) therebetween being maintained to be several tens micrometers to several hundreds micrometers.

Slit coating is employed to form a much larger as compared with spin coating. Thus, when the coating liquid is discharged from its elongated slit, the difference in speed must be maintained in some degree between the coater and an object to be coated. The coating liquid used in slit coating, therefore, is required to have good flowability. Also, the various conditions under which the coating liquid is discharged from the slit onto the substrate must be maintained unchanged during coating over the entire coating width. When the coating liquid is insufficient in liquid properties such as flowability and viscoelasticity, uneven coating is likely to occur. As a result, it is difficult to maintain the thickness of the coated product constant in the coating width direction, whereby a uniform coated film cannot be obtained.

In view of this, various attempts have been made to improve the coating liquid in flowability and viscoelasticity, in order to obtain a uniform coated film without unevenness.

For example, the resin component of the photosensitive composition is improved. As such improvement, for example, there have been proposed methods in which a copolymer containing, as a monomer component, (meth)acrylic acid, (meth)acrylate having an ethylene oxide molecular chain, and a styrene derivative is used as a binder for dispersing a pigment (see, for example, Patent Literatures 9 to 12).

However, the above methods cannot sufficiently solve the above existing problems. In particular, when slit coating is employed, the coating liquid is not satisfactory in liquid properties such as flowability and viscoelasticity.

Patent Literature 1: Japanese Patent Application Laid-Open (JP-A) No. 60-166306
Patent Literature 2: JP-A No. 2001-233842
Patent Literature 3: JP-A No. 2000-80068
Patent Literature 4: JP-A No. 2005-182004
Patent Literature 5: JP-A No. 61-243869
Patent Literature 6: JP-A No. 07-50473
Patent Literature 7: Japanese Patent Application Publication (JP-B) No. 07-17737
Patent Literature 8: JP-A No. 2006-36750

Patent Literature 9: Japanese Patent (JP-B) No. 2665696
Patent Literature 10: JP-A No. 2000-194132
Patent Literature 11: JP-A No. 08-179120
Patent Literature 12: JP-A No. 07-294726

Disclosure of Invention

A first object of the present invention is to provide a novel oxime compound which is excellent in storage stability and sensitivity and which is contained in a polymerizable composition to enable it to be excellent in storage stability without causing polymerization reaction during storage and to be a highly sensitive polymerizable composition by generating active radicals upon irradiation of energy beam, especially, light, to thereby efficiently and quickly allow the polymerizable compounds to be polymerized; and a high sensitive photosensitive composition whose polymerizable compounds can be efficiently polymerized in short time and which is excellent in storage stability and allows easy handling, by using the oxime compound as a photopolymerization initiator.

A second object of the present invention is to provide a photosensitive composition which has a wide development latitude, which can be suitably used as a coating liquid for slit coating attaining easy coating on a large substrate, and which is excellent in coating properties such as uniformity of a coated product; a color filter using the photosensitive composition; a production method for the color filter; and a liquid crystal display element.

Means for solving the problems pertinent in the art are as follows.

< 1 > An oxime compound represented by General Formula (1):

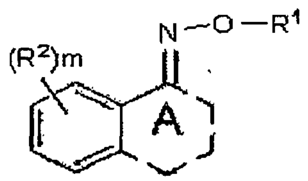

General Formula (1)

in General Formula (1), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group, m is an integer of 0 to 4; when m is an integer of 2 or more, $R^2$s may be linked together to form a ring; and A represents a 4-, 5-, 6- or 7-membered ring.

< 2 > The oxime compound according to < 1 > above, wherein the oxime compound is represented by General Formula (2):

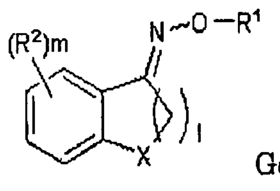

General Formula (2)

in General Formula (2), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group; m is an integer of 0 to 4; X represents $CH_2$, O or S; and l is an integer of 1 to 3.

< 3 > The oxime compound according to < 1 > above, wherein the oxime compound is represented by General Formulas (3) or (4):

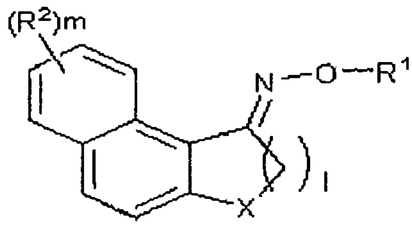

General Formula (3)

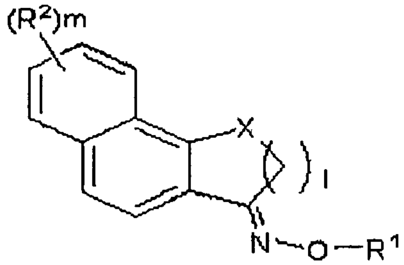

General Formula (4)

in General Formula (3) or (4), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group; m is an integer of 0 to 4; X represents $CH_2$, O or S; and l is an integer of 1 to 3.

< 4 > The oxime compound according to any one of < 2 > and < 3 > above, wherein X is O or S, l is an integer of 1 or 2, and $R^1$ is an acyl group or an alkoxycarbonyl group, each of which may have a substituent.

< 5 > A photosensitive composition including:

at least one ethylenically unsaturated photopolymerizable compound, and
at least one of the oxime compounds according to any one of < 1 > to < 4 > above, each serving as a photopolymerization initiator.

< 6 > A photosensitive composition including:

(A) a photopolymerization initiator,
(B) an ethylenically unsaturated compound,
(C) a binder,
(D) a coloring material, and
(E) a solvent,

wherein the photopolymerization initiator is an oxime compound represented by General Formula (A-1), and
wherein the concentration of solid matter contained in the photosensitive composition is 2.5% by mass to 15% by mass:

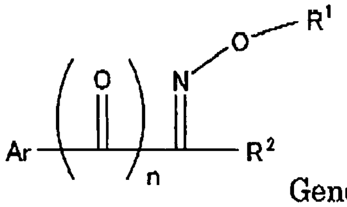

General Formula (A-1)

in General Formula (A-1), $R^1$ represents an acyl group, an alkyloxycarbonyl group or an aryloxylcarbonyl group; $R^2$ represents a hydrogen atom, an alkyl group or a cyano group; Ar represents an aromatic or heteroaromatic ring; Ar and $R^2$ may be linked together to form a ring; and n is an integer of 0 or 1.

< 7 > A photosensitive composition including:

(A) a photopolymerization initiator,
(B) an ethylenically unsaturated compound,
(C) a binder,
(D) a coloring material, and
(E) a solvent,

wherein the photopolymerization initiator is the oxime compound according to any one of < 1 > to < 4 > above, and wherein the concentration of solid matter contained in the photosensitive composition is 2.5% by mass to 15% by mass.

< 8 > The photosensitive composition according to < 6 > above, wherein the oxime compound represented by General Formula (A-1) is a compound represented by General Formula (A-2):

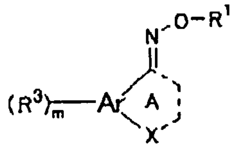

General Formula (A-2)

in General Formula (A-2), $R^1$ represents an acyl group, an alkyloxycarbonyl group or an aryloxylcarbonyl group, each of which may have a substituent: $R^3$ represents an alkyl group, an alkyloxy group, an aryloxy group, an alkylthio group, an arylthio group, a halogen atom, an alkyloxycarbonyl group, a nitro group or an acylamino group; m is an integer of 0 or more; when m is an integer of 2 or more, $R^3$s may be identical or different and may be linked together to form a ring; Ar represents an aromatic or heteroaromatic ring; A represents a 4-, 5- or 6-membered ring, each of which may have a substituent: and X represents an oxygen atom or a sulfur atom.

< 9 > The photosensitive composition according to < 8 > above, wherein the oxime compound represented by General Formula (A-2) is a compound represented by General Formula (A-3) or (A-4):

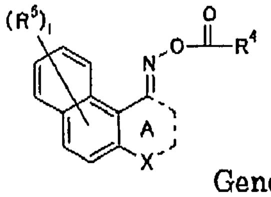

General Formula (A-3)

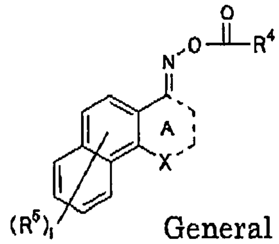

General Formula (A-4)

in General Formula (A-3) or (A-4), $R^4$ represents an alkyl group or an alkyloxy group, each of which may have a substituent; $R^5$ represents an alkyl group, an alkyloxy group, an aryloxy group, an alkylthio group, an arylthio group, a halogen atom, an alkyloxycarbonyl group, a nitro group or an acylamino group; l is an integer of 0 to 6; when l is an integer of 2 or more, $R^5$s may be identical or different and may be linked together to form a ring; A represents a 5- or 6-membered ring, each of which may have a substituent; and X represents an oxygen atom or a sulfur atom.

< 10 > The photosensitive composition according to any one of < 6 > to < 9 > above, further including a sensitizer represented by General Formula (F-1):

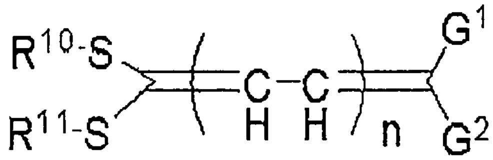

General Formula (F-1)

in General Formula (F-1), $R^{10}$ and $R^{11}$, which may be identical or different, each represent an alkyl group, an aryl group or an alkenyl group, each of which may have a substituent; $R^{10}$ and $R^{11}$ may form, together with sulfur atoms to which $R^{10}$ and $R^{11}$ are bonded, a ring composed of non-metal elements; n is an integer of 0, 1 or 2; $G^1$ and $G^2$, which may be identical or different, each represent an alkoxycarbonyl group, an aryloxylcarbonyl group, an acyl group, an arylcarbonyl group, an alkylthio group, an arylthio group, an alkylsulfonyl group, an arylsulfonyl group, a fluoroalkylsulfonyl group, each of which may have a substituent, or each represent a hydrogen atom or a cyano group; one of $G^1$ and $G^2$ is an alkoxycarbonyl group, an aryloxylcarbonyl group, an acyl group, an arylcarbonyl group, an alkylsulfonyl group, an arylsulfonyl group, a fluoroalkylsulfonyl group, each of which may have a substituent, or is a cyano group; and $G^1$ and $G^2$ may form, together with carbon atoms to which $G^1$ and $G^2$ are bonded, a ring composed of non-metal atoms.

< 11 > The photosensitive composition according to < 10 > above, wherein the sensitizer represented by General Formula (F-1) is a compound represented by General Formula (F-2):

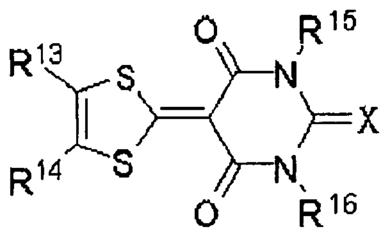

General Formula (F-2)

in General Formula (F-2), $R^{13}$ and $R^{14}$, which may be identical or different, each represent a hydrogen atom, an alkyl group, an aryl group, an acyl group or a halogen atom; $R^{13}$ and $R^{14}$ may be linked together to form an aromatic ring which may have an alkyl group, an alkyloxy group or a halogen atom; $R^{15}$ and $R^{16}$, which may be identical or different, each represent a hydrogen atom, an alkyl group or an aryl group; and X represents an oxygen atom or a sulfur atom.

< 12 > A production method for a color filter, the method including:

forming a photosensitive layer by applying the photosensitive composition according to any one of < 6 > to < 11 > above onto a substrate and by drying the applied photosensitive composition,
patternwise exposing and curing the photosensitive layer so as to have a desired pattern, and
developing the exposed photosensitive layer to remove uncured portions.

< 13 > The production method according to < 12 > above, wherein the patternwise exposing and curing is forming a two-dimensional image through scanning of the photosensitive layer by moving light having a wavelength of 350 nm to 420 nm relatively to the photosensitive layer while modifying the light based on image data, using two dimensionally arranged spatial light modulation devices.

< 14 > The production method according to any one of < 12 > and < 13 > above, wherein the substrate has a size of 1 $m^2$ or greater.

< 15 > A color filter obtained by the production method according to any one of < 12 > to < 14 > above.

< 16 > A liquid crystal display element including:

the color filter according to < 15 > above as at least one member.

The present invention provides a high-sensitive photosensitive composition, as a first embodiment, whose polymerizable compounds can be efficiently polymerized in short time and which is excellent in storage stability and allows easy handling by using, as a photopolymerization initiator, a novel oxime compound which is excellent in storage stability and sensitivity and which is contained in a polymerizable composition to enable it to be excellent in storage stability without causing polymerization reaction during storage and to be a highly sensitive polymerizable composition by generating active radicals upon irradiation of energy beam, especially, light, to thereby efficiently and quickly allow the polymerizable compounds to be polymerized. This photosensitive composition can solve the problems pertinent in the art.

The present invention provides a photosensitive composition, as a second embodiment, which has a wide development latitude, which can be suitably used as a coating liquid for slit coating attaining easy coating on a large substrate, and which is excellent in coating properties such as uniformity of a coated product; a color filter using the photosensitive composition; a production method for the color filter; and a liquid crystal display element.

Best Mode for Carrying Out the Invention

(Oxime compound)

An oxime compound of the present invention is represented by General Formula (1):

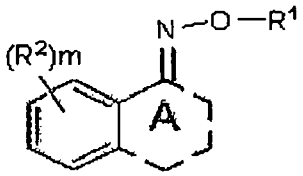

General Formula (1)

in General Formula (1), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group; m is an integer of 0 to 4; when m is an integer of 2 or more, $R^2$s may be linked together to form a ring; and A represents a 4-, 5-, 6- or 7-membered ring.

The oxime compound of the present invention is excellent in storage stability and sensitivity. Thus, incorporation of the oxime compound can provide a high-sensitive polymerizable composition whose polymerizable compounds can be efficiently polymerized in short time, which is excellent in storage stability without causing polymerization reaction, and which allows easy handling, since the oxime compound contained therein generates active radicals upon irradiation of energy beam, especially, light, to thereby efficiently and quickly allow the polymerizable compounds to be polymerized.

Also, the oxime compound is more preferably an oxime compound represented by General Formula (2):

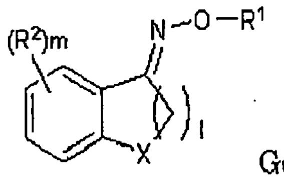

General Formula (2)

in General Formula (2), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group; m is an integer of 0 to 4; X represents $CH_2$, O or S; and l is an integer of 1 to 3.

Also, the oxime compound is more preferably an oxime compound represented by General Formula (3) or (4):

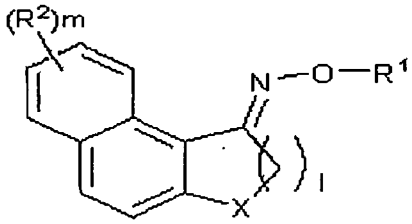

General Formula (3)

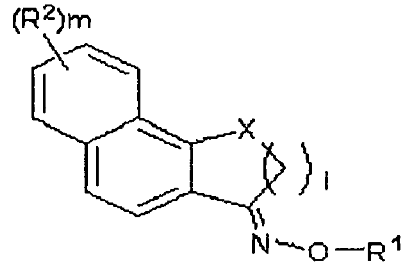

General Formula (4)

in General Formula (3) or (4), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group; m is an integer of 0 to 4; X represents $CH_2$, O or S; and l is an integer of 1 to 3.

The oxime compounds represented by General Formulas (2) to (4) are preferably those represented by General Formula (2) to (4) in which X is O or S, l is an integer of 1 or 2, and $R^1$ is an acyl group or an alkoxycarbonyl group, each of which may have a substituent.

In General Formulas (1), (2), (3) and (4), $R^1$ represents a hydrogen atom, an acyl group, an alkoxycarbonyl group or an aryloxylcarbonyl group, each of which may have a substituent.

The acyl group may be aliphatic, aromatic or heterocyclic. The total number of carbon atoms contained therein is preferably 2 to 30, more preferably 2 to 20, particularly preferably 2 to 16. Further, the acyl group may have a substituent. The substituent is preferably an alkoxy group, an aryloxy group or a halogen atom.

Examples of the acyl group which may have a substituent include acetyl, n-propanoyl, i-propanoyl, methylpropanoyl, butanoyl, pivaloyl, hexanoyl, cyclohexanecarbonyl, octanoyl, decanoyl, dodecanoyl, octadecanoyl, benzylcarbonyl, phenoxyacetyl, 2-ethylhexanoyl, chloroacetyl, benzoyl, toluenecarbonyl, paramethoxybenzoyl, 2,5-dibutoxybenzoyl, 1-naphthoyl, 2-naphthoyl, pyridylcarbonyl, methacryloyl and acryloyl.

The alkoxycarbonyl group may have a substituent, and the total number of carbon atoms contained therein is preferably 2 to 30, more preferably 2 to 20, particularly preferably 2 to 16. Examples of the alkoxycarbonyl group include methoxycarbonyl, ethoxycarbonyl, isopropoxycarbonyl, butoxylcarbonyl, isobutyloxycarbonyl, allyloxycarbonyl, octyloxycarbonyl, dodecyoxycarbonyl and ethoxyethoxycarbonyl.

The aryloxylcarbonyl group may have a substituent, and the total number of carbon atoms contained therein is preferably 7 to 30, more preferably 7 to 20, particularly preferably 7 to 16. Examples of the aryloxylcarbonyl group include phenoxycarbonyl, 2-naphthoxycarbonyl, paraphenoxycarbonyl, 2,5-diethoxyphenoxycarbonyl, parachlorophenoxycarbonyl, paranitrophenoxycarbonyl and paracyanophenoxycarbonyl.

In each of General Formulas (1), (2), (3) and (4), $R^2$ represents or $R^2$s each represent a halogen atom, an alkyl group, an aryl group, an alkyloxy group, an aryloxy group, an alkylthio group, an arylthio group or an amino group. In particular, $R^2$ represents an aliphatic group, an aromatic group, a heteroaromatic group, a halogen atom, $-OR^3$, $-SR^3$ or $-NR^3R^4$. $R^3$ and $R^4$ may be linked together to form a ring. $R^3$ and $R^4$ each represent a hydrogen atom, an aliphatic group, an aromatic group or a heteroaromatic group. When m is an integer of 2 or more and $R^2$s are linked together to form a ring, independently selected $R^2$s may be directly linked to together to form a ring, or may be linked together via at least of $R^3$ and $R^4$ to form a ring.

For example, the substituents represented by $R^2$ form a ring as follows:

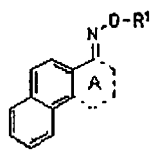

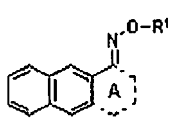

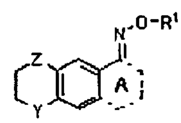

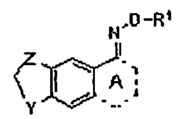

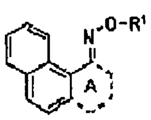 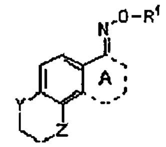 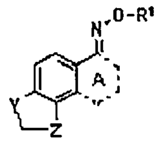 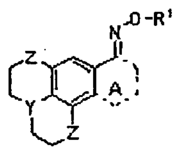

in the above Structural Formulas, Y and Z each represent $CH_2$, -O-, -S- or -NR-.

The alkyl group may have a substituent, and the total number of carbon atoms contained, therein is preferably 1 to 18, particularly preferably 1 to 10. Examples of the alkyl group include methyl, ethyl, n-propyl, i-propyl, n-butyl, i-butyl, s-butyl, t-butyl, n-hexyl, n-octyl, t-octyl and n-decyl.

The aryl group may have a substituent, and the total number of carbon atoms contained therein is preferably 6 to 20, particularly preferably 6 to 12. Examples of the alkyl group include phenyl, biphenyl, naphthyl, chlorophenyl and methoxyphenyl.

The alkyloxy group may have a substituent, and the total number of carbon atoms contained therein is preferably 1 to 18, particularly preferably 1 to 12. Examples of the alkyloxy group include methoxy, ethoxy, n-propyloxy, i-propyloxy, n-butyloxy, t-butyloxy, 2-ethylhexyloxy, n-decyloxy, phenethyloxy and phenoxyethoxy.

The aryloxy group may have a substituent, and the total number of carbon atoms contained therein is preferably 6 to 20, particularly preferably 6 to 12. Examples of the aryloxy group include phenoxy, naphthyloxy, chlorophenyloxy and methoxyphenyloxy.

The alkylthio group may have a substituent, and the total number of carbon atoms contained therein is preferably 1 to 18, particularly preferably 1 to 12. Examples of the alkylthio group include methylthio, ethylthio, i-propylthio, n-butylthio, n-octylthio, n-dodecylthio and 2-ethylhexylthio.

The total number of carbon atoms contained in the arylthio group is preferably 6 to 20, particularly preferably 6 to 12. Examples of the arylthio group include phenylthio, tolylthio, chlorophenylthio and ethoxycarbonylphenylthio.

The amino group may have, as a substituent, at lease one of an alkyl group and an aryl group, and the total number of carbon atoms contained therein is preferably 1 to 20, particularly preferably 1 to 12. Examples of the amino group include $-NH_2$, diethylamino, diphenylamino and methylphenylamino.

Specific examples of the aliphatic, aromatic and heteroaromatic groups represented by $R^2$, $R^3$ or $R^4$ include those listed above in relation to $R^1$.

Specific examples of the oxime compounds represented by General Formulas (1) to (4) include compounds having Structural Formulas (1) to (52) given below, which should not be construed as limiting the present invention thereto.

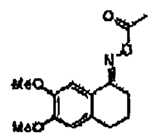
Structural Formula (1)

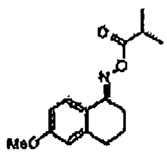
Structural Formula (2)

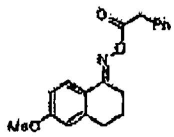
Structural Formula (3)

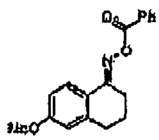
Structural Formula (4)

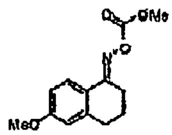
Structural Formula (5)

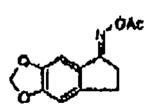
Structural Formula (6)

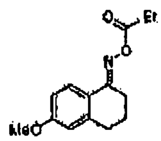
Structural Formula (7)

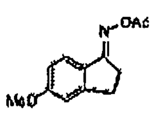
Structural Formula (8)

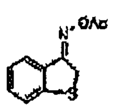
Structural Formula (9)

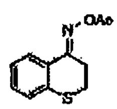
Structural Formula (10)

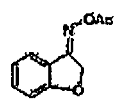
Structural Formula (11)

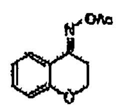
Structural Formula (12)

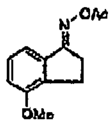
Structural Formula (13)

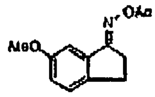
Structural Formula (14)

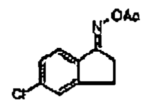
Structural Formula (15)

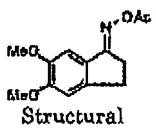
Structural Formula (16)

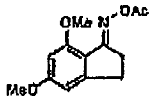
Structural Formula (17)

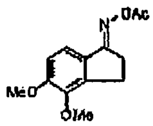
Structural Formula (18)

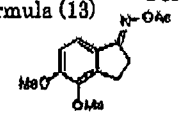
Structural Formula (19)

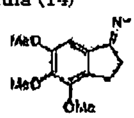
Structural Formula (20)

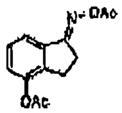
Structural Formula (21)

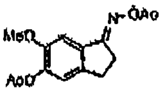
Structural Formula (22)

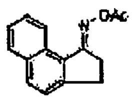
Structural Formula (23)

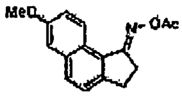
Structural Formula (24)

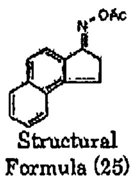
Structural Formula (25)

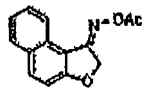
Structural Formula (26)

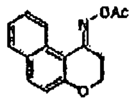
Structural Formula (27)

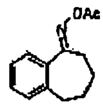
Structural Formula (28)

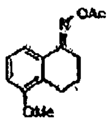
Structural Formula (29)

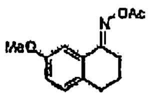
Structural Formula (30)

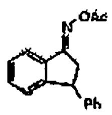
Structural Formula (31)

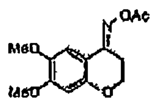
Structural Formula (32)

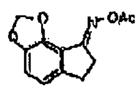
Structural Formula (33)

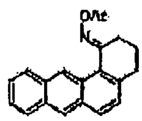
Structural Formula (34)

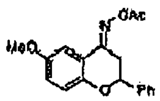
Structural Formula (35)

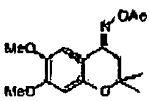
Structural Formula (36)

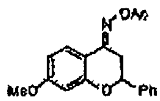
Structural Formula (37)

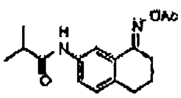
Structural Formula (38)

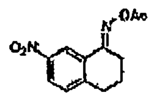
Structural Formula (39)

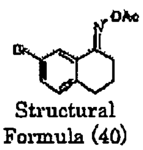
Structural Formula (40)

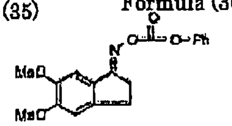
Structural Formula (41)

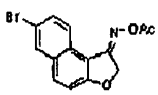
Structural Formula (42)

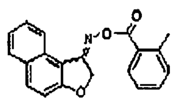
Structural Formula (43)

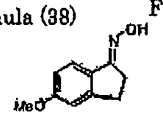
Structural Formula (44)

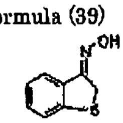
Structural Formula (45)

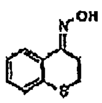
Structural Formula (46)

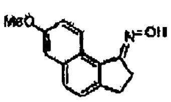
Structural Formula (47)

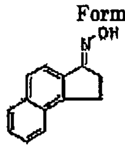
Structural Formula (48)

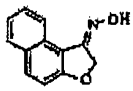
Structural Formula (49)

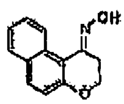
Structural Formula (50)

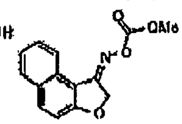
Structural Formula (51)

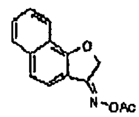
Structural Formula (52)

Notably, a novel oxime compound of the present invention can be identified through $^1$H-NMR spectral analysis or UV-vis absorption apectral analysis.

(Method for producing oxime compound)

The oxime compound of the present invention can be easily synthesized by reacting an oxime compound corresponding thereto (hereinafter referred to as an "oxime compound raw material") and serving as a starting material with acyl chloride or anhydride in the presence of a base (e.g., triethylamine and pyridine) in an inert solvent (e.g., THF,

DMF and acetonitrile) or a basic solvent (e.g., pyridine). The reaction temperature is preferably -10°C to 60°C.

Also, when a chloroformic acid ester, alkylsulfonyl chloride or arylsulfonyl chloride are used as the acyl chloride, corresponding various oxime ester compounds can be synthesized.

The oxime compound raw material used for the production of the oxime compound of the present invention as a starting material can be synethesized by a variety of methods described in standard chemistry textbooks (for instance in J. March, Advanced Organic Chemistry, 4th Edition, Wiley Interscience, 1992), or in specialized monographs, for example, S. R. Sandler & W. Karo, Organic functional group preparations, Vol. 3, Academic Press.

One of the particularly preferable methods of synthesizing the oxime compound raw material is, for example, the reaction of aldehydes or ketones with hydroxylamine or its salt in polar solvents like ethanol or aqueous ethanol. In that case, a base such as sodium acetate or pyridine is added to control the pH of the reaction mixture. It is well known that the rate of the reaction is pH-dependent, and the base can be added at the beginning or continuously during the reaction.

Basic solvents such as pyridine can also be used as base and/or solvent or cosolvent.

The reaction temperature is generally the refluxing temperature of the mixture, namely, is preferably 60°C to 120°C.

Another preferable synthesis method of the oxime compound raw material is the nitrosation of "active" methylene groups with nitrous acid or an alkyl nitrite. Both alkaline conditions, as described for example in Organic Syntheses coll. Vol. VI (J. Wily & Sons, New York, 1988), pp 199 and 840, and acidic conditions, as described, for example, in Organic Synthesis coll. vol V, pp 32 and 373, coll. vol. III, pp 191 and 513, coll. vol. II pp. 202, 204 and 363, are suitable for the synthesis of the oxime compound raw material used as the starting material in the present invention.

Nitrous acid is usually generated from sodium nitrite.

Examples of the alkyl nitrite include methyl nitrite, ethyl nitrite, isopropyl nitrite, butyl nitrite, and isoamyl nitrite.

Every oxime ester group can exist in two steric configurations, (Z) or (E). It is possible to separate the isomers by conventional methods, but it is also possible to use the isomeric mixture as such as photoinitiating species. Therefore, the oxime compound of the present invention may be a mixture of configurational isomers of compounds of Structural Formulas (1) to (52).

The oxime compound of the present invention is excellent in storage stability and sensitivity and thus, is contained in a polymerizable composition to enable it to be excellent in storage stability without causing polymerization reaction during storage and to be a highly sensitive polymerizable composition by generating active radicals upon irradiation of energy beam, especially, light, to thereby efficiently and quickly allow the polymerizable compounds to be polymerized. Thus, the oxime compound of the present invention can be used as a radical generator in printing inks, clear finish materials, powder coating materials, marking materials for roads and buildings, photographic reproduction techniques, holographic recording materials, image recording techniques, production of printing plates which can be developed with organic solvents or with aqueous alkali, daylight-curable coating materials for production of masks for screen printing, dental filling compositions, adhesives, pressure-sensitive adhesives, laminating resins, liquid etching resists (film), solder resists, electroplating resists, permanent resists, and photostructurable dielectric for printed circuit boards and/or electronic circuits. Also, the oxime compound of the present invention can be used in various materials applied to display, constituent materials in the production process of plasma-display panels and electroluminescence displays, color filters, optical switches, optical lattices (interference lattices), materials for optical circuits, materials for production of three-dimensional articles by mass curing (UV curing in transparent molds) or by the stereolithographic technique, composite materials (e.g., styrene polyesters), other thick-layered compositions, resists for coating or sealing electronic components and integrated circuits, materials for optical fibers, and coating materials for production of optical lenses. Further, the oxime compound of the present invention can be used in production of medical devices, medical aids, implants, gels with thermotropic properties. In particular, the oxime compound of the present invention suitably used in the photosensitive composition of the present invention.

More, the oxime compound of the present invention can be used as an initiator, in emulsion polymerization, pearl polymerization and suspension polymerization. Furthermore, the oxime compound of the present invention may be additionally used as a polymerization initiator for orderly arranging liquid crystal monomers or oligomers, and used as an initiator for binding dyes to organic materials.

« Photosensitive composition according to first embodiment >>

A photosensitive composition according to a first embodiment of the present invention contains at least one ethylenically unsaturated photopolymerizable compound (a), and at least one oxime compound of the present invention serving as a photopolymerization initiator, and preferably further contains a photopolymerization initiator (c) other than the oxime compound, and an additive (d), if necessary, contains binder (e).

< (a) Polymerizable Compound >

An unsaturated photopolymerizable compound (a) may include one or more olefinic double bonds. They may be of low (monomeric) or high (oligomeric) molecular mass.

Examples of monomers containing a double bond include alkyl, hydroxyalkyl or amino acrylates, or alkyl, hydroxyalkyl or amino methacrylates, for example methyl, ethyl, butyl, 2-ethylhexyl or 2-hydroxyethyl acrylate, isobornyl acrylate, methyl methacrylate or ethyl methacrylate. Silicone acrylates are also advantageous. Other examples thereof include acrylonitrile, acrylamide, methacrylamide, N-substituted (meth)acrylamides, vinyl esters such as vinyl acetate, vinyl ethers such as isobutyl vinyl ether, styrene, alkyl- and halostyrenes, N-vinylpyrrolidone, vinyl chloride or vinylidene chloride.

Examples of monomers containing two or more double bonds include the diacrylates of ethylene glycol, propylene glycol, neoprene glycol, hexamethylene glycol or of bisphenol A, and 4,4'-bis(2-acryl-oyloxyethoxy)diphenylpropane, trimethylolpropane triacrylate, pentaerythritol triacrylate or tetraacrylate, dipentaerythritol hexaacrylate vinyl acrylate, divinylbenzene, divinyl succinate, diallyl phthalate, triallyl phosphate, triallyl isocyanurate or tris(2-acryloylethyl) isocyanurate.

Examples of polyunsaturated compounds having relatively high molecular mass (oligomers) include acrylated epoxy resins, polyesters containing acrylate-, vinyl ether- or epoxy-groups, and also polyurethanes and polyethers. Further examples of unsaturated oligomers are unsaturated polyester resins, which are usually prepared from maleic acid, phthalic acid and at least one diol and have molecular weights of from about 600 to 3,000. In addition it is also possible to employ vinyl ether monomers and oligomers, and also maleate-terminated oligomers with polyester, polyurethane, polyether, polyvinyl ether and epoxy main chains.

Of particular suitability are combinations of oligomers which have vinyl ether groups and of polymers as described in International Publication No. WO 90/01512. However, copolymers of vinyl ether and maleic acid-functionalized monomers are also suitable. Unsaturated oligomers of this kind can also be referred to as prepolymers.

Particularly suitable examples are esters of ethylenically unsaturated carboxylic acids and polyols or polyepoxides, and polymers having ethylenically unsaturated groups in the chain or in side groups, for example unsaturated polyesters, polyamides and polyurethanes and copolymers thereof, polymers and copolymers containing (meth)acrylic groups in side chains. These may be used alone or in mixtures of one or more such polymers.

Examples of unsaturated carboxylic acids include acrylic acid, methacrylic acid, crotonic acid, itaconic acid, cinnamic acid, and unsaturated fatty acids such as linolenic acid or oleic acid. Among these, acrylic and methacrylic acid are preferred.

Suitable polyols are aromatic and, in particular, aliphatic and cycloaliphatic polyols. Examples of aromatic polyols include hydroquinone, 4,4'-hydroxydiphenyl, 2,2-di(4-hydroxyphenyl)propane, and also novolaks and resols.

Examples of polyepoxides include those based on the above-mentioned polyols, especially the aromatic polyols, and epichlorohydrin. Other suitable polyols include polymers and copolymers containing hydroxyl groups in the polymer chain or in side groups, examples being polyvinyl alcohol and copolymers thereof or polyhydroxyalkyl methacrylates or copolymers thereof. Further polyols which are suitable are oligoesters having hydroxyl end groups.

Examples of aliphatic and cycloaliphatic polyols include alkylenediols having preferably 2 to 12 carbon atoms, such as ethylene glycol, 1,2- or 1,3-propanediol, 1,2-, 1,3- or 1,4-butanediol, pentanediol, hexanediol, octanediol, dodecanediol, diethylene glycol, triethylene gicyol, polyethylene glycols having molecular weights of preferably from 200 to 1500, 1,3-cyclopentanediol, 1,2-, 1,3- or 1,4-cyclohexanediol, 1,4-dihydroxymethylcyclohexane, glycerol, tris(β-hydroxyethyl)amine, trimethylolethane, trimethylolpropane, pentaerythritol, dipentaerythritol and sorbitol.

The polyols may be partially or completely esterified with one carboxylic acid or with different unsaturated carboxylic acids, and in partial esters the free hydroxyl groups may be modified, for example etherified or esterified with other carboxylic acids.

Examples of esters include trimethylolpropane triacrylate, trimethylolethane triacrylate, trimethylolpropane trimeth-acrylate, trimethylolethane trimethacrylate, tetramethylene glycol dimethacrylate, triethylene glycol dimethacrylate, tetraethylene glycol diacrylate, pentaerythritol diacrylate, pentaerythritol triacrylate, pentaerythritol tetraacrylate, dipentaerythritol diacrylate, dipentaerythritol triacrylate, dipentaerythritol tetraacrylate, dipentaerythritol pentaacrylate, dipentaerythritol hexaacrylate, tripentaerythritol octaacrylate, pentaerythritol dimethacrylate, pentaerythritol trimethacrylate, dipentaerythritol dimethacrylate, dipentaerythritol tetramethacrylate, tripen-taerythritol octamethacrylate, pentaerythritol diitaconate, dipentaerythritol tris-itaconate, dipentaerythritol pentaitaconate, dipentaerythritol hexaitaconate, ethylene glycol diacrylate, 1,3-butanediol diacrylate, 1,3-butanediol dimethacrylate, 1,4-butanediol diitaconate, sorbitol triacrylate, sorbitol tetraacrylate, pentaerythritol-modified triacrylate, sorbitol tetra methacrylate, sorbitol pentaacrylate, sorbitol hexaacrylate, oligoester acrylates and methacrylates, glycerol diacrylate and triacrylate, 1,4-cyclohexane diacrylate, bisacrylates and bismethacrylates of polyethylene glycol with a molecular weight of from 200 to 1500, or mixtures thereof.

Also suitable as unsaturated photopolymerizable compounds (a) are the amides of identical or different, unsaturated carboxylic acids with aromatic, cycloaliphatic and aliphatic polyamines having preferably 2 to 6, especially 2

to 4, amino groups. Examples of the polyamines include ethylenediamine, 1,2- or 1,3-propylenediamine, 1,2-, 1,3- or 1,4-butylenediamine, 1,5-pentylenediamine, 1,6-hexylenediamine, octylenediamine, dodecylenediamine, 1,4-diaminocyclohexane, isophoronediamine, phenylenediamine, bisphenylenediamine, di-β-aminoethyl ether, diethylenetriamine, triethylenetetramine, di(β-aminoethoxy)- or di(β-aminopropoxy)ethane.

Examples of other suitable polyamines include polymers and copolymers, preferably with additional amino groups in the side chain, and oligoamides having amino end groups. Examples of such unsaturated amides include methylenebisacrylamide, 1,6-hexamethylenebisacrylamide, diethylenetriaminetrismethacrylamide, bis(mathacrylamidopropoxy)ethane, β-methacrylamidoethyl methacrylate and N[(β-hydroxyethoxy)ethyl]acrylamide.

Suitable unsaturated polyesters and polyamides are derived, for example, from maleic acid and from diols or diamines. Some of the maleic acid can be replaced by other carboxylic acids. They can be used together with ethylenically unsaturated comonomers, for example styrene. The unsaturated polyesters and polyamides may also be derived from dicarboxylic acids and from ethylenically unsaturated diols or diamines, especially from those with relatively long chains of, for example 6 to 20 carbon atoms. Examples of polyurethanes include those composed of saturated or unsaturated diisocyanates and of unsaturated or, respectively, saturated diols.

Polymers with (meth)acrylate groups in the side chain are not particularly limited and may be appropriately selected from those known depending on the purpose. They may, for example, be reaction products of epoxy resins based on novolaks with (meth)acrylic acid, or may be homo- or copolymers of vinyl alcohol or hydroxyalkyl derivatives thereof which are esterified with (meth)acrylic acid, or may be homo- and copolymers of (meth)acrylates which are esterified with hydroxyalkyl (meth)acrylates.

Other suitable polymers with acrylate or methacrylate groups in the side chains are, for example, solvent soluble or alkaline soluble polyimide precursors, such as poly(amic acid ester) compounds, having the photopolymerizable side groups either attached to the backbone or to the ester groups in the molecule, i.e. according to EP 624826. Such oligomers or polymers can be formulated with the novel oxime compound of the present invention and optionally reactive diluents, such as polyfunctional (meth)acrylates in order to prepare highly sensitive polyimide precursor resists.

The photopolymerizable compound (a) may be used alone or combined appropriately with other compounds. It is preferred to use mixtures of polyol (meth)acrylates.

Examples of the photopolymerizable compounds (a) include polymers or oligomers having at least two ethylenically unsaturated groups and at least one carboxyl function within the molecule structure, such as a resin obtained by the reaction of a saturated or unsaturated polybasic acid anhydride with a product of the reaction of an epoxy compound and an unsaturated monocarboxylic acid (for example, EB9696, UCB Chemicals; KAYARAD TCR1025, manufactured by Nippon Kayaku Co., LTD.), or an addition product formed between a carboxyl group-containing resin and an unsaturated compound having an $\alpha,\beta$-unsaturated double bond and an epoxy group (for example, ACA200M, Daicel Industries, Ltd.).

When a mono- or poly-functional ethylenically unsaturated compound, or mixtures of several of said compounds is contained in a diluent, the amount thereof is 95% by mass or less based on the solid constitution of the photosensitive composition.

The unsaturated photopolymerizable compounds (a) can also be used as a mixture with non-photopolymerizable compound, thin film-forming components. Examples of the non-photopolymerizable compound include physically drying polymers, such as nitrocellulose or cellulose acetobutyrate. They may also, however, be chemically and/or thermally curable (heat-curable) resins, examples being polyisocyanates, polyepoxides and melamine resins, as well as polyimide precursors. The use of heat-curable resins in combination with the unsaturated photopolymerizable compounds (a) is important for the photosensitive composition. The photosensitive composition is photopolymerized so as to form a cured film in a first exposing step, and then thermally crosslinked in a second heating step, thereby increasing film strength of the cured film.

The photosensitive composition of the present invention also provides compositions containing as the unsaturated photopolymerizable compounds (a) at least one ethylenically unsaturated photopolymerizable compound which is emulsified or dissolved in water. Various kinds of such radiation-curable aqueous prepolymer dispersions are commercially available and easily obtainable. A prepolymer dispersion is understood as being a dispersion of water and at least one prepolymer dispersed therein. The amount of water in the aqueous prepolymer dispersion is, for example, from 5% by mass to 80% by mass, in particular from 30% by mass to 60% by mass. The amount of the radiation-curable aqueous prepolymer or prepolymer mixture is, for example, from 20% by mass to 95% by mass, in particular from 40% by mass to 70% by mass. In these compositions the sum of the percentages given for water and prepolymer is in each case 100% by mass, with auxiliaries and additives being added in varying quantities depending on the intended use.

The radiation-curable, film-forming prepolymers which are dispersed or dissolved in water are not particularly limited and may be suitably selected from those known depending on the purpose. For example, aqueous prepolymer dispersions of mono or polyfunctional, ethylenically unsaturated prepolymers is used, and can be initiated by free radicals and preferably have a content of from 0.01 mol to 1.0 mol of polymerizable double bonds per 100 g of prepolymer and an average molecular weight of, for example, at least 400, in particular from 500 to 10,000. Prepolymers having higher

molecular weights, however, may also be used depending on the intended application. Example of those preferably used in the present invention include polyesters containing polymerizable C-C double bonds and having an acid number of 10 or less, polyethers containing polymerizable C-C double bonds, hydroxyl-containing reaction products of a polyepoxide, containing at least two epoxide groups per molecule, with at least one α,β-ethylenically unsaturated carboxylic acid, polyurethane (meth)acrylates and cyclic copolymers which contain α,β-ethylenically unsaturated acrylic radicals. Specific examples thereof include those as described in EP 12339. Mixtures of these prepolymers can likewise be used. Also suitable are the polymerizable prepolymers described in EP 33896, which are thioether adducts of polymerizable prepolymers having an average molecular weight of at least 600, a carboxyl group content of from 0.2% to 15% and a content of from 0.01 mol to 0.8 mol of polymerizable C-C double bonds per 100 g of prepolymer. Other suitable aqueous dispersions, based on specific alkyl (meth)acrylate polymers, are described in EP 41125, and suitable water-dispersible, radiation-curable prepolymers of urethane acrylates can be found in DE 2936039.

Further additives which may be included in these radiation-curable aqueous prepolymer dispersions are dispersion auxiliaries, emulsifiers, antioxidants, e.g. 2,2-thiobis(4-methyl-6-t-butylphenol) or 2,6-di-t-butylphenol, light stabilizers, dyes, pigments, fillers (such as glass, alumina, talc, gypsum, silicic acid, rutile, carbon black, zinc oxide, iron oxides), reaction accelerators, levelling agents, lubricants, wetting agents, thickeners, flatting agents, antifoams and other auxiliaries customary in paint technology. Suitable dispersion auxiliaries are water-soluble organic compounds which have high molecular mass and contain polar groups, examples being polyvinyl alcohols, polyvinylpyrrolidone or cellulose ethers. Emulsifiers which can be used are nonionic emulsifiers and, if desired, ionic emulsifiers as well.

< Photopolymerization initiator >

The photosensitive composition of the present invention contains the oxime compound (b) of the present invention as a photopolymerization initiator, and further contains conventionally known photopolymerization initiator (c) other than the oxime compound as necessary.

< (b) Oxime Compound >

In the photosensitive composition, only one type of the oxime compound of the present invention may be contained, and a mixture of two or more kinds thereof is preferably contained. The oxime compound of the present invention is a compound represented by the above General Formula (1).

< (c) Conventionally Known Photopolymerization Initiator >

The photosensitive composition of the present invention may include the conventionally known photopolymerization initiators other than the oxime compound (b) of the present invention.

Examples of the photopolymerization initiator (c) include camphor quinone, benzophenone, benzophenone derivatives, acetophenone, acetophenone derivatives, for example α-hydroxycycloalkyl phenyl ketones or 2-hydroxy-2-methyl-1-phenyl-propanone, dialkoxyacetophenones, .alpha.-hydroxy-or α-aminoacetophenones, e.g, (4-methylthiobenzoyl)-1-methyl-1-morpholinoethane, (4-morpholinobenzoyl)-1-benzyl-1-dimethylaminopropane, 4-aroyl-1,3-dioxolanes, benzoin alkyl ethers and benzil ketals, e.g. dimethyl benzil ketal, phenylglyoxahc esters and derivatives thereof, dimeric phenylglyoxalic esters, diacetyl, peresters, e.g. benzophenone tetracarboxylic peresters as described for example in EP 126541, monoacyl phosphine oxides, e.g, (2,4,6-trimethylbenzoyl)diphenylphosphine oxide, bisacylphosphine oxides, bis(2,6-dimethoxy-benzoyl)-(2,4,4-trimethyl-pentyl)-phosphine oxide, bis(2,4,6-trimethylbenzoyl)-phenylphosphine oxide, bis(2,4,6-trimethylbenzoyl)-2,4-dipentoxyphenylphosphine oxide, trisacylphoephine oxides, halomethyltriazines, e.g. 2-[2-(4-methoxy-phenyl)-vinyl]-4,6-bis-trichloromethyl-[1,3,5]triazine, 2-(4-methoxy-phenyl)-4,6-bis-trichloromethyl-[1,3,5]triazine, 2-(3,4-dimethoxy-phenyl)-4,6-bis-trichloromethyl-[1,3,5]triazine, 2-methyl-4,6-bis-trichloromethyl-[1,3,5]triazine, 2-(4-N,N-di(ethoxycarbonylmethyl)aminophenyl)-4,6-bis(trichloromethyl)-[1, 3,5]triazine, 2-(4-methoxy-naphthyl)-4,6-bis-trichloromethyl-[1,3,5]triazine, 2-(1,3-benzodioxol-5-yl)-4,6-bis-trichloromethyl-[1,3,5]triazine, 2-[2-[4-(pentyloxy)phenyl]ethenyl]-4,6-bis-trichloromethyl-[1,3,5]triazine, 2-[2-(3-methyl-2-furanyl)-ethenyl]-4,6-bis-trichloromethyl-[1,3,5]triazine, 2-[2-(5-methyl-2-furanyl)-ethenyl]-4,6-bis-trichloromethyl-[1,3,5]triazine, 2-[2-(2,4-dimethoxy-phenyl)-ethenyl]-4,6-bis-trichloromethyl-[1,3,5]triazine, 2-[2-(2-methoxy-phenyl)ethenyl]-4,8-bis-trichloromethyl-[1,3,5]-triazine, 2-[2-(4-isopropyloxy-phenyl)-ethenyl]-4,6-bis-trichloromethyl-[1,3,6]triazine 2-[2'(3-chloro-4-methoxy-phenyl)ethenyl]-4,6-bis-trichloromethyl-[1,3,5]triazine, 2-[2-bromo-4-N,N-di(ethoxycarbonylmethyl)amino-phenyl]-4,6-bis-trichloromethyl-[ 1,3,5]triazine, 2-[2-chloro-4-N,N-di(ethoxycarbonylmethyl)amino-phenyl]-4,6-bis-trichloromethyl-[ 1,3,5]triazine, 2-[3-bromo-4-N,N-di(ethoxycarbonylmethyl)amino-phenyl]-4,6-bis-trichloromethyl-[ 1,3,5]triazine, 2-[3-chloro-4-N, N-di(ethoxycarbonylmethyl)amino-phenyl]-4,6-bis-trichloromethyl-[1,3,5]triazine, or other halomethyl-triazines as described for example in G. Buhr, R. Dammel and C. Lindley Polym. Mater. Sci. Eng. 61,269 (1989), and EP 022788; halomethyl-oxazol photoinitiators, such as described in U.S. Pat. No. 4,371,606 and U.S. Pat.

No. 4,371,607; 1,2-disulfones, such as described in E. A, Bartmann, Synthesis 5, 490 (1993); hexaarylbisimidazole, and hexaarylbisimidazole/coinitiators systems, e.g. ortho-chlorohexaphenyl-bisimidazole combined with 2-mercaptobenzthiazole, ferrocenium compounds, or titanocenes, e.g. bis(cyclopentadienyl)-bis(2,6-difluoro-3-pyrryl-phenyl)titanium.

Moreover, examples of the photopolymerization initiator (c) further include cationic photoinitiators, peroxide compounds, (such as benzoyl peroxide, peroxide compounds described in U.S. Pat. No. 4,950,581 column 19, lines 17-25), aromatic sulfonium-, phosphonium- or iodonium salts as described for example in U.S. Pat. No. 4,950,581, column 18, line 60 to column 19, line 10 or cyclopentadienyl-arene-iron(II) complex salts, for example ($\eta^6$-iso-propylbenzene)($\eta^5$-cyclopentadienyl)iron(II) hexafluorophosphate, and oxime sulfonic acid esters (for example, those described in EP 780729). Also pyridinium and (iso)quinolinium salts as described e.g. in EP 497531 and EP 441232 may be used in combination with the oxime compound of the present invention.

The oxime compound of the present invention, either alone or in mixtures with other conventionally known photopolymerization initiators and sensitizers, can be used also in the form of a dispersion or emulsion in water or aqueous solutions.

The photosensitive composition generally contains 0.01% by mass to 25% by mass, preferably 0.01% by mass to 10% by mass, in particular 0.01% by mass to 5% by mass of the photopolymerization initiator, based on the solid composition. The amount refers to the sum of all photopolymerization initiators added, when the oxime compound of the present invention and photopolymerization initiators other than the oxime compound are used in combination. Accordingly, the amount either refers to the oxime compound (b) or the oxime compound (b) + the photopolymerization initiator (c).

< (d) Additive >

As the additive (d), a thermal inhibitor, which is intended to prevent premature polymerization is used. Examples thereof include hydroquinone, hydroquinone derivatives, p-methoxyphenol, β-naphthol or sterically hindered phenols, such as 2,6-di-tert-butyl-p-cresol.

In order to increase the stability on storage in the dark it is possible, for example, to use copper compounds, such as copper naphthenate, stearate or octoate, phosphorus compounds, for example triphenylphosphine, tributylphosphine, triethyl phosphite, triphenyl phosphite or tribenzyl phosphite, quaternary ammonium compounds, for example tetramethylammonium chloride or trimethylbenzylammonium chloride, or hydroxylamine derivatives, for example N-diethylhydroxylamine.

To exclude atmospheric oxygen during the polymerization it is possible to add paraffin or similar wax-like substances which, being of inadequate solubility in the polymer, migrate to the surface in the beginning of polymerization and form a transparent surface layer which prevents the ingress of air. It is also possible to apply an oxygen-impermeable layer on top of the coating, for example poly(vinylalcohol-co-vinylacetate).

Light stabilizers which can be added in a small amount, and examples thereof include UV absorbers, for example those of the hydroxyphenylbenzotriazole, hydroxyphenyl-benzophenone, oxalamide or hydroxyphenyl-s-triazine type. These compounds can be used alone or in mixtures, with or without sterically hindered amines (HALS).

Examples of the UV absorber and light stabilizer include 2-(2'-hydroxyphenyl)benzotriazoles, 2-hydroxybenzophenones, esters of substituted or unsubstituted benzoicacids, acrylates, sterically hindered amines, oxalamides, 2-(2-hydroxyphenyl)-1,3,5-triazines, and phosphites and phosphonites.

Examples of the 2-(2'-hydroxyphenyl)benzotriazoles include 2-(2'-hydroxy-5'-methylphenyl)benzotriazole, 2-(3',5'-di-tert-butyl-2'-hydroxyphenyl)benzotriazole, 2-(5'-tert-butyl-2'-hydroxyphenyl)benzotriazole, 2-(2'-hydroxy-5'-(1-,1-,3,3-tetramethylbutyl)phenyl)benzotriazole, 2-(3',5'-di-tert-butyl-2'-hydroxyphenyl)-5-chlorobenzotriazole, 2-(3'-tert-butyl-2'-hydroxy-5'-methylphenyl)-5-chlorobenzotriazole, 2-(3'-sec-butyl-5'-tert-butyl-2'-hydroxyphenyl)benzotriazole, 2-(2'-hydroxy-4'-octoxyphenyl)benzotriazole, 2-(3',5'-di-tert-amyl-2'-hydroxyphenyl)benzotriazole, 2-(3',5'-bis-((α, α-dimethylbenzyl)-2'-hydroxyphenyl)-benzotria zole, mixture of 2-(3'-tert-butyl-2'-hydroxy-5'-(2-octyloxycarbonylethyl) phenyl)-5-chlorobenzotriazole, 2-(3'-tert-butyl-5'-[2-(2-ethyl-hexyl-oxy)carbonylethyl]-2'-hydroxyphenyl) -5-chlorobenzotriazole, 2-(3'-tert-butyl-2'-hydroxy-5'-(2-methoxycarbonylethyl)phenyl)-5-chlorobenzotriazole, 2-(3'-tert-butyl-2'-hydroxy-5'-(2-methoxycarbonylethyl)phenyl)-benzotriazole, 2-(3'-tert-butyl-2'-hydroxy-5'-(2-octyloxycarbonylethyl)phenyl)benzotriazole, 2-(3'-tert-butyl-5'-[2-(2-ethylhexyloxy)carbonylethyl]-2'-hydroxyphenyl)benzotriazole, 2-(3'-dodecyl-2'-hydroxy5'-methylphenyl)benzotriazole, and 2-(3'-tert-butyl-2'-hydroxy-5'-(2-isooctyloxycarbonylethyl)phenyl)benzotriazole; 2,2'-methylene-bis[4-(1,1,3,3-tetramethylbutyl)-6-benzotriazol-2-yl-phenol]; transesterification product of 2-[3'-tert-butyl-5'-(2-methoxycarbonylethyl)-2'-hydroxy-phonyl]-benzotriazole with polyethylene glycol 300; $(R-CH_2C)H_2-COO(CH_2)_3]_2$ (where R=3'-tert-butyl-4'-hydroxy-5'-2H-benzotriazol-2-yl-phenyl).

Examples of the 2-hydroxybenzophenones include 4-hydroxy-, 4-methoxy-, 4-octoxy-, 4-decyloxy-, 4-dodecyloxy-, 4-benzyloxy-, 4,2',4'-trihydroxy- and 2'-hydroxy-4,4'-dimethoxy derivative.

Examples of the esters of substituted or unsubstituted benzoic acids iclude 4-tert-butylphenyl salicylate, phenyl salicylate, octylphenyl salicylate, dibenzoylresorcinol, bis(4-tert-butylbenzoyl)resorcinol, benzoylresorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, and 2-methyl-4,6-di-tert-butylphenyl 3,5-di-tert-butyl-4-hydroxybenzoate.

Examples of the acrylates include isooctyl or ethyl α-cyano-β,β, methyl α-carbomethoxycinnamate, butyl or methyl α-cyano-β-methyl-p-methoxycinnamate, methyl α-carboxymethoxy-p-methoxycinnamate and N-(β-carbomethoxy-β-cyanovinyl)-2-methylindoline.

Examples of the sterically hindered amines include bis-(2,2,6,6-tetramethylpiperidyl)sebacate, bis-(2,2,6,6-tetramethylpiperidyl)succinate bis-(1,2,2,6,6-pentamethylpiperidyl)sebacate, bis-(1,2,2,6,6-pentamethylpiperidyl)n-butyl-3,6-di-tert-butyl-4-hydroxyben zylmalonate, condensation product of 1-hydroxyethyl-2,2,6,6-tetramethyl-4-hydroxypiperidine and succinic acid, condensation product of N,N'-bis-(2,2,6,6-tetramethyl-4-piperidyl)hexa-methylenediamine and 4-tert-octylamino-2,6-dichloro-1,3,5-s-triazine, tris-(2,2,6,6-tetramethyl-4-piperidyl)nitrilotriacetate, tetrakis-(2,2,6,6-tetramethyl-4-piperidyl)-1,2,3,4-butane tetraoate, 1,1'-(1,2-ethandiyl)bis(3,3,5,5-tetramethyl-piperazinone), 4-benzoyl-2,2,6,6-tetramethylpiperidine, 4-stearyloxy-2,2,6,6-tetramethylpiperidine, bis-(1,2,2,6,6-pentamethyl-4-piperidyl)2-n-butyl-2-(2-hydroxy-3,5-di-tert-butylbenzyl )malonate, 3-n-octyl-7,7,9,9-tetramethyl-1,3,8-triazaspiro-[4.5]decane-2,4-dione, bis-(1-octyloxy-2,2,6,6-tetramethylpiperidyl)sebacate, bis-(1-octyloxy-2,2,6,6-tetramethylpiperidyl)succinate, condensation product of N,N'-bis-(2,2,6,6-tetra-methyl-4-piperidyl)hexamethylenediamine and 4-morpholino-2,6-dichloro-1,3,5-triazine, condensation product of 2-chloro-4,6-di-(4-n-butylamino-2,2,6,6-tetramethylpiperidyl)-1,3,5-triazine and 1,2-bis-(3-aminopropyl-amino)ethane, condensation product of 2-chloro-4,6-di-(4-n-butylamino-1,2,2,6,6-pentamethylpiperidyl)-1,3,5-triazine and 1,2-bis-(3-aminopropylamino)ethane, 8-acetyl-3-dodecyl-7,7,9,9-tetramethyl-1,3,8-triazaspiro[4.5]decane-2,4-dione, 3-dodecyl-1-(2,2,6,6-tetramethyl-4-piperidyl)pyrrolidine-2,5-dione and 3-dodecyl-1-(1,2,2,6,6-penta-methyl-4-piperidyl)-pyrrolidine-2,5-dione.

Example of the oxalamides include 4,4'-dioctyloxyoxanilide, 2,2'-diethoxyoxanilide, 2,2'-dioctyloxy-5,5'-di-tert-butyloxanilide, 2,2'-didodecyloxy-5,5'di-tert-butyloxanilide, 2-ethoxy-2'-ethyl-oxanilide, N,N'-bis-(3-dimethylaminopropyl)oxalamide, 2-ethoxy-5-tert-butyl-2'-ethyloxanilide, 2-ethoxy-2'-ethyl-5,4'-di-tert-butyloxanilide and mixtures thereof, mixtures of o- and p-methoxy- and of o- and p-ethoxy-disubstituted oxanilides.

Examples of the 2-(2-hydroxyphenyl)-1,3,5-triazines include 2,4,6-tris(2-hydroxy-4-octyloxyphenyl)-1,3,5-triazine, 2-(2-hydroxy-4-octyloxyphenyl)-4,6-bis-(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxy-phenyl)-6-(2,4-dimethylpheny)-1,3,5-triazine, 2-(2-hydroxy-4-octyloxyphenyl)-4,6-bis(4-methylphanyl)-1,3,5-triazine, 2-(2-hydroxy-4-dodecyloxyphenyl)-4,6-bis(2,4-dimethylphenyl)-1,3,5-triazine, 2-[2-hydxoxy-4-(2-hydxoxy-3-butyloxy-propyloxy)phenyl]-4,6-bis(2,4-dimethylphenyl) -1,3,5-triazine, 2-[2-hydroxy-4-(2-hydroxy-3-octyloxy-propyloxy)phenyl]-4,6-bis(2,4-dimethylphenyl) -1,3,5-triazine, 2-[4-dodecyl/ tridecyl-oxy-(2-hydroxypropyl)oxy-2-hydroxy-phenyl]-4,6-bis(2,4-dimethylph enyl)-1,3,5-triazine.

Example of the phosphites and phosphonites include triphenyl phosphite, diphenyl alkyl phosphites, phenyl dialkyl phosphites, tris(nonylphenyl) phosphite, trilauryl phosphite, trioctadecyl phosphite, distearyl pentaerythrityl diphosphite, tris-(2,4-di-tert-butylphenyl) phosphite, diisodecyl pentaerythrityl diphosphite, bis-(2,4-di-tert-butylphenyl) pentaerythrityl diphosphite, bis-(2,6-di-tert-butyl-4-methylphenyl) pentaerythrityl diphosphite, bis-isodecyloxy pentaerythrityl diphosphite, bis-(2,4-di-tert-butyl-6-methylphenyl) pentaerythrityl diphosphite, bis-(2,4,6-tri-tert-butylphenyl)pentaerythrityl diphosphite, tristearyl sorbityl triphosphite, tetrakis-(2,4-di-tert-butylphenyl)-4,4'-biphenylene diphosphonite, 6-isooctyloxy-2,4,8,10-tetra-tert-butyl-12H-di-benzo[d,g]-1,3,2-dioxaphosphocine, 6-fluoro-2,4,8,10-tetra-tert-butyl-12-methyl-dibenzo[d,g]-1,3,2-dioxaphosphocine, bis-(2,4-di-tert-butyl-6-methylphenyl)methyl phosphite and bis(2,4-di-tert-butyl-6-methylphenyl)ethyl phosphite.

To accelerate the photopolymerization it is possible to add amines as the additive (d). Examples of the amines include triethanolamine, N-methyldiethanolamine, ethyl-p-dimethylaminobenzoate, 2-(dimethylamino)ethyl benzoate, 2-ethylhexyl-p-dimethylaminobenzoate, octyl-para-N,N-dimethylaminobenzoate, N-(2-hydroxyethyl)-N-methyl-para-toluidine or Michler's ketone. The action of the amines can be intensified by the addition of aromatic ketones of the benzophenone type.

The amines can be also used as oxygen scavengers. Examples thereof include substituted N,N-dialkyloximes, as are described in EP 339841.

As the additives, other accelerators, coinitiators and autoxidizers may be used. Examples thereof include thiols, thioethers, disulfides, phosphonium salts, phosphine oxides or phosphines, as described, for example, in EP 438123, in GB 2180358 and in JP-A No. 06-68309.

It is further possible to add chain transfer agents which are customary in the art to the photosensitive composition of the present invention as the additive (d). Examples thereof include mercaptans, amines and benzothiazol.

Photopolymerization of the photosensitive composition of the present invention can also be accelerated by adding further photosensitizers or coinitiators, as the additive (d), which shift or broaden the spectral sensitivity. In particular, aromatic compounds are preferable as the photosensitizers or coinitiators, and examples thereof include benzophenone and derivatives thereof, thioxanthone and derivatives thereof, anthraquinone and derivatives thereof, coumarin and phenothiazine and derivatives thereof, 3-(aroylmethylene)thiazolines, rhodanine, camphorquinone, eosine, rhodamine, erythrosine, xanthene, thioxanthene, acridine, e.g. 9-phenylacridine, 1,7-bis(9-acridinyl)heptane, 1,5-bis(9-acridinyl)pentane, cyanine and merocyanine dyes.

Examples of the thioxanthones include thioxanthone, 2-isopropylthioxanthone, 2-chlorothioxanthone, 1-chloro-4-propoxythioxanthone, 2-dodecylthioxanthone, 2,4-diethylthioxanthone, 2,4-dimethylthioxanthone, 1-methoxycarbonylthioxanthone, 2-ethoxycarbonylthioxanthone, 3-(2-methoxyethoxycarbonyl)-thioxanthone, 4-butoxycarbonylthioxanthone, 3-butoxycarbonyl-7-methylthioxanthone, 1-cyano-3-chlorothioxanthone, 1-ethoxycarbonyl-3-chlorothioxanthone, 1-ethoxycarbonyl-3-ethoxythioxanthone, 1-ethoxycarbonyl-3-aminothioxanthone, 1-ethoxycarbonyl-3-phenylsulfurylthioxanthone, 3,4-di-[2-(2-methoxyethoxy)ethoxycarbonyl]-thioxanthone, 1,3,dimethyl-2-hydroxy-9H-thioxanthen-9-one 2-ethylhexylether, 1-ethoxycarbonyl-3-(1-methyl-1-morpholinoethyl)-thioxanthone, 2-methyl-6-dimethoxymethyl-thioxanthone, 2-methyl-6-(1,1-dimethoxybenzyl)-thioxanthone, 2-morpholinomethylthioxanthone, 2-methyl-6-morpholinomethylthioxanthone, N-allylthioxanthone-3,4-dicarboximide, N-octylthioxanthone-3,4-dicarboximide, N-(1,1,3,3-tetramethylbutyl)-thioxanthone-3,4-dicarboximide, 1-phenoxythioxanthone, 6-ethoxycarbonyl-2-methoxythioxanthone, 6-ethoxycarbonyl-2-methylthioxanthone, thioxanthone-2-carboxylic acid polyethyleneglycol ester, 2-hydroxy-3-(3,4-dimethyl-9-oxo-9H-thioxanthon-2-yloxy)-N,N,N-trimethyl-1-propanaminium chloride.

Examples of the benzophenones include benzophenone, 4-phenyl benzophenone, 4-methoxy benzophenone, 4,4'-dimethoxy benzophenone, 4,4'-dimethyl benzophenone, 4,4'-dichlorobenzophenone 4,4'-bis(dimethylamino)benzophenone, 4,4'-bis(diethylamino)benzophenone, 4,4'-bis(methylethylamino)benzophenone, 4,4'-bis(p-isopropylphenoxy)benzophenone, 4-methyl benzophenone, 2,4,6-trimethylbenzophenone, 4-(4-methylthiophenyl)-benzophenone, 3,3'-dimethyl-4-methoxy benzophenone, methyl-2-benzoylbenzoate, 4-(2-hydroxyethylthio)-benzophenone, 4-(4-tolylthio)benzophenone, 1-[4-(4-benzoyl-phenylsulfanyl)-phenyl]-2-methyl-2-(toluene-4-sulfonyl)-propan-1-on e, 4-benzoyl-N,N,N-trimethylbenzenemethanaminium chloride, 2-hydroxy-3-(4-benzoylphenoxy)-N,N,N-trimethyl-1-propanaminium chloride monohydrate, 4-(13-acryloyl-1,4,7,10,13-pentaoxatridecyl)-benzophenone, 4-benzoyl-N,N-dimethyl-N-[2-(1-oxo-2-propenyl)oxylethyl-benzenemethanaminium chloride.

Examples of the the Coumarins include Coumarin 1, Coumarin 2, Coumarin 6, Coumarin 7, Coumarin 30, Coumarin 102, Coumarin 106, Coumarin 138, Coumarin 152, Coumarin 153, Coumarin 307, Coumarin 314, Coumarin 314T, Coumarin 334, Coumarin 337, Coumarin 500, 3-benzoyl coumarin, 3-benzoyl-7-methoxycoumarin, 3-benzoyl-5,7-dimethoxycoumarin, 3-benzoyl-5,7-dipropoxycoumarin, 3-benzoyl-6,8-dichlorocoumarin, 3-benzoyl-6-chloro-coumarin, 3,3'-carbonyl-bis[5,7-di(propoxy)coumarin], 3,3'-carbonyl-bis(7-methoxycoumarin), 3,3-carbonyl-bis(7-diethylamino-coumarin), 3-isobutyroylcoumarin, 3-benzoyl-5,7-dimethoxy-coumarin, 3-benzoyl-5,7-diethoxy-coumarin, 3-benzoyl-5,7-dibutoxycoumarin, 3-benzoyl-5,7-di(methoxyethoxy)-coumarin, 3-benzoyl-5,7-di(allyloxy)coumarin, 3-benzoyl-7-dimethylaminocoumarin, 3-benzoyl-7-diethylaminocoumarin, 3-isobutyroyl-7-dimethylaminocoumarin, 5,7-dimethoxy-3-(1-naphthoyl)-coumarin, 5,7-diethoxy-3-(1-naphthoyl)-coumarin, 3-benzoylbenzo[f]coumarin, 7-diethylamino-3-thienoylcoumarin, 3-(4-cyanobenzoyl)-5,7-dimethoxycoumarin, 3-(4-cyanobenzoyl)-6,7-dipropoxycoumarin, 7-dimethylamino-3-phenylcoumarin, 7-diethylamino-3-phenylcoumarin, and the coumarin derivatives disclosed in JP-A Nos. 09-179299 and 09-325209, for example 7-[{4-chloro-6-(diethylamino)-S-triazine-2-yl}amino]-3-phenylcoumarin.

Examples of the 3-(aroylmethylene)-thiazolines include 3-methyl-2-benzoylmethylene-β-naphthothiazoline, 3-methyl-2-benzoylmethylene-benzothiazoline, 3-ethyl-2-propionylmethylene-β-naphthothiazoline.

Examples of the rhodanines include 4-dimethylaminobenzalrhodanine, 4-diethylaminobenzalrhodanine, 3-ethyl-5-(3-octyl-2-benzothiazolinylidene)-rhodanine, the rhodanine derivatives represented by formulas [1], [2], [7], disclosed in JP-A No, 08-305019.

Examples of compounds to be used other than the above-described compounds include acetophenone, 3-methoxyacetophenone, 4-phenylacetophenone, benzil, 4,4'-bis(dimethylamino)benzil, 2-acetyinaphthalene, 2-naphthaldehyde, dansyl acid derivatives, 9,10-anthraquinone, anthracene, pyrene, aminopyrene, perylene, phenanthrene, phenanthrenequinone, 9-fluorenone, dibenzosuberone, curcumin, xanthone, thiomichler's ketone, α-(4-dimethylaminobenzylidene) ketones, e.g. 2,5-bis(4-diethylaminobenzylidene)cyclopentanone, 2-(4-dimethylamino-benzylidene)-indan-1-one, 3-(4-dimethylamino-phenyl)-1-indan-5-yl-propenone, 3-phenylthiophthalimide, N-methyl-3,5-di(ethylthio)-phthalimide, N-methyl-3,5-di(ethylthio)phthalimide, phenothiazine, methylphenothiazine, amines, e.g. N-phenylglycine, ethyl 4-dimethylaminobenzoate, butoxyethyl 4-dimethylaminobenzoate, 4-dimethylaminoacetophenone, triethanolamine, methyldiethanolamine, dimethylaminoethanol, 2-(dimethylamino)ethyl benzoate, poly(propylenegylcol) -4-(dimethylamino)benzoate.

As the additive (d) added in the photosensitive composition of the present invention, at least one photosensitizer compound selected from the group consisting of benzophenone and its derivatives, thioxanthone and its derivatives, anthraquinone and its derivatives, and coumarin derivatives is preferred.

When the photosensitive composition of the present invention is photopolymerized, and then subjected to curing process by heating, as the additaive (d), the photosensitizer or a component which under thermal conditions forms free radicals is added to assist the curing process, as described for example in EP 245639.

The photosensitizer is not particularly limited and may be appropriately selected depending on the purpose. Those colored with titanium dioxide are particularly preferable.

Examples of the component which forms free radicals under thermal conditions include an azo compound such

as 2,2'-azobis(4-methoxy-2,4-dimethylvaleronitrile), a triazene, diazo sulfide, pentazadiene and a peroxy compound, such as a hydroperoxide or peroxycarbonate, for example t-butyl hydroperoxide.

The photosensitive composition of the present invention may further contain a photoreducable dye as the additive (d). Examples of the photoreducable dye include xanthene-, benzoxanthene-, benzothioxanthene, thiazine-, pyronine-, porphyrine- or acridine dyes, and trihalogenmethyl compounds which can be cleaved by irradiation. Moreover, the compounds described in EP 445624 can be also used.

As the additive (d), a flow improver, an adhesion promoter, and the like may also be used. The flow improver and an adhesion promoter are not particularly limited and may be appropriately selected from those known depending on the purpose. Examples thereof include vinyltrimethoxysilane, vinyltriethoxysilane vinyltris(2-methoxyethoxy)silane, N'(2-aminoethyl)-3-aminopropylmethyldimethoxysilane, N-(2-aminoethyl)-3-aminopropyltrimethoxysilane, 3-aminopropyltriethoxysilane, 3-glycidoxypropyltrimethoxysilane, 3-glycidoxypropylmethyldimethoxysilane, 2-(3,4-epoxycyclohexyl)ethyltrimethoxysilane, 3-chloropropylmethyldimethoxysilane, 3-chloropropyltrimethoxysilane, 3-methacryloxypropyltrimethoxysilane and 3-mereaptopropyltrimethoxysilane.

Moreover, surfactants, optical brighteners, pigments, dyes, wetting agents, levelling assistants, dispersants, aggregation preventers, antioxidants or fillers are further used as the additive (d).

When the photosensitive composition of the present invention is used as coatings, in order to cure thick and colored coatings, it is appropriate to add glass microspheres or pulverized glass fibers, as described for example in U.S. Pat. No. 5,013,768.

The additive (d) is preferably selected depending on the field of application and on properties required for this field. The additives described above are customary in the art and accordingly are added in amounts which are usual in the respective application.

< (e) Binder >

The binder (e) is preferably added when the photosensitive composition is a liquid or viscous substance. The binder is not particularly limited and may be suitably selected depending on the field of application and on properties required for this field, such as the capacity for development in aqueous and organic solvent systems, adhesion to substrates and sensitivity to oxygen.

The amount of the binder may, for example, be 2% by mass to 98% by mass, preferably 5% by mass to 95% by mass and especially 20% by mass to 90% by mass, relative to the all solid content of the photosensitive composition.

Examples of suitable binders are polymers having a molecular weight of 2,000 to 2,000,000, preferably 5,000 to 1,000,000.

Alkali developable binders are preferable. Examples thereof include acrylic polymer having carboxylic acid function as a pendant group, such as conventionally known copolymers obtained by copolymerizing an ethylenically unsaturated carboxylic acid such as (meth)acrylic acid, 2-carboxyethyl (meth)acrylic acid, 2-carboxypropyl (meth)acrylic acid, itaconic acid, crotonic acid, maleic acid and fumaric acid; ω-carboxypolycaprolactone mono(meth)acrylate with one or more monomers selected from esters of (meth)acrylic acid, such as methyl (meth)acrylate, ethyl (meth)acrylate, propyl (meth)acrylate, butyl (meth)acrylate, benzyl (meth) acrylate, 2-ethylhexyl (meth)acrylate, hydroxyethyl (meth) acrylate, hydroxypropyl (meth)acrylate, glycerol mono (meth)acrylate, tricyclo[5.2.1.0$^{2,6}$]decane-8-yl (meth)acrylate, glycidyl (meth)acrylate, 2-methlyglycidyl (meth)acrylate, 3,4-epoxybutyl(meth)acrylate, 6,7-epoxyheptyl(meth)acrylate; vinyl aromatic compounds, such as styrene, α-methyletyrene, vinyltoluene, p-chlorostyrene, vinylbenzylglycidylether; amide unsaturated compounds, (meth)acrylamide diacetone acrylamide, N-methylolacrylamide, N-butoxymethacrylamide; and polyolefin compounds, such as butadiene, isoprene, chloroprene and the like; methacrylonitrile, methyl isopropenyl ketone, mono-2-[(meth)acryloyloxy]ethyl succinate, N-phenylmaleimide, maleic anhydride, vinyl acetate, vinyl propionate, vinyl pivalate, polystyrene macromonomer, or polymethyl (meth)acrylate macromonomer.

Examples of copolymers include copolymers of acrylates and methacrylates with acrylic acid or methacrylic acid and with styrene or substituted styrene, phenolic resins, for example novolak, (poly)hydroxystyrene, and copolymers of hydroxystyrene with alkyl acrylates, acrylic acid and/or methacrylic acid.

Preferable examples of copolymers include copolymers of methyl methacrylate/methacrylic acid, copolymers of benzyl methacrylate/methacrylic acid, copolymers of methyl methacrylate/ethyl acrylate/methacrylic acid, copolymers of benzyl methacrylate/methacrylic acid/styrene, copolymers of benzyl methacrylate/methacrylic acid/hydroxyethyl methacrylate, copolymers of methyl methacrylate/butyl methacrylate/methacrylic acid/styrene, copolymers of methyl methacrylate/benzyl methacrylate/methacrylic acid/hydroxyphenyl methacrylate.

Examples of a solvent developable binder include poly(alkyl methacrylates), poly(alkyl acrylates), poly(benzylmethacrylate-co-hydroxyethylmethacrylate-co-methacrylic acid), poly(benzylmethacrylate-co-methacrylic acid); cellulose esters and cellulose ethers, such as cellulose acetate, cellulose acetobutyrate, methylcellulose, ethylcellulose; polyvinylbutyral, polyvinylformal, cyclized rubber, polyethers such as polyethylene oxide, polypropylene oxide and polytetrahydrofuran; polystyrene, polycarbonate, polyurethane, chlorinated polyolefins, polyvinyl chloride, vinyl chloride/vinylidene copolymers, copolymers of vinylidene chloride with acrylonitrile, methyl methacrylate and vinyl acetate, polyvinyl acetate, copoly(ethylene/vinyl acetate), polymers such as polycaprolactam and poly(hexamethylene adipamide), and polyesters such as poly(ethylene glycol terephtalate) and poly(hexamethylene glycol succinate) and polyimide binder resins.

The polyimide binder resin added to the photosensitive composition of the present invention can either be a solvent soluble polyimide or a polyimide precursor, for example, a poly(amic acid).

The photosensitive composition of the present invention preferably contains a copolymer of methacrylate and methacrylic acid as the binder (e).

When the photosensitive composition of the present invention used in a color filter, those described in JP-A No. 10-171119 are preferably used as the binder (e).

As the photosensitive composition of the present invention contains the oxime compound of the present invention as the photopolymerization initiator, it is excellent in storage stability without causing polymerization reaction during storage and generates active radicals upon irradiation of energy beam, especially, light, to thereby efficiently and quickly allow the polymerizable compounds to be polymerized. Thus, the photosensitive composition of the present invention can be used for various purposes, for example as printing ink, e.g. screen printing inks, inks for offset- or flexo printing, as a clear finish, as a white or colored finish, for example for wood or metal, as powder coating, as a coating material, in particular for paper, wood, metal or plastic, as a daylight-curable coating for the marking of buildings, materials, and roadmarking, for photographic reproduction techniques, for holographic recording materials, for image recording techniques or to produce printing plates which can be developed with organic solvents or with aqueous alkalis, for producing masks for screen printing, as dental filling compositions, as adhesives, as pressure-sensitive adhesives, as laminating resins, as etch resists, solder resists, electroplating resists, or permanent resists, both liquid and dry films, as photostructurable dielectric for printed circuit boards and electronic circuits, as resists to produce color filters for a variety of display applications or to generate structures in the manufacturing process of plasma-display panels and electroluminescence displays, (for example color filters described in U.S. Pat. No. 5,853,446, EP 863534, JP-ANos. 09-244230, 10-62980, 08-171863, U.S. Pat. No. 5,840,465, EP 855731, JP-A Nos. 05-271576, 05-67405) for the production of optical switches, optical lattices (interference lattice), light circuits, for producing three-dimensional articles by mass curing (UV curing in transparent moulds) or by the stereolithography technique, as is described, for example, in U.S. Pat. No. 4,575,330, to produce composite materials (for example styrenic polyesters, which may, if desired, contain glass fibers and/or other fibers and other auxiliaries) and other thick-layered compositions, for coating or sealing electronic components and integrated circuits, or as coatings for optical fibers, or for producing optical lenses, e.g. contact lenses or Fresnel lenses. The photosensitive composition of the present invention is further suitable for the production of medical equipments, auxiliaries or implants. Further, the photosensitive composition of the present invention is suitable for the preparation of gels with thermotropic properties, as for example described in DE 19700064 and EP 678534.

(Method for Forming Photosensitive Film)

The photosensitive composition of the present invention is applied on a substrate (base material) and dried to form a photosensitive layer, and moreover, a photosensitive layer formed from the photosensitive composition of the present invention is deposited on the substrate so as to form a photosensitive film. The photosensitive layer of the photosensitive film is laminated on the substrate for use. A solution of the photosensitive composition is applied onto the support so as to form the photosensitive layer. The support is preferably a poly(ethylene terephthalate). film, or a poly(ethylene terephthalate) film on which an oxygen-shielding layer and a peeling layer or the peeling layer and the oxygen-shielding layer are provided.

Generally, a protective film made of a synthetic resin is laminated on the surface of the photosensitive layer for a protection in handling.

Moreover, the photosensitive film may be formed by providing a polyalkali soluble thermoplastic resin layer and an intermediate layer on a poly(ethylene terephthalate) film, and then providing the photosensitive layer formed of the photosensitive composition thereon (JP-A No. 05-173320).

The protective film is peeled from the photosensitive layer for use, and the photosensitive layer is laminated on the substrate in the same manner as in first embodiment. Subsequently, peeling is carried out between the photosensitive layer and the support when the oxygen-shielding layer and the peeling layer are provided, between the peeling layer and the oxygen-shielding layer when the peeling layer and the oxygen-shielding layer are provided, and between the photosensitive layer and the support when either the peeling layer or the oxygen-shielding layer is not provided, and the support is removed.

When the photosensitive film is used for color filters, metal, glass, ceramics, and synthetic resin films are preferably used as the support. Of these, glass and synthetic resin films which are transparent and have excellent dimension stability are particularly preferred as the support.

The thickness of the photosensitive composition and photosensitive layer of the photosensitive film is preferably

0.1 μm to 50 μm, particular preferably 0.5 μm to 5 μm.

[Developing Step of Photosensitive Film]

When the photosensitive film is exposed and developed, as a developer an alkaline developer which is prepared by diluting an alkaline material is preferably used. The alkaline developer is more preferably used when the photosensitive composition contains an alkali soluble resin or alkali soluble monomer or oligomer. Moreover, a developer solution prepared by adding a small amount of a water-miscible organic solvent to a diluted alkaline material is preferably used as the developer for the photosensitive composition of the present invention.
Examples of suitable alkaline materials include alkali metal hydroxides (for example, sodium hydroxide and potassium hydroxide), alkali metal carbonates (for example, sodium carbonate and potassium carbonate), alkali metal bicarbonates (for example, sodium bicarbonate and potassium bicarbonate), alkali metal silicates (for example, sodium silicate and potassium silicate), alkali metal metasilicates (for example, sodium metasilicate and potassium metasilicate), triethanolamine, diethanolamine, monoethanolamine, morpholine, tetraalkylammonium hydroxides (for example, tetramethylammonium hydroxide), or trisodium phosphate.
The alkaline material preferably has a concentration of 0.01% by mass to 30% by mass, and a pH of 8 to 14.
Examples of the organic solvents which are miscible with water include methanol, ethanol, 2-propanol, 1-propanol, butanol, diacetone alcohol, ethylene glycol monomethyl ether, ethylene glycol monoethyl ether, ethylene glycol mono-n-butyl ether, diethyleneglycol dimethyl ether, propyleneglycol monomethyl ether acetate, ethyl-3-ethoxypropionate, methyl-3-methoxypropionate, n-butyl acetate, benzyl alcohol, acetone, methyl ethyl ketone, cyclopentanone, cyclohexanone, 2-heptanone, 2-pentanone, ε-caprolactone, γ-butylolactone, dimethylformamide, dimethylacetoamide, hexamethylphosphoramide, ethyl lactate, methyl lactate, ε-caprolactam, and N-methyl-pyrrolidinone.
The concentration of the organic solvent which is miscible with water is 0.1% by mass to 30% by mass.
Further, a conventionally known surfactant can be added in the developer. The concentration of the surfactant is preferably 0.001% by mass to 10 % by mass.
The photosensitive composition of the present invention can be also developed with organic solvents, including a mixture of two or more solvents, not containing alkaline compounds.
Examples of the organic solvents include methanol, ethanol, 2-propanol, 1-propanol, butanol, diacetone alcohol, ethylene glycol monomethyl ether, ethylene glycol monoethyl ether, ethylene glycol mono-n-butyl ether, diethyleneglycol dimethyl ether, propyleneglycol monomethyl ether acetate, ethyl-3-ethoxypropionate, methyl-3-methoxypropionate, n-butyl acetate, benzyl alcohol, acetone, methyl ethyl ketone, cyclopentanone, cyclohexanone, 2-heptanone, 2-pentanone, ε-caprolactone, γ-butylolactone, dimethylformamide, dimethylacetamide, hexamethylphosphoramide, ethyl lactate, methyl lactate, ε-caprolactam, and N-methylpyrrolidinone. Optionally, water can be added to the organic solvents up to a level at which still a transparent developer is obtained and at which sufficient solubility of the photosensitive composition in the photosensitive layer is maintained.
A form of the developer is not particularly limited and may be suitably selected from any forms known to the person skilled in the art depending on the purpose. Examples thereof include a form of a bath solution, puddle, and a spraying solution. In order to remove the non-cured portion of the photosensitive layer of the photosensitive composition, there can be combined the methods such as rubbing with a rotary brush and rubbing with a wet sponge.
Usually, the temperature of the developing solution is preferably at room temperature to 40°C. The developing time is variable according to the specific kind of the photosensitive composition, the alkalinity and temperature of the developing solution, and the type and concentration of the organic solvent in the case where it is added. Usually, it is preferably 10 seconds to 2 minutes. A rinsing step may be performed after the developing step.
A final heat treatment is preferably performed after the developing step. Accordingly, a support (base material) having a layer which is photopolymerized by exposing (hereinafter referred to as a photocured layer) is heated in an electric furnace and a drier, or the photocured layer is irradiated with an infrared lamp or heated on a hot plate. The heating temperature and time depend on the photosensitive composition used and the thickness of the formed photosensitive layer. In general, heating is preferably applied at 120°C to 250°C, for 5 minutes to 60 minutes.

(Specific Application of Photosensitive Composition)

Hereinafter, the photosensitive composition of the present invention will be explained with specific applications.

< Coating Material >

As a coating material, a mixture of a prepolymer with polyunsaturated monomers is frequently used, and the coating material may additionally include a monounsaturated monomer as well. It is the prepolymer here which primarily dictates the properties of the coating film, and the properties of the cured film can be adjusted by varying the prepolymer

appropriately.

The polyunsaturated monomer functions as a crosslinking agent which renders the film insoluble.

The monounsaturated monomer functions as a reactive diluent, which is used to reduce the viscosity without the need to employ a solvent.

The unsaturated polyester resins are usually used in two-component systems together with a monounsaturated monomer, preferably with styrene. For photoresists, for example polymaleimides, polychalcones or polyimides, as described in DE 2308830 are often used.

The photosensitive composition of the present invention can also be used for the polymerization of radiation-curable powder coating (hereinafter, also referred to as UV-curable powder coatings). The UV-curable powder coating contains at least a solid resin and a monomer containing a reactive double bond, for example maleates, vinyl ethers, acrylates, acrylamides and mixtures thereof.

The UV-curable powder coating can be formulated by mixing unsaturated polyester resins with solid acrylamides (for example methyl methylacrylamidoglycolate) and the novel oxime compound of the present invention. Such UV-curable powder coatings are as described, for example, in the paper "Radiation Curing of Powder Coating", Conference Proceedings, Radtech Europe 1993 by M. Wittig and Th. Gohmann.

The UV-powder coating can also contain the binder, as are described, for example, in DE 4228514 and in EP 636669. The UV-curable powder coating can also be formulated by mixing the unsaturated polyester resin with solid acrylates, methacrylates or vinyl ethers and with the novel oxime compound of the present invention. The powder coating may also contain a binder as described, for example, in DE 4228514 and in EP 636669.

The UV-curable powder coating may additionally contain a white or colored pigment. For example, preferably rutile titanium dioxide can be employed in concentrations of 50% by mass or less in order to give the UV-curable powder coating having excellent hiding power. The procedure normally contains electrostatic or frictional electric spraying of the powder onto the substrate, for example metal or wood, melting of the powder by heating. After a smooth film has been formed, the coating is UV-cured by ultraviolet and/or visible light, for example, using medium-pressure mercury lamps, metal halide lamps or xenon lamps.

A particular advantage of the UV-curable powder coating over the heat-curable powder coating is that the flow time after melting the powder particles can be delayed in order to ensure the formation of a smooth, high-gloss coating. In contrast to the heat-curable system, the radiation-curable powder coating can be formulated to melt at lower temperatures without decreasing their durability. For this reason, they are also suitable as coatings for heat-sensitive base materials, for example wood or plastics.

In addition to the oxime compound of the present invention, as the UV-curable powder coating formulations may also include UV absorbers as the additive. Appropriate examples are listed in the paper above in sections 1-8.

The powder coating is suitably used, for example, as the coating material for providing the protective layer on the base material, or the coating material on the base material for forming an image by exposing imagewise. Examples of the base material include wood, textiles, paper, ceramics, glass, plastics such as polyesters, polyethylene terephthalate, polyolefins or cellulose acetate, and metals such asAl, Cu, Ni, Fe, Zn, Mg or Co and GaAs, Si or $SiO_2$.

< Resist Material >

The photosensitive composition of the present invention is suitably used as a negative resist, having a very high sensitivity to light and being able to be developed in an aqueous alkaline medium without swelling. They are suitably used for the production of printing paper for relief printing, lithography printing, photogravure or of screen printing, for the production of relief copies, for example for the production of texts in braille, for the production of stamps, for use in chemical milling, and as a microresist in the production of integrated circuits. The photosensitive composition further may be used as photopatternable dielectric layer or coating, encapsulating material and isolating coating in the production of computer chips, printed boards and other electric or electronic components. The layer structure, processing conditions of the support, or substrate may be appropriately selected depending on the purpose.

The photosensitive composition of the present invention may also used for a photosensitive thermosetting resin composition and a method of forming a solder resist pattern by the use thereof, and more particularly used for a photosensitive thermosetting resin composition useful as a material for the production of printed circuit boards, the precision fabrication of metallic products, the etching of glass and stone products, the relief of plastic products, and the preparation of printing plates and particularly useful as a solder resist for printed circuit boards. Moreover, the photosensitive composition of the present invention is particularly preferably used in a method for forming a solder resist pattern by the steps of exposing the photosensitive composition or the photosensitive layer of the photosensitive composition through a photomask having a pattern and developing the unexposed part of the layer.

The solder resist is used during the soldering of a given part to a printed circuit board for the purpose of preventing molten solder from adhering to irrelevant portions and protecting circuits. It is, therefore, required to possess such properties as high adhesion, insulation resistance, resistance to soldering temperature, resistance to solvents,

resistance to alkalis, resistance to acids, and resistance to plating. By the use of the photosensitive composition of the present invention, these excellent properties can be obtained.

< Color Filter >

Because the photosensitive composition of the invention has a good thermal stability and is sufficiently resistant to inhibition by oxygen, it is particularly suitable for the production of color filters or color mosaic systems, such as described, for example, in EP 320264. The color filters are generally employed in the manufacturing of LCD displays, projection systems and image sensors. The color filters can be used, for example, for display and image scanner in television receivers, video monitors or computers, in flat panel display technology etc.

The color filter is generally prepared by forming red, green and blue pixels and a black matrix on a glass substrate. In these processes the photosensitive composition of the present invention can be employed.

A particularly preferred method of forming the color filter using the photosensitive composition of the present invention includes a step of adding of the coloring materials, dyes and pigments of red, green and blue colors to the photosensitive composition of the present invention, coating of the substrate with the photosensitive composition, drying of the coating with a short heat treatment so as to form a photosensitive layer, a step of patternwise exposing the photosensitive layer to actinic radiation and the step of developing the pattern in an aqueous alkaline developer solution and optionally a heat treatment step. Thus, by subsequently applying a red, green and blue colored photosensitive layer, in any desired order, on top of each other on the base material by repeating the photosensitive layer forimg step, the exposing step, and the developing step, a color filter layer with red, green and blue color pixels can be produced.

The developing step is carried out by washing out the areas, which were not polymerized in the exposing step, with a suitable alkali developing solution. This developing step is repeated to form the image having a plurality of colors.

In the photosensitive composition of the present invention, with a process in which at least one picture element is formed on a transparent substrate and then an exposure is given from a side of the transparent substrate, on which the above picture elements are not formed, the above picture elements can be utilized as a light-shielding mask. In this case, for example, in the case where an overall exposure is performed on the photosensitive layer, a position adjustment of a light-shielding mask is unnecessary and a concern on a position slippage thereof is removed. And, it is possible to cure all of the part on which the above picture elements are not formed. Further, in this case, it is possible as well to develop and remove a part of the portion on which the above picture elements are not formed by using partially a light-shielding mask.

Since in either case, no gap is formed between the picture elements which are formed formerly and those which are formed later, the photosensitive composition of the present invention is suitable for, for example, a material for forming the color filter. To be concrete, the coloring materials, dyes and pigments of red, green and blue colors are added to the photosensitive composition of the present invention, and the processes for forming an image are repeated to form the picture elements of red, green and blue colors. Then, the photosensitive composition to which, for example, the black coloring materials, dyes and pigments are added is provided on an overall face. An overall exposure (or a partial exposure via a light-shielding mask) can be provided on the photosensitive layer so as to form the picture elements of a black color all over the spaces (or all but a partial region of the light-shielding mask) between the picture elements of red, green and blue colors.

The pigment which can be comprised in the photosensitive composition for the color filter, including a pigmented color filter resist composition, is preferably a processed pigment, for example a powdery or pasty product prepared by finely dispersing a pigment into at least one resin selected from the group consisting of acrylic resin, vinyl chloride-vinyl acetate copolymer, maleic acid resin and ethyl cellulose resin. These pigments can be used for the photosensitive composition of the present invention used not only for the color filter but also for other application as necessary.

Examples of the red pigment include an anthraquinone pigment alone, a perylene pigment alone, or a mixture consisting of at least one of them and a disazo type yellow pigment or an isoindoline type yellow pigment, in particular C. I. Pigment Red 177 alone, C. I. Pigment Red 155 alone or a mixture containing at least one selected from the group consisting of C. I. Pigment Red 177, C. I. Pigment Red 155 and C. I. Pigment Yellow 83 or C. I. Pigment Yellow 139 ("C.I." refers to the Color Index, known to the person skilled in the art and publicly available).

Further suitable examples of the pigments include C.I. Pigment Red 105, 144, 149, 176, 177, 185, 202, 209, 214, 222, 242, 254, 255, 264, 272 and C.I. Pigment Yellow 24, 31, 53, 83, 93, 95, 109, 110, 128, 129, 138, 139, 166 and C.I. Pigment Orange 43.

Examples of the green pigment include a halogenated phthalocyanine pigment alone or a mixture of the halogenated phthalocyanine pigmen with a disazo yellow pigment or an isoindoline yellow pigment, in particular C. I. Pigment Green 7 alone, C. I. Pigment Green 36 alone, C. I. Pigment Green 37 alone or a mixture containing at least one selected from the group consisting of C. I. Pigment Green 7, C. I. Pigment Green 36, C. I. Pigment Green 37, C.I. Pigment Green 136 and C. I. Pigment Yellow 83 or C. I. Pigment Yellow 139.

Examples of other suitable green pigments include C.I. Pigment Green 15 and 25.

Examples of the blue pigments include phthalocyanine pigments, used either alone or in combination with a dioxazine violet pigment, for instance, a combination of C. I. Pigment Blue 15:3 and C. I. Pigment Violet 23.

Further examples of the blue pigments include C.I. Blue 15:3, 15:4, 15:6, 16 and 60, i.e. Phthalocyanine C.I. Pigment Blue 15:3, or Phthalocyanine C.I. Pigment Blue 15:6. Examples of other suitable pigments include C.I. Pigment Blue 22, 28, C.I. Pigment Violet 14, 19, 23, 29, 32, 37, 177 and C.I. Orange 73.

The pigment of the black matrix photopolymeric composition preferably contains at least one member selected from the group consisting of carbon, titanium black and iron oxide. However, a mixture of other pigments which, in total, give the black appearance, can also be used. For example, C.I. Pigment Black 1 and 7 can be used alone or in combination.

For any color, these pigments may be used alone or in combination. Especially suitable in the color filter applications are powdery processed pigments prepared by finely dispersing the above mentioned pigments into a resin.

The concentration of the pigment in the total solid component (pigments of various colors and resin) is for example in the range of 5% by mass to 80% by mass, in particular in the range of 20% by mass to 45% by mass.

The pigments in the photosensitive composition for the color filter preferably have an average particle diameter smaller than the wavelength of visible light (400 nm to 700 nm), and particularly preferably have an average particle diameter of less than 100 nm.

If necessary, the pigments may be stabilized in the photosensitive composition by pretreatment of the pigments with a dispersant to improve the dispersion stability of the pigment in the liquid formulation.

Preferably, the photosensitive composition for the color filter additionally contains at least one addition polymerizable monomeric compound as the photopolymerizable compounds (a).

As the addition polymerizable monomeric compound, the following compounds can be used alone or in combination with the other monomers as the addition-polymerizable monomer having an ethylenically unsaturated double bond used in the present invention. Specific examples thereof include t-butyl(meth)acrylate, ethylene glycol di(meth) acrylate, 2-hydroxypropyl (meth)acrylate, triethylene glycol di(meth)acrylate, trimethylolpropane tri(meth)acrylate, 2-ethyl-2-butylpropanediol di(meth)acrylate, pentaerythritol tri(meth)acrylate, pentaerythritol tetra(meth)acrylate, dipentaerythritol hexa(meth)acrylate, dipentaerythritol penta(meth)acrylate, polyoxyethylated trimethylolpropane tri(meth)acrylate, tris(2-(meth)acryloyloxyethyl)isocyanurate, 1,4-diisopropenyl-benzene, 1,4-dihydroxybenzene (meth)acrylate, decamethylene glycol di(meth)acrylate, styrene, diallyl fumarate, triallyl trimellitate, lauryl (meth)acrylate, (meth)acrylamide, and xylenebis(meth)acrylamide.

Further, there can be used a reaction product of a compound having a hydroxyl group, such as 2-hydroxyethyl (meth)acrylate, 2-hydroxypropyl (meth)acrylate, and polyethylene glycol mono(meth)acrylate with diisocyanate such as hexamethylenediisocyanate, toluenediisocyanate, and xylenediisocyanate.

Particularly preferred are pentaerythritol tetraacrylate, dipentaerythritol hexaacrylate, dipenta-erythritol pentaacrylate, and tris(2-acyloyloxyethyl)-isocyanurate.

In the photosensitive composition for the color filter, the whole amount of the monomers contained in the photopolymerizable composition is preferably 5% by mass to 80% by mass, in particular 10% by mass to 70% by mass based on the whole solid contents of the photosensitive composition, i.e. the amount of all components without the solvent(s).

As the binder (e) a compound which is soluble in an alkaline aqueous solution and insoluble in water is preferably added in the photosensitive composition for the color filter. Examples thereof include a homopolymer of a polymerizable compound having at least one acid group and at least one polymerizable unsaturated bond in the molecule, or a copolymer of two or more kinds thereof, and a copolymer of at least one polymerizable compound having at least one unsaturated bond copolymerizable with these compounds and containing no acid group, can be used. Such compounds can be obtained by copolymerizing at least one low molecular compound having at least one acid group and at least one polymerizable unsaturated bond in the molecule with at least one polymerizable compound having at least one unsaturated bond copolymerizable with these compounds and containing no acid group.

Examples of acids groups include a -COOH group, a $-SO_3H$ group, a $-SO_2NHCO$ group, a phenolic hydroxy group, a $-SO_2NH$ group, and a -CO-NH-CO group. Among those, a high molecular compound having a -COOH group is particularly preferred.

As the binder in the photosensitive composition for the color filter, those containing, as addition polymerizable monomer units, at least an unsaturated organic acid compound such as acrylic acid, methacrylic acid and the like, and the organic polymer containing an alkali soluble copolymer are preferable.

It is preferred to use as a further co-monomer for the polymer an unsaturated organic acid ester compound such as methyl acrylate, ethyl (meth)acrylate, benzyl (meth)acrylate, styrene and the like to balance properties such as alkaline solubility, adhesion rigidity, chemical resistance etc.

The organic polymer binder may either be a random copolymer or a block copolymer, for example, such as described in U.S. Pat. No. 5,368,976.

Examples of polymerizable compounds having at least one acid group and at least one polymerizable unsaturated bond in the molecule include the following compounds:

acrylic acid, methacrylic acid, itaconic acid, crotonic acid, maleic acid, vinylbenzoic acid, and cinnamic acid are examples of the polymerizable compounds having at least one -COOH group and one or more polymerizable unsaturated bond in a molecule.

Examples of the polymerizable compounds having at least one $-SO_3H$ group and at least one polymerizable unsaturated bond include vinylbenzenesulfonic acid and 2-(meth)acrylamide-2-methylpropanesulfonic acid

Examples of the polymerizable compounds having at least one $-SO_2NHCO$ group and one or more polymerizable unsaturated bond include N-methylsulfonyl (meth)acrylamide, N-ethylsulfonyl (meth)acrylamide, N-phenylsulfonyl (meth)acrylamide, and N-(p-methylphenylsulfonyl) (meth)acrylamide.

Examples of polymerizable compounds having at least one phenolic hydroxy groups and at least one polymerizable unsaturated bonds in a molecule include hydroxyphenyl (meth)acrylamide, dihydroxyphenyl (meth)acrylamide, hydroxyphenyl-carbonyloxyethyl (meth)acrylate, hydroxyphenyloxyethyl (meth)acrylate, hydroxyphenylthioethyl (meth) acrylate, dihydroxyphenylcarbonyloxyethyl (meth)acrylate, dihydroxyphenyloxyethyl (meth)acrylate, and dihydroxy-phenylthioethyl (meth)acrylate.

Examples of the polymerizable compounds having at least one $-SO_2NH$ group and at least one polymerizable unsaturated bond in the molecule include compounds represented by Formula (a) or (b):

$$CH_2=CHA_1-Y_1-A_2-SO_2-NH-A_3 \qquad \text{Formula (a)}$$

$$CH_2=CHA_4-Y_2-A_5-NH-SO_2-A_6 \qquad \text{Formula (b)}$$

In Formulas (a) and (b), $Y_1$ and $Y_2$ each represents -COO-, $-CONA_{7-}$, or a single bond; $A_1$ and $A_4$ each represents H or $CH_3$; $A_2$ and $A_5$ each represents $C_1$-$C_{12}$ alkylene optionally having a substituent, which is cycloalkylene, arylene, or aralkylene, or $C_2$-$C_{12}$ alkylene into which an ether group and a thioether group are inserted, cycloalkylene, arylene, or aralkylene; $A_3$ and $A_6$ each represents H, $C_1$-$C_{12}$ alkyl optionally having a substituent, which is a cycloalkyl group, an aryl group, or an aralkyl group; and $A_7$ represents H, $C_1$-$C_{12}$ alkyl optionally having a substituent, which is a cycloalkyl group, an aryl group, or an aralkyl group.

The polymerizable compounds having at least one -CO-NH-CO- group and at least on polymerizable unsaturated bond include maleimide and N-acryloyl-acrylamide. These polymerizable compounds become the high molecular compounds containing a -CO-NH-CO- group, in which a ring is formed together with a main chain by polymerization.

Further, a methacrylic acid derivative and an acrylic acid derivative each having a -CO-NH-CO- group can be used as well. Such methacrylic acid derivatives and the acrylic acid derivatives include, for example, a methacrylamide derivative such as N-acetylmethacrylamide, N-propionylmethacrylamide, N-butanoylmethacrylamide, N-pentanoylmethacrylamide, N-decanoylmethacrylamide, N-dodecanoylmethacrylamide, N-benzoylmethacrylamide, N-(p-methylbenzoyl)methacryl-amide, N-(p-chlorobenzoyl)methacrylamide, N-(naphthyl-carbonyl)methacrylamide, N-(phenylacetyl)-methacrylamide, and 4-methaeryloylaminophthalimide, and an acrylamide derivative having the same substituent as these. These polymerizable compounds polymerize to be compounds having a -CO-NH-CO- group in a side chain.

Examples of polymerizable compounds having one or more polymerizable unsaturated bond and containing no acid group include a compound having a polymerizable unsaturated bond, selected from (meth)acrylates, (meth) acrylamides, acxylic compounds, vinyl ethers, vinyl esters, styrenes, and crotonates, and specifically, include (meth) acrylates such as alkyl (meth)acrylate or substituted alkyl (meth)acrylate (for example, methyl (meth)acrylate, ethyl (meth)acrylate, propyl (meth)acrylate, isopropyl (meth)acrylate, butyl (meth)acrylate, amyl (meth)acrylate, hexyl (meth) acrylate, cyclohexyl (meth)acrylate, ethylhexyl (meth)acrylate, octyl (meth)acrylate, t-octyl (meth)acrylate, chloro-ethyl (meth)acrylate, allyl (meth)acrylate, 2-hydroxy-ethyl (meth)acrylate, 2-hydroxypropyl (meth)acrylate, 4-hydroxybutyl (meth)acrylate, 2,2-dimethyl-3-hydroxy-propyl (meth)acrylate, 5-hydroxypentyl (meth)acrylate, trimethyfolpropane mono (meth)acrylate, pentaerythritol mono (meth)acrylate, benzyl (meth)acrylate, methoxy-benzyl (methacrylate, chlorobenzyl (meth)acrylate, furfuryl (meth)acrylate, tetrahydrofurfuryl (meth)acrylate, phenoxyethyl (meth)acrylate, and aryl (meth) acrylate (for example, phenyl (meth)acrylate, cresyl (meth)acrylate, and naphthyl (meth)acrylate); (meth)acrylamides such as (meth)acrylamide, N-alkyl(meth)acrylamide (the alkyl group includes, for example, methyl, ethyl, propyl, butyl, t-butyl, heptyl, octyl, ethylhexyl, cyclohexyl, hydroxyethyl, and benzyl), N-aryl(meth)acrylamide (the aryl group includes, for example, phenyl, tolyl, nitrophenyl, naphthyl, and hydroxyphenyl), N,N-dialkyl(meth)acrylamide (the alkyl group includes, for example, methyl, ethyl, butyl, isobutyl, ethylhexyl, and cyclohexyl), N,N-diaryl (meth)acrylamide (the aryl group includes, for example, phenyl), N-methyl-N-phenyl (meth)acryl-amide, N-hydroxyethyl-N-methyl (meth)acrylamide, N-2-acetoamidethyl-N-acetyl(meth)acrylamide, N-(phenyl-sulfonyl)(meth)acrylamide, and N-(p-methylphenyl-sulfonyl)(meth)acrylamide.

Moreover, examples thereof include allyl compounds such as allyl esters (for example, allyl acetate, allyl caproate, allyl caprylate, allyl laurate, allyl palmitate, allyl stearate, allyl benzoate, allyl acetoacetate, and allyl lactate), and allyloxyethanol; vinyl ethers such as alkyl vinyl ether (the alkyl group includes, for example, hexyl, octyl, decyl,

ethylhexyl, methoxyethyl, ethoxyethyl, chloroethyl, 1-methyl-2,2-dimethylpropyl, 2-ethylbutyl, hydroxyethyl, hydroxyethoxyethyl, dimethylaminoethyl, diethylaminoethyl, butylaminoethyl, benzyl, and tetrahydrofurfuryl), and vinyl aryl ether (the aryl group includes, for example, phenyl, tolyl, chlorophenyl, 2,4-dichloro-phenyl, naphthyl, and anthranyl).

Examples thereof include vinyl esters such as vinyl butylate, vinyl isobutylate, vinyl trimethylacetate, vinyl diethylacetate, vinyl barate, vinyl caproate, vinyl chloroacetate, vinyl dichloroacetate, vinyl methoxyacetate, vinyl butoxyacetate, vinyl phenylacetate, vinyl acetoacetate, vinyl lactate, vinyl-b-phenylbutylate, vinyl cyclohexylcarboxylate, vinyl benzoate, vinyl salicylate, vinyl chlorobenzoate, vinyl tetrachlorobenzoate, and vinyl naphthoate; styrenes such as styrene, alkylstyrene (for example, methylstyrene, dimethylstyrene, trimethyletyrene, ethylstyrene, diethylstyrene, isopropylstyrene, butylstyrene, hexylstyrene, cyclohexylstyrene, decylstyrene, benzylstyrene, chloromethylstyrene, trifluoromethylstyrene, ethoxymethylstyrene, and acetoxymethylstyrene), alkoxystyrene (for example, methoxystyrene, 4-methoxy-3-methylstyrene, and dimethoxystyrene), and halogenostyrene (for example, chlorostyrene, dichlorostyrene, trichlorostyrene, tetrachlorostyrene, pentachlorostyrene, bromostyrene, dibromostyrene, iodostyrene, fluorostyrene, trifluorostyrene, 2-bromo-4-trifluoromethylstyrene, and 4-fluoro-3-trifluoromethyl-styrene).

Further more exemples thereof include crotonates such as alkyl crotonate (for example, butyl crotonate, hexyl crotonate, and glycerine monocrotonate); dialkyl itaconates (for example, dimethyl itaconate, diethyl itaconate, and dibutyl itaconate); dialkyl maleates or fumarates (for example, dimethyl maleate and dibutyl fumarate); and (meth) acrylonitrile.

There can be used as well hydroxystyrene homo- or copolymers or novolak phenol resins, for example, poly (hydroxystyrene) and poly(hydroxystyrene-co-vinylcyclohexanol), novolak resins, cresol novolak resins, and halogenated phenol novolak resins. More specifically, it includes, for example, the methacrylic acid copolymers, the acrylic acid copolymers, the itaconic acid copolymers, the crotonic acid copolymers, the maleic anhydride co-polymers, for example, with styrene as a co-monomer, and maleic acid copolymers, and partially esterified maleic acid copolymers each described in, for example, JP-B No. 59-44615 (the term "'JP-B" as used herein refers to an Japanese patent application publication), JP-A Nos. 54-34327, 68-12577, and 54-25957, 59-53836, 59-71048, 60-159743, 60-258689, 1-152449, 2-1999403, and 2-199404, and which copolymers can be further reacted with an amine, as disclosed in U.S. Pat. No. 5,650,263.

Further, a cellulose derivative having a carboxyl group on a side chain can be used, and particularly preferred are copolymers of benzyl (meth)acrylate and (meth)acrylic acid and copolymers of benzyl (meth)acrylate, (meth)acrylic acid and other monomers, for example as described in U.S. Pat. No. 4,139,391, JP-B No. 59-44615, JP-A No. 60-159743 and 60-258539.

With respect to those having carboxylic acid groups among the above organic polymers, it is possible to react some or all of the carboxylic acid groups with glycidyl(meth)acrylate or an epoxy(meth)acrylate to obtain photopolymerizable organic polymers for the purpose of improving the photosensitivity, coating film strength, the coating solvent and chemical resistance and the adhesion to the substrate. Examples of the photopolymerizable organic polymers include those disclosed in, JP-B Nos. 50-34443 and 50-34444, U.S. Pat. No. 5,153,095, T. Kudo et al. in J. Appl. Phys., Vol. 37 (1998), p. 3594-3603, U.S. Pat. No. 5,677,385, and U.S. Pat. No. 5,650,233.

The mass-average molecular weight of the binders is preferably 500 to 1,000,000, more preferably 3,000 to 1,000,000, and particularly preferably 5,000 to 400,000.

These binders may be used alone or as a mixture of two or more types.

The amount of the binder in the photosensitive composition is preferably 10% by mass to 95% by mass, more preferably 15% by mass to 90% by mass based on the total solid components.

Further, in the color filter an ionic impurity-scavenger, e.g. an organic compound having an epoxy group may be contained. The amount of the ionic impurity scavenger in the photosensitive composition is preferably in the range from 0.1% by mass to 10% by mass in the total solid component.

Examples of color filters, especially with respect to the above described combinations of pigments and ionic impurity scavenger are given in EP 320264. That is, the oxime compound of the present invention as the photoinitiators can be added instead of the photopolymerization initiator in the photosensitive composition of the color filter described in EP 320264.

The photosensitive composition of the present invention can contain additionally a crosslinking agent which is activated by an acid, for example as described in JP-A No. 10-221843, and a compound which generates acid thermally or by actinic radiation and which activates a crosslinking reaction.

The photosensitive composition of the present invention can also contain a latent pigment which is transformed into a finely dispersed pigment during the heat treatment of the latent pigment containing photosensitive pattern or coating. The heat treatment can be performed after exposure or after development of the latent pigment-containing photosensitive layer. Such latent pigments are soluble pigment precursors which can be transformed into insoluble pigments by means of chemical, thermal, photolytic or radiation induced methods as described, for example, in U.S. Pat. No. 5,879,855. This transformation of such latent pigments can be enhanced by adding a compound which generates acid upon actinic exposure or by adding an acidic compound to the photosensitive composition. Therefore, the color

filter can also be produced, which contains the latent pigment in the photosensitive composition of the present invention.

Examples of the color filter, and the formulation and processing conditions of the photosensitive composition for the color filter include those given by T. Kudo et al., Jpn. J. Appl. Phys. Viol. 37 (1998) 3594; T. Kudo et al., J. Photopolym. Sci. Technol. Vol 9 (1996) 109; K. Kobayashi, Solid State Techno. November 1992, p. S15-S18; U.S. Pat. Nos. 5,368,976; 5,800,952; 5,882,843; 5,879,855; 5,866,298; 5,863,678; JP-A No. 06-230212; EP 320264; JP-A Nos. 09-269410; 10-221843; 01-090516; 10-171119, U.S. Pat. Nos. 5,821,016, 5,847,015, 5,882,843, 6,719,008, EP 881541, or EP 902327.

The oxime compound of the present invention can be used for the photosensitive composition for the color filter, for example, such as the photosensitive composition given as examples above, or can partially or fully replace the known photoinitiator in the photosensitive composition. It is understood by a person skilled in the art that the use of the novel oxime compound of the present invention is not limited to the specific binder resins, crosslinkers and formulations of the photosensitive composition for the color filter, the novel oxime compound can be used in various applications as a radical polymerizable component, in combination with a dye or color pigment or latent pigment to form a photosensitive color filter ink or color filter resist.

Accordingly, the color filter is preferably prepared by providing red, green and blue (RGB) color elements and, optionally a black matrix, all formed from the photosensitive composition containing a photosensitive resin and a pigment on a transparent substrate and providing a transparent electrode either on the surface of the substrate or on the surface of the color filter layer formed from the photosensitive composition. The photosensitive composition contains a polyfunctional acrylate monomer, an organic polymer binder and the oxime compound represented by Genearal Formula (1) as described above as a photopolymerization initiator. The monomers and binder components, and pigments are as described above can be used. In the production of the color filter the transparent electrode layer can be formed either by applying the composition on the surface of the transparent substrate or by applying the composition on the surface of the red, green and blue color filters and the black matrix. The transparent substrate is for example a glass substrate which can additionally have an electrode layer on its surface.

It is preferred to apply a black matrix between RGB three color picture elements in order to improve the contrast of the color filter.

Instead of forming a black matrix using the photosensitive composition and patterning the black photosensitive composition photolithographically by patternwise exposure (i.e. through a suitable mask) to form the black pattern separating the red green and blue colored areas on the transparent substrate, it is alternatively possible to use an inorganic black matrix. Such inorganic black matrix can be formed from deposited (i.e. sputtered) metal (i.e. chromium) film on the transparent substrate by a suitable imaging process, for example utilizing photolithographic patterning by means of an etch resist, etching the inorganic layer in the areas not protected by the etch resist and then removing the remaining etch resist.

Further, known different methods of forming the black matrix in the color filter production process include as follows: the photosensitive composition for black matrix can either be applied directly on the transparent substrate prior to formation of the red, green and blue (RGB) color filter as already mentioned above, or it can be applied after the RGB color filter is formed on the substrate.

In a different embodiment of a color filter for a liqid crystal display, according to U.S. Pat. No. 5,626,796, the black matrix can also be applied on a surface of the substrate opposite to the surface on which the RGB color filter layer is provided. Thus, the black matrix is separate from the RGB color filter layer due to a liquid crystal layer.

When the transparent electrode layer is deposited after applying the RGB color filter layer and optionally, the black matrix, an additional overcoat film as a protective layer can be applied on the RGB color filter layer prior to deposition of the electrode layer, for example, as described in U.S. Pat. No. 5,650,263.

As a material of the protective layer, photosensitive resins or thermosetting resin compositions are employed. The photosensitive composition of the present invention can also be used as the material of the protective layer so as to obtain a protective layer which is excellent in flatness, hardness, chemical and thermal resistance, transparency especially in a visible region, adhesion to a substrate, and to suitabily form a transparent conductive film, e.g., an ITO film, thereon. In the production of the protective layer, there has been a demand that unnecessary parts of the protective layer, for example on scribing lines for cutting the substrate and on bonding pads of solid image sensors should be removed from the substrate as described in JP-A Nos. 57-42009, 1-130103 and 1-134306. In this regard, it is difficult to selectively form a protective layer with high precision in areas other than on scribing lines for cutting the substrate and on bonding pads of solid image sensors, using the conventionally known thermosetting resins. The photosensitive composition of the present invention, however, allows to easily remove the unnecessary parts of the protective layer by photolithography.

The color filter produced by using the photosensitive composition of the present invention is not particularly limited to the above-described structure of production methods, as long as it can be used for generating picture elements of red, green and blue and a black matrix, and any known processing methods can be used, a protective layer, a transparent conductive layer, and additional layers can be deposited, and any design of the color filter may be employed.

The photosensitive composition of the present invention can suitably be used for forming a color filter but will not be limited to this application. It is useful as well for a recording material, a resist material, a protective layer, a dielectric layer, in display applications and display elements, a paint, and a printing ink.

< Liquid Crystal Display >

The photosensitive composition of the present invention is also suitable for producing an interlayer insulating layer or dielectric layer in a liquid crystal display, and more particularly in a reflection type liquid crystal display including an active matrix type display having a thin film transistor (TFT) as a switching device, and a passive matrix type without a switching device.

In recent years, liquid crystal displays have, for example, been widely used for pocket-type TV sets and terminal devices for communication by virtue of its small thickness and light weight. A reflection type liquid crystal display without necessity of using a back light is in particular in demand because it is ultra-thin and light-weight, and it can significantly reduce power consumption. However, even if a back light is removed out of a presently available transmission type color liquid crystal display and a light reflection plate is added to a substrate of the display, it would cause a problem in that the efficiency of utilizing lights is low, and it is not possible to have practical brightness.

As a solution to this problem, there have been suggested various reflection type liquid crystal displays for enhancing an efficiency of utilizing lights. For instance, a certain reflection type liquid crystal display is designed to include a pixel electrode having reflection function.

The reflection type liquid crystal display includes an insulating substrate and an opposing substrate spaced away from the insulating material. A space between the substrates is filled with liquid crystals. A gate electrode is formed on the insulating substrate, and both the gate electrode and the insulating substrate are covered with a gate insulating film. A semiconductor layer is then formed on the gate insulating film above the gate electrode. A source electrode and a drain electrode are also formed on the gate insulating film in contact with the semiconductor layer. The source electrode, the drain electrode, the semiconductor layer, and the gate electrode cooperate with one another to thereby constitute a bottom gate type TFT as a switching device.

An interlayer insulating film is formed covering the source electrode, the drain electrode, the semiconductor layer, and the gate insulating film therewith. A contact hole is formed throughout the interlayer insulating film on the drain electrode. A pixel electrode made of aluminum is formed on both the interlayer insulating film and an inner sidewall of the contact hole. The drain electrode of the TFT is eventually in contact with the pixel electrode through the interlayer insulating film. The interlayer insulating layer is generally designed to have a roughened surface by which the pixel electrode acts as a reflection plate which diffuses lights to get a wider angle for viewing (angle of visibility).

The reflection type liquid crystal display remarkably enhances an efficiency of using lights by virtue that the pixel electrode acts as a light reflection plate.

In the above-mentioned reflection type liquid crystal display, the interlayer insulating film is designed to have projections and recesses by photolithography. To form and control a fine shape of the projections and recesses in micrometer order for surface roughness and to form contact holes, photolithography methods using positive and negative photoresists are used. For these resists the photosensitive composition of the present invention is especially suitable.

< Spacer for Liquid Crystal Display >

The photosensitive composition of the present invention can further be used for producing a spacer, which controls a cell gap of the liquid crystal part in a liquid crystal display panel. Since the properties of light transmitted or reflected through the liquid crystal layer in a liquid crystal display are dependent on the cell gap, the thickness accuracy and uniformity over the pixel array are critical parameters for the performance of the liquid crystal display unit. In a liquid crystal cell, the spacing between the substrates in the liquid cell is maintained constant by sparsely distributing glass or polymer spheres about several micrometers in diameter as spacers between the substrates. The spacers are thus held between the substrates to maintain the distance between the substrates at a constant value. The distance is determined by the diameter of the spacers. The spacers assure the minimum spacing between the substrates; i.e., they prevent a decrease in distance between the substrates.

However, they cannot prevent the substrates from being separated apart from each other, i.e., the increase in distance between the substrates. Additionally, this method of using spacer beads has problems of the uniformity in the diameter of spacer beads and difficulty in the even dispersion of spacer beads on the panel, as well as nonuniform orientation and decrease in brightness and/or optical aperture depending on the location of spacers on pixel array region.

Liquid crystal displays having a large image display area have recently been attracting much attention. However, the increase in the area of a liquid crystal cell generally produces the distortion of the substrates constituting the cell. The layer structure of the liquid crystal tends to be destroyed due to the deformation of the substrate. Thus, even when spacers are used for maintaining the spacing between the substrates constant, a liquid crystal display having a large

image display area is unfeasible because the display experiences disturbances.

Instead of the above spacer sphere dispersion method, a method of forming columns in the cell gap as spacers has been proposed. In this method, columns of a resin are formed as spacers in the region between the pixel array region and the counter electrode to form a prescribed cell gap. This formation is performed by photolithography using the photosensitive composition having adhesive property, which is commonly used for instance, in the production process of color filters. This method is advantageous compared with the conventional method using spacer beads in the points that location, number and height of the spacers may be controlled freely, In a color liquid crystal display panel, such spacers are formed in the nonimaging area under black matrix of color filter elements. Therefore, the spacers formed using photosensitive composition do not decrease brightness and optical aperture.

The photosensitive composition for producing a protective layer with a spacer for color filter is disclosed in JP-A No. 2000-81701 and film type photosensitive composition (photoresist) for a spacer is also disclosed in JP-A Nos. 11-174459 and 11-174464. As described in the documents, liquid and film photoresists, contain at least an alkaline or acid soluble binder polymer, a radically polymerizable monomer, and a radical initiator. As necessry, thermally crosslinkable components such as epoxide and carboxylic acid may additionally be included.

The steps of forming a spacer using the photosensitive composition of the present invention are as follows: a photosensitive composition is applied to a substrate, for instance a color filter panel and after the substrate is prebaked, it is exposed to light through a mask. Then, the substrate is developed with a developer and patterned to form a desired spacer. When the photosensitive composition contains a thermosetting component, usually a postbaking is carried out to thermally cure the pattern.

The photosensitive composition of the present invention is suitable for producing the spacer for liquid crystal display as described above because of its high sensitivity.

< Microlens Array >

The photosensitive composition of the present invention is also suitable for producing a microlens array used in a liquid crystal display panel, an image sensor and the like. The Microlens is a microscopic passive optical component that fit on active optoelectronic devices such as detectors, displays, and light emitting devices (light-emitting diodes, transversal and vertical cavity lasers) to improve their optical input or output quality. The areas of applications are wide and cover areas such as telecommunications, information technology, audio-visual services, solar cells, detectors, solid-state light sources, and optical interconnects.

At present, optical systems use a variety of techniques to obtain efficient coupling between microlenses and microoptical devices.

The microlens array is used for condensing illuminating light on the picture element region of a nonluminescent display device, such as a liquid crystal display device, to increase the brightness of the display, for condensing incident light or as means for forming an image on the photoelectric conversion regions of a line image sensor used for example in facsimiles and the like to improve the sensitivity of these devices, and for forming an image to be printed on a photosensitive means used in liquid crystal printers or light emitting diode (LED) printers.

The most common application is their use to improve the efficiency of photodetector arrays of a solid-state image sensing device such as a charge coupled device (CCD). In a detector array, the collection of as much light as possible in each detector element or pixel is demanded. When a microlens is put on top of each pixel, the lens collects incoming light and focuses it onto an active area that is smaller than the size of the lens.

According to the prior art, the microlens arrays can be produced by a variety of the following methods; (1) A method for obtaining convex lenses wherein a pattern of the lenses in a planar configuration is drawn on a thermoplastic resin by a conventional photolithographic technique or the like, and then the thermoplastic resin is heated to a temperature above the softening point of the resin to have flowability, thereby causing a sag in the pattern edge (so called "reflowing") (see, e.g., JP-A Nos. 60-38989, 60-165623, 61-67003, and 2000-39503). In this method, when the thermoplastic resin used is photosensitive, a pattern of the lenses can be obtained by exposure of this resin to light. (2) A method for forming a plastic or glass material by the use of a mold or a stamper. As lens material, a photocurable resin and a thermosetting resin can be used in this method (see, e.g., International Publication No. WO 99/38035). (3) A method for forming convex lenses on the basis of a phenomenon in which when a photosensitive composition is exposed to light in a desired pattern by the use of an aligner, unreacted monomers move from the unexposed portions to the exposed portions, resulting in a swell of the exposed portions (see, e.g., Journal of the Research Group in Microoptics Japanese Society of Applied Physics, Colloquium in Optics, Vol. 5, No. 2, pp. 118-123 (1987) and Vol. 6, No. 2, pp. 87-92(1988)).

On the upper surface of a supporting substrate, a photosensitive layer is formed from the photosensitive composition. Thereafter, with the use of a separate shading mask, the upper surface of the photosensitive layer is illuminated with light from a mercury lamp or the like, so that the photosensitive layer is exposed to the light. As a result, the exposed portions of the photosensitive layer swell into the shape of convex lenses to form the light condensing layer having a plurality of microlens. (4) A method for obtaining convex lenses wherein a photosensitive composition is exposed

to light by a proximity exposure technique in which a photomask is not brought into contact with the resin, to cause a blur at the pattern edge, so that the amount of photochemical reaction products is distributed depending upon the degree of blurring at the pattern edge (see, e.g., JP-A No. 61-163602). (5) A method for generating a lens effect wherein a photosensitive composition is exposed to light with a particular intensity distribution to form a distribution pattern of refractive index depending upon the light intensity (see, e.g., JP-A Nos. 60-72927 and 60-166946).

The photosensitive composition of the present invention can be used as a photocurable resin composition to form microlens arrays in any one of the above-mentioned methods (1) to (5).

As other methods, there are many methods for forming the microlens from the thermoplastic resin such as a photoresist. For example, reference can be made to a publication by Popovic et al. SPIE 898, pp.23-25 (1988). The technique, named reflow technique, includes the steps of defining the lenses' footprint in a thermoplastic resin, e.g. by photolithography using a photosensitive composition such as a photoresist, and subsequently heating this material above its reflow temperature. The surface tension draws the island of photoresist into a spherical cap with a volume equal to the original island before the reflow. This cap is a plano-convex microlens. Advantages of the technique are, amongst others, the simplicity, the reproducibility, and the possibility of integration directly on top of a light-detecting optoelectronic device.

As necessary, an overcoat layer is formed on the patterned lens units with a rectangular shape prior to reflowing to avoid a sagging of the island of the resin in the middle without reflow into a spherical cap in the reflow step. The overcoat layer acts as a permanent protective layer. The coating layer may also be formed of the photosensitive composition of the present invention.

Microlens arrays can also be produced by the use of a mold or a stamper as, for example, disclosed in EP0932256A2. A process of producing the planar microlens array is as follows: (1) a release agent is applied on a shaped surface of a stamper on which convex portions are densely arranged, and a photocurable synthetic resin material having a high refractive index is set on the shaping surface of the stamper. Next, the base glass plate is pushed onto the synthetic resin material, thereby spreading the synthetic resin material, and the synthetic resin material is cured by irradiating with ultraviolet radiation or by heating and is shaped to form the convex microlens. Thereafter, the stamper is peeled off. Then, a photocurable synthetic resin material having a low refractive index is additionally coated onto the convex microlens as an adhesive layer and a glass substrate which is made into a cover glass plate is pushed onto the synthetic resin material, thereby spreading the synthetic resin material. The synthetic resin material is then cured and finally the planar microlens array is formed.

(2) As disclosed in U.S. Pat. No. 5,969,867, a similar method using a mold is applied for the production of a prism sheet, which is used as a part of backlight units for color liquid crystal display panels to enhance the brightness. A prism sheet forming a prism row on one side is mounted on the light-emitting surface of the backlight. For producing a prism sheet, an active energy ray-curable composition is cast and spread in a lens mold which is made of metal, glass or resin and forms the lens shape of the prism row, etc., after which a transparent substrate sheet is placed onto it and active energy rays from an active energy ray-emitting source are irradiated through the substrate sheet for curing. The prepared lens sheet is then released from the lens mold to obtain the lens sheet.

The active energy ray-curable composition used to form the lens section must have a variety of properties, including adhesion to the transparent substrate, and suitable optical characteristics. The microlens arrays using photoresists in the prior art are not suitable for some applications since the optical transmittance in the blue end of the optical spectrum is poor.

Because the photosensitive composition of the present invention has low yellowing properties, both thermally and photochemically, it can solve the conventional defects and is suitable for the production of microlens arrays as described above.

## < Plasma Display Panel >

The photosensitive composition of the present invention is also suitable for a photolithographic step used in the production process of plasma display panels (PDP), particularly for the image forming process of barrier rib, phosphor layer and electrodes.

The PDP is a planar display for displaying images and information by virtue of the emission of light by gas discharge. By the construction of panel and the method of operation, it is known in two types, i.e. DC (direct current) type and AC (alternating current) type.

By way of example, the principle of the DC type color PDP will be briefly explained. In the DC type color PDP, the space intervening between two transparent substrates (generally glass plates) is divided into numerous minute cells by latticed barrier ribs interposed between the transparent substrates. In the individual cells a discharge gas, such as He or Xe, is sealed. On the rear wall of each cell there is a phosphor layer which, on being excited by the ultraviolet light generated by the discharge of the discharge gas, emits visible light of three primary colors. On the inner faces of the two substrates, electrodes are disposed as opposed to each other across the relevant cells. Generally, the cathodes

are formed of a film of transparent electroconductive material such as NESA glass. When a high voltage is applied between these electrodes formed on the fore wall and the rear wall, the discharge gas which is sealed in the cells induces plasma discharge and, by virtue of the ultraviolet light radiated consequently, incites the fluorescent elements of red, blue, and green colors to emit lights and effect the display of an image. In the full-color display system, three fluorescent elements severally of the three primary colors of red, blue, and green mentioned above jointly form one picture element.

The cells in the DC type PDP are divided by the component barrier ribs of a lattice, whereas those in the AC type PDP are divided by the barrier ribs which are arranged parallel to each other on the faces of the substrates. In either case, the cells are divided by barrier ribs. These barrier ribs are intended to confine the luminous discharge within a fixed area to preclude false discharge or cross talk between adjacent discharge cells and ensure ideal display.

< Printing Ink >

Photocuring is of great importance for printings, since the drying time of the ink is a critical factor for the production rate of graphic products, and should be in the order of fractions of seconds. UV-curable inks are particularly important for screen printing and offset inks. The photosensitive composition of the present invention can be preferably used as the material of the UV-curable inks.

< Printing Plate >

As already mentioned above in the printing ink, the photosensitive composition of the present invention is highly suitable also for producing printing plates. This application uses, for example, mixtures of soluble linear polyamides or styrene/butadiene and/or styrene/isoprene rubber, polyacrylates or polymethyl methacrylates containing carboxyl groups, polyvinyl alcohols or urethane acrylates with photopolymerizable monomers, for example acrylamides and/or methacrylamides, or acrylates and/or methacrylates, and a photoinitiator. Films and plates of wet or dry system are exposed over the negative or positive of the printed original, and the uncured parts are subsequently washed out using an appropriate solvent or aqueous solutions.

< Other Coating Materials >

Another field where the photosensitive composition of the present invention is preferably employed is the coating of metal surfaces, in the case, for example, of the coating of metal plates and tubes, cans or bottle caps, and the photocuring of polymer coatings, for example of floor or wall coverings based on PVC. Examples of the photocuring of paper coatings are the colorless varnishing of labels, record sleeves and book covers. For these, the photosensistive composition of the present invention can be preferably used.

< Composite Material >

The photosensitive composition of the present invention is particularly preferably used for curing shaped articles made from composite compounds. The composite compound consists of a self-supporting matrix material, for example a glass fiber fabric, or, plant fibers (see. K. -P. Mieck, T. Reussmann in Kunststoffe 85 (1995), 366-370), which is impregnated with the photocurable photosensitive composition. Shaped parts containing composite compounds, when produced using the photosensitive composition of the present invention including the novel oxime compound of the present invention, attain a high level of mechanical stability and resistance. The novel oxime compound of the present invention can also be employed as a photocuring agent in molding, impregnating and coating compositions as are described, for example, in EP 7086. Examples of such compositions include gel coat resin, which are subject to stringent requirements regarding curing activity and yellowing resistance, and fiber-reinforced moldings, for example, light diffusing panels which are planar or have lengthwise or crosswise corrugation. Technique for producing such moldings, such as hand lay-up, spray lay-up, centrifugal casting or filament winding, are described, for example, by P. H. Selden in "Glasfaserveretaerkte Kunststoffe", p. 610, Springer Verlag Berlin-Heidelberg-New York 1967. Examples of articles which can be produced by these techniques are boats, fiber board or chipboard panels with a double-sided coating of glass fiber-reinforced plastic, pipes, containers, etc. Further examples of moldings, impregnating and coating compositions include UP resin gel coats for moldings containing glass fibers (GRP), such as corrugated sheets and paper laminates. Paper laminates may be produced, based on urea resins or melamine resins. Prior to production of the laminate, the gel coat is produced on a support (for example a film). The photosensitive composition can also be used for casting resins or for embedding articles, for example electronic components, etc.

< Other Applications >

The photosensitive composition of the present invention can be preferably used for the production of holographies, waveguides, optical switches wherein advantage is taken of the development of a difference in the index of refraction between irradiated and unirradiated areas.

The photosensitive composition of the present invention is suitably used as the photocurable compositions for imaging techniques and for the optical production of information carriers. In such applications, as already described above, the photosensitive layer (wet or dry) applied to the support is irradiated imagewise, e.g. through a photomask, with UV or visible light, and the unexposed areas of the photosensitive layer are removed by treatment with a developer. Application of the photocurable layer to metal can also be carried out by electrodeposition. The exposed areas are polymeric through crosslinking and are therefore insoluble to the developer and remain on the support. Appropriate coloration produces visible images, Where the support is a metallized layer, the metal can, following exposure and development, be etched away at the unexposed areas or reinforced by electroplating. In this way it is possible to produce electronic circuits and photoresists.

When the photosensitive composition is used in the image forming materials the oxime compound of the present invention provides excellent performance in generating so called printout images, whereby a color change is induced due to UV irradiation. To form such printout images different dyes and/or their leuco form are used and examples for such print out image systems can be found in International Publication No. WO 96/41240, EP 706091, EP 511403, U.S. Pat. Nos. 3,579,339, and 4,622,286.

The photosensitive composition of the present invention can be preferably used for forming a dielectric layer of the multilayer circuit-board produced by a sequential lamination method.

The present invention, as described above, provides the composition for producing pigmented and non-pigmented paints and varnishes, powder coatings, printing inks, printing plates, adhesives, gel coats, dental compositions, photoresists for electronics such as electroplating resist, etch resist, both liquid and dry films, photoresists for producing solder resist, as resists to produce color filters for a variety of display applications or to generate structures in the producing processes of plasma-display panels (e.g. barrier rib, phosphor layer, electrode), electroluminescence displays and LCD (e.g. interlayer insulating layer, spacers, microlens array), as composition for encapsulating electrical and electronic components, for producing magnetic recording materials, micromechanical parts, waveguides, optical switches, plating masks, etch masks, color proofing systems, glass fiber cable coatings, screen printing stencils, for producing three-dimensional objects by means of stereolithography, and as image recording material, especially for holographic recordings, microelectronic circuits, decolorizing materials, decolorizing materials for image recording materials, for image recording materials using microcapsules.

(Photopolymerization Method of Photosensitive Composition of the Present Invention)

The photosensitive composition of the present invention is laminated on the base material, and at least exposed, and if necessary developed, and then subjected to thermal treatment.

[Formation of Laminate for Photographic Material]

Examples of the base material used for photographic information recordings include, for example, films of polyester, cellulose acetate or polymer-coated papers. The base material for offset printing paper include specially treated aluminium, the base material for producing printed circuits include copper-clad laminates, and the base material for producing integrated circuits include, for example, silicon wafers.

The thickness of the photosensitive layer for photographic materials and offset printing forms is generally from about 0.5 μm to 10 μm, while for printed circuits it is from 0.1 μm to about 100 μm. Following the coating of the substrate (base material) with the photosensitive composition, the solvent is removed, generally by drying, to form a coat of the photoresist (photosensitive layer) on the substrate.

Application of the photosensitive composition to the base material can be carried out by applying to the base material a liquid photosensitive composition, a solution or a suspension. The solvents and the concentration are suitably selected depending on the type of composition and on the coating technique. The solvent should be inert, i.e. it should not undergo a chemical reaction with the components and should be able to be removed again, after coating, in the course of drying. Examples of suitable solvents are ketones, ethers and esters. Specific examples thereof include methyl ethyl ketone, isobutyl methyl ketone, cyclopentanone, cyclohexanone, N-methylpyrrolidone, dioxane, tetrahydrofuran, 2-methoxyethanol, 2-ethoxyethanol, 1-methoxy-2-propanol, 1,2-dimethoxyethane, ethyl acetate, n-butyl acetate, ethyl 3-ethoxypropionate, 2-methoxypropylacetate, methyl-3-methoxypropionate, 2-heptanone, 2-pentanone, and ethyl lactate.

The solution for the photosensitive composition is applied uniformly to a substrate by means of known coating

techniques, for example by spin coating, dip coating, knife coating, curtain coating, brushing, spraying, especially by electrostatic spraying, and reverse-roll coating, and also by means of electrophoretic deposition.

It is also possible to apply the solution for the photosensitive composition to a temporary, flexible support so as to form a photosensitive layer and then the photosensitive layer is provided on the final substrate, for example a copper-clad circuit board, or a glass substrate by transferring the photosensitive layer via lamination.

The amount applied of the solution for the photosensitive composition (thickness of the photosensitive layer) and the nature of the substrate (layer support) are suitably selected in accordance with the application. The thickness of the photosensitive layer is generally about 0.1 μm or more, preferably 100 μm or more, more preferably 0.1 μm to 1 cm, and particularly preferably 0.5 μm to 1,000 μm.

By the use of the photosensitive composition of the present invention, the photosensitive composition is applied to the base material, and then the solvent is removed, generally by drying, to leave an essentially dry resist film (photosensitive layer) of the photoresist on the base material.

[Exposing Step]

The photosensitivity of the photosensntive composition of the present invention can extend in general from about 150 nm to 600 nm, for example 190 nm to 600 nm, (UV to visible light ranges). Suitable radiation is present, for example, in sunlight or light from artificial light sources. Light sources are not particularly limited and may be appropriately selected depending on the purpose. Both point sources and arrays ("lamp carpets") are suitable. Examples thereof include carbon arc lamps, xenon are lamps, low-, medium-, high- and super high-pressure mercury lamps, possibly with metal halide dopes (metal-halogen lamps), microwave-stimulated metal vapour lamps, excimer lamps, superactinic fluorescent tubes, fluorescent lamps, argon incandescent lamps, electronic flashlights, photographic flood lamps, light emitting diodes (LED), electron beams and X-xays. The distance between the lamp and the base material including the photosensitive composition of the present invention to be exposed may vary depending on the intended application and the type and output of lamp, and may be, for example, from 2 cm to 150 cm. Laser light sources, for example excimer lasers, such as $F_2$ excimer lasers at 157 nm exposure, KrF excimer lasers for exposure at 248 nm and ArF excimer lasers for exposure at 193 nm are also suitable. Lasers in the visible region can also be employed.

The base material including the photosensitive composition of the present invention is exposed imagewise. The term "imagewise" exposure includes both, exposure through a photomask including a predetermined pattern, for example a slide, a chromium mask, a stencil mask or a reticle, as well as exposure by means of a laser or light beam, which for example is moved under computer control over the surface of the coated substrate coated with the photosensitive composition of the present invention and in this way produces an image. Suitable UV laser exposure systems for the purpose are, for example, provided by Etec and Orbotech (DP-100 DIRECT IMAGING SYSTEM). The computer-controlled irradiation can also be achieved by electron beams. It is also possible to use masks made of liquid crystals that can be addressed pixel by pixel to generate digital images, as is, for example, described in A. Bertsch, J. Y. Jezequel, J. C. Andre in Journal of Photochemistry and Photobiology A: Chemistry 1997, 107, p. 275-281 and by K. -P, Nicolay in Offset Printing 1997, 6, p. 34-37.

[Thermal Treatment Step]

Following the imagewise exposure of the base material and prior to development, it may be advantageous to carry out thermal treatment (prebake) for a short time. After the development a thermal treatment (postbake) can be performed to cure a curable film and to remove all traces of solvents. The temperature for thermal treatment is preferably 50°C to 250°C, and more preferably 80°C to 220°C; the duration of the thermal treatment is preferably 0.25 minutes to 60 minutes.

The photosensitive composition of the present invention may additionally be used in a process for producing printing plates or photoresists as is described, for example, in DE 4013358. In such a process the composition is exposed for a short time to visible light with a wavelength of at least 400 nm, without a mask, prior to, simultaneously with or following imagewise irradiation.

[Developing Step]

After the exposure and, thermal treatment, the unexposed areas of the photosensitive layer are removed with a developer in a conventionally known method.

As already mentioned, the photosensitive composition of the present invention can be developed by aqueous alkalis or organic solvents. Examples of the aqueous-alkaline developer solutions include aqueous solutions of tetraalkylammonium hydroxides or of alkali metal silicates, phosphates, hydroxides and carbonates. Small amount of wetting agents and/or organic solvents may also be added, as necessary, to these solutions. Examples of typical organic solvents,

which may be added to the developer in small amount, include cyclohexanone, 2-ethoxyethanol, toluene, acetone and mixtures of such solvents. Depending on the base material, organic solvents, or above-mentioned mixtures of aqueous alkali solutions with the organic solvents may be used as developer.

Examples of the developer include methanol, ethanol, 2-propanol, 1-propanol, butanol, diacetone alcohol, ethylene glycol monomethyl ether, ethylene glycol monoethyl ether, ethylene glycol mono-n-butyl ether, diethyleneglycol dimethyl ether, propyleneglycol monomethyl ether acetate, ethyl-3-ethoxypropionate, methyl-3-methoxypropionate, n-butyl acetate, benzyl alcohol, acetone, methyl ethyl ketone, cyclopentanone, cyclohexanone, 2-heptanone, 2-pentanone, ε-caprolactone, γ-butylolactone, dimethylformamide, dimethylacetamide, hexamethylphosphoramide, ethyl lactate, methyl lactate, ε-caprolactam, and N-methyl-pyrrolidinone. Optionally, water can be added to these solvents up to a level at which still a clear solution is obtained and at which sufficient solubility of the unexposed areas of the photosensitive composition is maintained.

The photosensitive composition of the first embodiment of the present invention can be preferably used for the method for photopolymerization of compounds containing ethylenically unsaturated double bonds, i.e. monomeric, oligomeric or polymeric compounds containing at least one ethylenically unsaturated double bond, and for the method including adding to these compounds at least one of the oxime compounds of the present invention represented by Genearl Formula (1) as described above as the photoinitiator and irradiating the resulting composition with electromagnetic radiation, in particular light of the wavelength 150 nm to 600 nm, in particular 190 nm to 600 nm, with electron beam, or with X-rays.

The photosensitive composition of the first embodiment of the present invention can be preferably used for the method for the photographic production of relief images wherein a coated substrate which is coated on at least one surface with the composition as described above is provided, and the coated substrate is subjected to imagewise exposure and then the unexposed portions are removed with a developer. Imagewise exposure may be effected by irradiating through a mask or by means of a laser or electron beam as already described above. Among these, the laser beam exposure already mentioned above is particularly preferable.

The photosensitive composition of the first embodiment of the present invention has excellent thermal stability and low volatility, and is also suitable for photopolymerizations in the presence of air (oxygen). Further, it is preferably used for the photopolymerization because only low yellowing is caused in the photosensitive composition of the present invention after photopolymerization.

« Photosensitive composition according to second embodiment »

(1) Photosensitive composition according to a second embodiment

A photosensitive composition according to a second embodiment of the present invention contains (A) a photopolymerization initiator, (B) an ethylenically unsaturated compound, (C) a binder, (D) a coloring material and (E) a solvent; and, if necessary, further contains other components.

The concentration of solid matter (solid matter concentration) in the photosensitive composition according to a second embodiment of the present invention is preferably 2.5% by mass to 15% by mass, more preferably 5% by mass to 12.5% by mass, most preferably 7.5% by mass to 10% by mass. When the solid matter concentration of the photosensitive composition is less than 2.5% by mass, the coating amount required for achieving a desired optical density is too large, which is not practical. When the solid matter concentration of the photosensitive composition is more than 15% by mass, the effects of the present invention cannot be obtained.

< (A) Photopolymerization initiators >

The photosensitive composition according to a second embodiment of the present invention contains, as a photopolymerization initiator, an oxime compound represented by the following General Formula (A-1) or the oxime compound of the present invention represented by any of General Formulas (1) to (4); and, if necessary, contains other conventionally known photopolymerization initiator(s) than the above oxime compound. Notably, the oxime compounds represented by General Formulas (1) to (4) are exemplified above.

- Oxime compound -

The oxime compound contained in the photosensitive composition according to a second embodiment of the present invention is represented by the following General Formula (A-1).

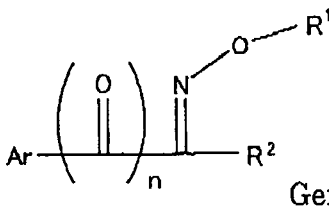

General Formula (A-1)

In General Formula (A-1), $R^1$ represents an acyl group, an alkyloxycarbonyl group or an aryloxylcarbonyl group.

The acyl group is not particularly limited and may be appropriately selected depending on the purpose. Examples thereof include aliphatic acyl groups, aromatic acyl groups and heteroaromatic acyl groups.

The total number of carbon atoms contained in the acyl group is not particularly limited and may be appropriately selected depending on the purpose. It is preferably 2 to 30, more preferably 2 to 20, particularly preferably 2 to 16.

The acyl group may have a substituent. Examples of the substituent include an alkyloxy group, an aryloxy group and halogen atoms.

Specific examples of the acyl group are not particularly limited and include acetyl, propanoyl, methylpropanoyl, butanoyl, pivaloyl, hexanoyl, cyclohexanecarbonyl, octanoyl, decanoyl, dodecanoyl, octadecanoyl, benzylcarbonyl, phenoxyacetyl, 2-ethylhexanoyl, chloroacetyl, benzoyl, paramethoxybenzoyl, 2,5-dibutoxybenzoyl, 1-naphthyl, 2-naphthoyl, pyridylcarbonyl, methacryloyl and acryloyl.

The alkyloxycarbonyl group is not particularly limited and may be appropriately selected depending on the purpose.

The total number of carbon atoms contained in the alkyloxycarbonyl group is not particularly limited and may be appropriately selected depending on the purpose. It is preferably 2 to 30, more preferably 2 to 20, particularly preferably 2 to 16.

The alkyloxycarbonyl group may have a substituent, and examples of the substituent include an alkyloxy group and halogen atoms.

Specific examples of the alkyloxycarbonyl group are not particularly limited and include methoxycarbonyl, ethoxycarbonyl, isopropoxycarbonylbutoxycarbonyl, isobutyloxycarbonyl, allyloxycarbonyl, octyloxycarbonyl, dodecyloxycarbonyl and ethoxyethoxycarbonyl,

The aryloxylcarbonyl group is not particularly limited and may be appropriately selected depending on the purpose.

The total number of carbon atoms contained in the aryloxylcarbonyl group is not particularly limited and may be appropriately selected depending on the purpose. It is preferably 7 to 30, more preferably 7 to 20, particularly preferably 7 to 16.

The aryloxylcarbonyl group may have a substituent, and examples of the substituent include an alkyloxy group and halogen atoms.

Specific examples of the aryloxylcarbonyl group are not particularly limited and include phanoxycarbonyl, 2-naphthoxycarbonyl, paramethoxyphenoxycarbonyl, 2,5-diethoxyphenoxycarbonyl, parachlorophenoxycarbonyl, paranitrophenoxycarbonyl and paracyanophenoxycarbonyl.

In General Formula (A-1), $R^2$ represents a hydrogen atom, an alkyl group or a cyano group.

The alkyl group is not particularly limited and may be appropriately selected depending on the purpose. Examples thereof include methyl, ethyl, isopropyl, tertiary butyl and tertiary octyl.

In General Formula (A-1), Ar represents an aromatic or heteroaromatic ring,

The aromatic ring is not particularly limited and may be appropriately selected depending on the purpose. Examples thereof include a benzene ring, a naphthalene ring and anthracene ring.

The heteroaromatic ring is not particularly limited and may be appropriately selected depending on the purpose. Examples thereof include a pyrrole ring, an indole ring, a benzofuran ring and a benzothiophene ring.

N is an integer of 0 (zero) or 1. Ar and $R^2$ may be linked together to form a ring.

Among the oxime compound represented by General Formula (A-1), a compound represented the following General Formula (A-2) is preferred from the viewpoint of high sensitivity.

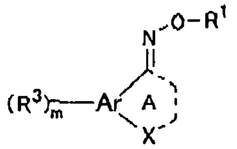

General Formula (A-2)

In General Formula (A-2), $R^1$ represents an acyl group, an alkyloxycarbonyl group or an aryloxylcarbonyl group, each of which may have a substituent; $R^3$ represents an alkyl group, an alkyloxy group, an aryloxy group, an alkylthio group, an arylthio group, a halogen atom, an alkyloxycarbonyl group, a nitro group or an acylamino group; m is an integer of 0 or more; when m is an integer of 2 or more, $R^3$s may be identical or different and may be linked together to form a ring; Ar represents an aromatic or heteroaromatic ring; A represents a 4-, 5- or 6-membered ring, each of which may have a substituent; and X represents an oxygen atom or a sulfur atom.

Notably, groups represented by $R^1$ and rings represented by Ar are similar to those described in relation to General Formula (A-1). Also, 4-, 5- or 6-membered rings represented by A are similar to the 5- or 6-membered rings of 4-, 5-, 6- or 7-membered rings described in relation to General Formula (A-1).

The acyl group is not particularly limited and may be appropriately selected depending on the purpose. Examples thereof include aliphatic acyl groups, aromatic acyl groups and heteroaromatic acyl groups.

The total number of carbon atoms contained in the acyl group is not particularly limited and may be appropriately selected depending on the purpose. It is preferably 2 to 30, more preferably 2 to 20, particularly preferably 2 to 16.

The acyl group may have a substituent, and examples of the substituent include an alkyloxy group, an aryloxy group and halogen atoms.

Specific examples of the acyl group are not particularly limited and include acetyl, propanoyl, methylpropanoyl, butanoyl, pivaloyl, hexanoyl, cyclohexanecarbonyl, octanoyl, decanoyl, dodecanoyl, octadecanoyl, benzylcarbonyl, phenoxyacetyl, 2-ethylhexanoyl, chloroacetyl, benzoyl, paramethoxybenzoyl, 2,5-dibutoxybenzoyl, 1-naphthoyl, 2-naphthoyl, pyridylcarbonyl, methacryloyl and acryloyl.

The alkyloxycarbonyl group is not particularly limited and may be appropriately selected depending on the purpose.

The total number of carbon atoms contained in the alkyloxycarbonyl group is not particularly limited and may be appropriately selected depending on the purpose. It is preferably 2 to 30, more preferably 2 to 20, particularly preferably 2 to 16.

The alkyloxycarbonyl group may have a substituent, and examples of the substituent include an alkoxy group and halogen atoms.

Specific examples of the alkyloxycarbonyl group are not particularly limited and include methoxycarbonyl, ethoxycarbonyl, isopropoxycarbonylbutoxycarbonyl, isobutyloxycarbonyl, allyloxycarbonyl, octyloxycarbonyl, dodecyloxycarbonyl and ethoxyethoxycarbonyl.

The aryloxylcarbonyl group is not particularly limited and may be appropriately selected depending on the purpose.

The total number of carbon atoms contained in the aryloxylcarbonyl group is not particularly limited and may be appropriately selected depending on the purpose. It is preferably 7 to 30, more preferably 7 to 20, particularly preferably 7 to 16.

The aryloxylcarbonyl group may have a substituent, and examples of the substituent include an alkoxy group and halogen atoms.

Specific examples of the aryloxylcarbonyl group is not particularly limited and include phenoxycarbonyl, 2-naphthoxycarbonyl, paramethoxyphenoxycarbonyl, 2,5-diethoxyphenoxycarbonyl, parachlorophenoxycarbonyl, paranitrophenoxycarbonyl and paracyanophenoxycarbonyl.

In General Formula (A-2), $R^3$ represents or $R^3$s each represent an alkyl group, an alkyloxy group, an aryloxy group, an alkylthio group, an arylthio group, a halogen atom, an alkyloxycarbonyl group, a nitor group or an acylamino group.

The alkyl group is not particularly limited and may be appropriately selected depending on the purpose. Examples thereof include methyl, ethyl, isopropyl, tertiary butyl and tertiary octyl.

The alkyloxy group is not particularly limited and may be appropriately selected depending on the purpose. Examples thereof include methoxy, ethoxy, propoxy and butoxy.

The aryloxy group is not particularly limited and may be appropriately selected depending on the purpose. Examples thereof include benzyloxy, phenoxy, 4-chlorophenoxy and 4-methylphenoxy.

The alkylthio group is not particularly limited and may be appropriately selected depending on the purpose.

Examples thereof include methylthio, butylthio, octylthio and dodecylthio.

The arylthio group is not particularly limited and may be appropriately selected depending on the purpose. Examples thereof include benzylthio, phenylthio and 4-methylphenylthio.

The halogen atom is not particularly limited and may be appropriately selected depending on the purpose. Examples thereof include chlorine, bomine and iodine.

The alkyloxycarbonyl group is not particularly limited and may be appropriately selected depending on the purpose. Examples thereof include methoxycarbonyl and ethoxycarbonyl.

The acylamino group is not particularly limited and may be appropriately selected depending on the purpose. Examples thereof include methylcarbonylamino, ethylcarbonylamino and benzenecarbonylamino.

In General Formula (A-2), m is an integer of 0 or more and may be appropriately determined as any number in consideration of the structure of a ring represented by Ar.

Notably, when m is an integer of 2 or more, $R^3$s may be identical or different and may be linked together to form a ring. Specific examples of the oxime compound represented by General Formula (A-1) in which $R^3$s are linked together to form a ring include compounds having the following Structural Formulas:

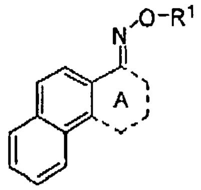
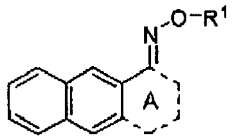
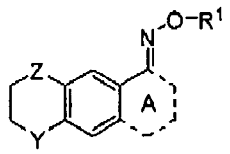
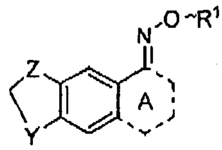

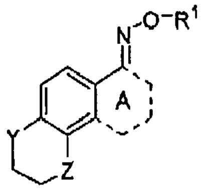
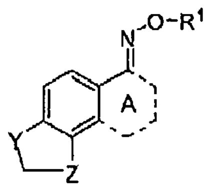
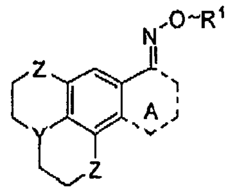
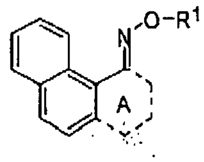

in these Structural Formulas, Y and Z each represent $CH_2$, -O-, -S- or -NR-(where R represents a hydrogen atom or an alkyl group).

In General Formula (A-2), Ar represents an aromatic or heteroaromatic ring.

The aromatic ring is not particularly limited and may be appropriately selected depending on the purpose. Examples thereof include a benzen ring, a naphthalene ring and an anthracene ring.

The heteaoaromatic ring is not particularly limited and may be appropriately selected depending on the purpose. Examples thereof include a pyrrole ring, an indole ring, a benzofuran ring and a benzothiophene ring.

In General Formula (A-2), A represents a 4-, 5- or 6-membered ring.

The 4-, 5- or 6-membered ring is not particularly limited and may be appropriately selected depending on the purpose. Preferably, this ring is formed of ring-forming substituents. Examples of the ring-forming substituents include alkylene, alkenylene and alkynylene. These ring-forming substituents may be individually used or in combination, and may contain a hetero atom such as oxygen and sulfur.

The 4-, 5-, 6- or 7-membered ring may have a substituent, and examples of the substituent include an alkyl group, an alkenyl group, an alkyloxy group, an aryloxy group and a halogen atom.

Among them, a ring represented by A is preferably a 5- or 6-membered ring from viewpoint of high sensitivity.

Among the oxime compound represented by General Formula (A-2), a compound represented by General Formula (A-3) or (A-4) is preferred from the viewpoint of high sensitivity.

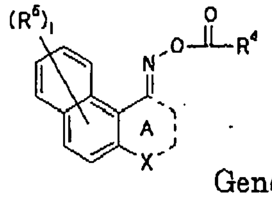

General Formula (A-3)

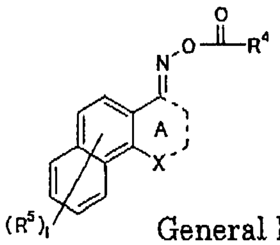

General Formula (A-4)

In General Formula (A-3) or (A-4), $R^4$ represents an alkyl group or an alkyloxy group.

The alkyl group is not particularly limited and may be appropriately selected depending on the purpose.

The alkyl group may have a substituent, examples of the substituent include an alkyloxy group and halogen atoms.

Specific examples of the alkyl group are not particularly limited and include methyl, ethyl, propyl, butyl and hexyl. Among them, methyl, ethyl and propyl are preferred from the viewpoint of high sensitivity.

The alkyloxy group is not particularly limited and may be appropriately selected depending on the purpose.

The alkyloxy group may have a substituent, and examples of the substituent include an alkyloxy group and halogen atoms.

Specific examples of the alkyloxy group are not particularly limited and include methoxy, ethoxy, propoxy and butoxy.

In General Formula (A-3) or (A-4), $R^5$ represents or $R^5$s each represent an alkyl group, an alkyloxy group, an aryloxy group, an alkylthio group, an arylthio group, a halogen atom, an alkyloxycarbonyl group, a nitro group or an acylamino group.

Notably, the alkyl, alkyloxy, aryloxy, alkylthio and arylthio groups, the halogen atom, and the alkyloxycarbonyl, nitro and acylamino groups are similar to those represented by $R^2$ in General Formula (A-1).

Among them, a group/atom represented by $R^6$ is preferably alkyloxy, arylthio or a halogen atom from the viewpoint of excellent absorption efficiency.

In General Formula (A-3) or (A-4), 1 is an integer of 0 to 6. Among them, preferably, 1 is an integer of 0 to 2 from the viewpoint of synthesis suitability.

Notably, when 1 is an integer of 2 or more, $R^5$s may be identical or different and may be linked together to form a ring.

In General Formula (A-3) or (A-4), A represents a 5- or 6-membered ring, which may have a substituent.

Notably, the 5- or 6-membered ring is similar to that represented by A in General Formula (A-2).

Among them, a group represented by A is preferably a 5-membered ring from the viewpoint of high sensitivity.

In General Formula (A-3) or (A-4), X represents an oxygen atom or a sulfur atom.

Specific examples of oxime compounds represented by at least one of General Formulas (A-1), (A-2), (A-3) and (A-4) include compounds having the following Structural Formulas (1) to (26), which should not be construed as limiting the present invention thereto:

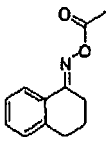
1

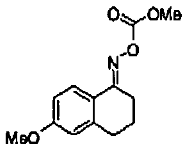
2

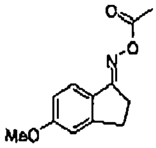
3

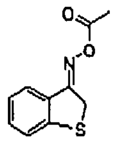
4

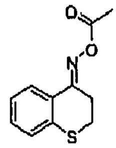
5

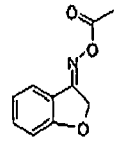
6

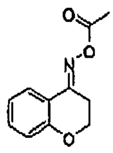
7

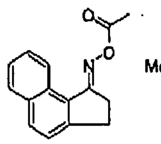
8

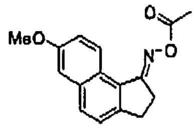
9

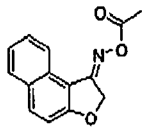
10

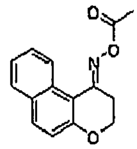
11

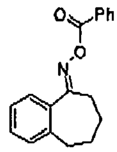
12

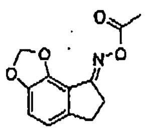
13

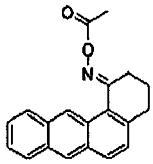
15

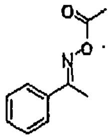
16

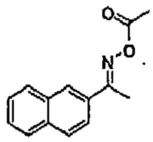
17

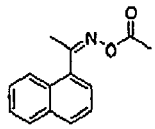
18

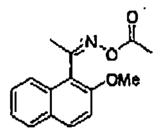
19

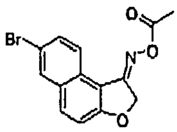
20

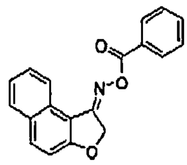
21

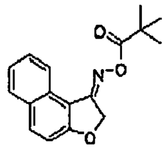
22

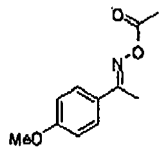
23

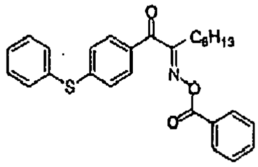
IRGACURE OXE01

24

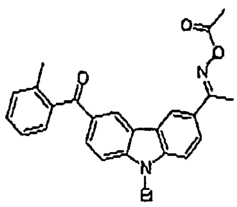
IRGACURE OXE02

25

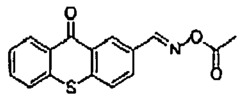
CGI.325

26

in Structural Formulas (1) to (26), Me represents a methyl group and Ph represents a phenyl group; and compounds having Structural Formulas (24) to (26) are respectively IRGACURE OXE01, IRGACURE OXE02 and CG1325 (these products are of CIBA Co.).

Among them, particularly preferred are those having Structural Formulas (10), (17), (18), (24), (25) and (26).

< (B) Ethylenically Unsaturated Compound>

The ethylenically unsaturated compound preferably has a crosslinking group, and is not specifically limited, as long as the effect of the present invention is not lost, and may be selected from those having at least one ethylenically unsaturated group that can undergo addition polymerization according to the purpose. Examples thereof include ester compounds, amide compounds, and other compounds. These can be used alone or in combination.

Examples of the ester compounds include monofunctional (meth)acrylic esters, polyfunctional (meth)acrylic esters, itaconic esters, crotonic esters, isocrotonic esters, maleic esters, and other ester compounds. Among these, monofunctional (meth)acrylic esters and polyfunctional (meth)acrylic esters are preferred.

Examples of the monofunctional (meth)acrylic esters include polyethylene glycol mono(meth)acrylate, polypropylene glycol mono(meth)acrylate, and phenoxyethyl mono(meth)acrylate.

Examples of the polyfunctional (meth)acrylic esters include polyethylene glycol di(meth)acrylate, ethylene glycol di(meth)acrylate, triethylene glycol di(meth)acrylate, 1,3-butanediol di(meth)acrylate, tetramethylene glycol di(meth) acrylate, hexanediol di(meth)acrylate, pentaerythritol tri(meth)acrylate, pentaerythritol tetra(meth)acrylate, dipentaerythritol di(meth)acrylate, dipentaerythritol tri(meth)acrylate, dipentaerythritol tetra(meth)acrylate, dipentaerythritol hexa (meth)acrylate, dipentaerythritol poly(meth)acrylate, sorbitol tri(meth)acrylate, sorbitol tetra(meth)acrylate, trimethylolethane tri(meth)acrylate, neopentyl glycol di(meth)acrylate, and hexanediol di(meth)acrylate.

Among these, dipentaerythritol poly(meth)acrylates are preferred.

Examples of the polyfunctional (meth)acrylic esters also include (meth)acrylates prepared by adding ethylene oxide or propylene oxide to a polyfunctional alcohol such as glycerol or trimethylolethane, and converting the resulting adduct into a (meth)acrylate; urethane acrylates described in JP-B Nos. 48-41708 and 50-6034, and JP-A No. 51-37193; polyester acrylates described in JP-A No. 48-64183, JP-B Nos. 49-43191 and 52-30490; epoxy acrylates as reaction products between an epoxy resin and (meth)acryli acid; and (meth)acrylates, urethane (meth)acrylates, and vinyl esters described in JP-A No. 60-258539.

Examples of the ester compounds also include trimethylolpropane tri(acryloyloxypropyl) ether, tri(acryloyloxyethyl) isocyanurate, photocurable monomers and oligomers described in Journal of the Adhesion Society of Japan, Vol. 20, No. 7, p. 300-308.

The amide compounds include, for example, amides (monomers) between an unsaturated carboxylic acid and an aliphatic polyamine compound. Specific examples thereof include methylenebis(meth)acrylamide, 1,6-hexamethylenebis(meth)acrylamide, diethylenetriamine tris(meth)acrylamide, and xylylenebis(meth)acrylamide, and (meth)acrylamides described in JP-A No. 60-258539.

Examples of other compounds include allyl compounds as described in JP-A No. 60-258539.

The amount of the ethylenically unsaturated compound in the photosensitive composition is preferably 10% by mass to 60% by mass, and more preferably 20% by mass to 50% by mass relative to the all solid content in the photosensitive composition.

< (C) Binder >

The binder contained in the photosensitive composition functions as a coating material when a layer formed from the photosensitive composition is formed on a substrate surface, and resins which can be alkali developed may be used. In terms of hardening sensitivity, the binder used is selected under the conditions that a polymer compound used as the binder preferably has a crosslinking group or a polymerizable group, which crosslinks with an unsaturated bond of the ethylenically unsaturated compound described later so that the crosslinking density in the photosensitive composition is 0.0073 mol/g or more.

A polymer compound constituting the binder itself preferably has a crosslinking group as described above. The polymer compound may be appropriately selected depending on the purpose, and may be monomeric polymer or copolymer of a plularity of monomers. Preferred is a copolymer at least including a structural unit having a carboxyl group, a structural unit represented by the following General Formula (1), and a structural unit having (meth)acrylate having at least an aromatic ring and/or an aliphatic ring.

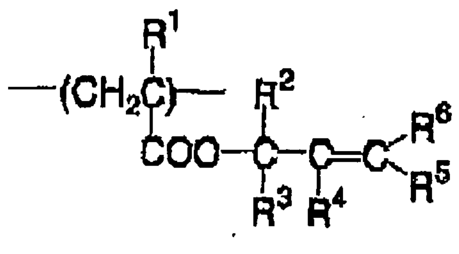

General Formula (1)

In General Formula (1), $R^1$ represents a hydrogen atom or a methyl group; and $R^2$ to $R^6$ each independently represents a hydrogen atom, an alkyl or aryl group that may be substituted, a halogen atom or a cyano group.

The copolymer is obtained by copolymerizing a monomer represented by the following Formula (2), and a polymerizable monomer having a carboxyl group, (meth)acrylate having at least an aromatic ring and/or an aliphatic

ring, and if necessary other monomers copolymerizable therewith by known methods.

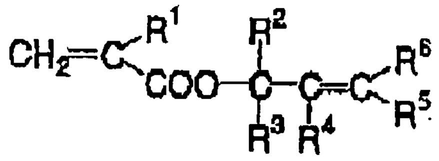

Formula (2)

In Formula (2), $R^1$ to $R^6$ represent as defined above.

Examples of the monomer represented by the aforementioned Formula (2) include allyl (meth)acrylate, 3-chloro-2-propenyl (meth)aerylate, 3-phenyl-2-propenyl (meth)acrylate, 3-(hydroxyphonyl)-2-propenyl (meth)acrylate, 3-(2-hydroxyphenyl)-2-propenyl (meth)acrylate, 3-(3,4-dihydroxyphenyl)-2-propenyl (meth)acrylate, 3-(2,4-dihydroxyphenyl)-2-propenyl (meth)acrylate, 3-(3,4,5-trihydroxyphenyl)-2-propenyl (meth)acrylate, 3-(3-methoxy-4-hydroxyphenyl)-2-propenyl (meth)acrylate, 3-(3,4-dihydroxy-5-methoxyphenyl)-2-propenyl (meth)acrylate, 3-(3,5-dimethoxy-4-hydroxyphenyl)-2-propenyl (meth)acrylate, 3-(2-hydroxy-4-methylphenyl)-2-propenyl (meth)acrylate, 3-(4-methoxyphenyl)-2-propenyl (meth)acrylate, 3-(4-ethoxyphenyl)-2-propenyl (meth)acrylate, 3-(2-methoxyphenyl)-2-propenyl (meth) acrylate, 3-(3,4-dimethoxyphenyl)-2-propenyl (meth)acrylate, 3-(3-methoxy-4-propoxyphenyl)-2-propenyl (meth)acrylate, 3-(2,4,6-trimethoxyphenyl)-2-propenyl (meth)acrylate, 3-(3-methoxy-4-benzyloxyphenyl)-2-propenyl (meth)acrylate, 3-(3-(3'-methoxyphenyl)-4-benzyloxyphenyl)-2-propenyl (meth)acrylate, 3-(3,4,5-trimethoxyphenyl)-2-propenyl (meth)acrylate, 3-(4-methylphenyl)-2-propenyl (meth)acrylate; 3-phenyl-3-(2,4,6-trimethylphenyl)-2-propenyl (meth)acrylate, 3,3-[di-(2,4,6-trimethylphenyl)]-2-propenyl (meth)acrylate, 3-phenyl-3-(4-methylphenyl)-2-propenyl (meth)acrylate, 3,3-diphenyl-2-propenyl (meth)acrylate, 3-(2-chlorophenyl)-2-propenyl (meth)acrylate, 3-(3-chlorophenyl)-2-propenyl (meth)acrylate, 3-(4-chlorophenyl)-2-propenyl (meth)acrylate, 3-(2,4-dichlorophenyl)-2-propenyl (meth)acrylate, 3-(2-bromophenyl)-2-propenyl (meth)acrylate, 3-bromo-3-phenyl-2-propenyl (meth)acrylate, 3-chloro-3-phenyl-2-propenyl (meth)acrylate, 3-(4-nitro-phenyl)-2-propenyl (meth)acrylate, 3-(2-nitro-phenyl)-2-propenyl (meth)acrylate, 3-(3-nitro-phenyl)-2-propenyl (meth)acrylate, 2-methyl-3-phenyl-2-propenyl (meth)acrylate, 2-methyl-3-(4-chlorophenyl)-2-propenyl (meth)acrylate; 2-methyl-3-(4-nitrophenyl)-2-propenyl (meth)acrylate, 2-methyl-3-(4-aminophenyl)-2-propenyl (meth)acrylate, 2-methyl-3,3-diphenyl-2-propenyl (meth)acrylate, 2-ethyl-1,3-diphenyl-2-propenyl (meth)acrylate, 2-ethoxymethylone-3-phonyl-2-propenyl (meth)acrylate, 2-mathyl-3-(4-methoxyphenyl)-2-propenyl (meth)acrylate, 2,3-diphenyl-2-propenyl (meth)acrylate, 1,2,3-triphenyl-2-propenyl (meth)acrylate, 2,3,3-triphenyl-2-propenyl (meth)acrylate, 1,3-dipheryl-2-propenyl (meth)acrylate, 1-(4-methylphenyl)-3-phenyl-2-propenyl (meth)acrylate, 1-phenyl-3-(4-methylphenyl)-2-propenyl (meth)acrylate, 1-phenyl-3-(4-methoxyphenyl)-2-propenyl (meth)acrylate, 1-(4-methoxyphenyl)-3-phonyl-2-propenyl (meth)acrylate, 1,3-di(4-chlorophenyl)-2-propenyl (meth)acrylate, 1-(4-bromophenyl)-3-phenyl-2-propenyl (meth)acrylate; 1-phenyl-3-(4-nitrophenyl)-2-propenyl (meth)acrylate, 1,3-di(2-nitrophenyl)-2-propenyl (meth)acrylate, 1-(4-dimethylaminophenyl)-3-phenyl-2-propenyl (meth)acrylate, 1-phenyl-3-(4-dimethylaminophenyl)-2-propenyl (meth)acrylate, 1,1-di(4-dimethylaminophenyl)-3-phenyl-2-prapenyl (meth)acrylate, 1,1,3-triphenyl-2-propenyl (meth)acrylate, 1,1,3,3-tetraphenyl-2-propenyl (meth)acrylate, 1-(4-methylphenyl)-3-phenyl-2-propenyl (meth)acrylate, 1-phenyl-2-propenyl (meth)acrylate, 1,2-diphenyl-2-propenyl (meth)acrylate, 1-phenyl-2-methyl-2-propenyl (meth) acrylate, 1-cyclohexyl-2-propenyl (meth)acrylate, 2-benzyl-2-propenyl (meth)acrylate, 1,1-di(4-chlorophenyl)-2-propenyl (meth)acrylate, 1-cyano-2-propenyl (meth)acrylate, 3-anilno-2-propenyl (meth)acrylate, 3-(2-methylphenyl)-2-propenyl (meth)acrylate, 3-(2,4-dimethylphenyl)-2-propenyl (meth) acrylate, 1-(2-carbetoxyisopropyl)-3-methyl-2-propenyl (meth) acrylate, 1-(1-carbetoxyisopropyl)-2-propenyl (meth)acrylate, 1-(1-carbetoxyethyl)-3-methyl-2-propenyl (meth)acrylate, 1-carbetoxy-3-chloro-3-methyl-2-propenyl (meth)acrylate, 1-carbetoxymethylene-3-methyl-2-propenyl (meth)acrylate, 1-cyano-3-methyl-2-propenyl (meth)acrylate, 1-cyclohexyl-3-(2-hydroxycyclohexyl)-2-propenyl(meth)acrylate, 3-cyclopentyl-2-propenyl (meth)acrylate; 3-furyl-2-propenyl (meth)acrylate, 3-chloro-2-propenyl (meth)acrylate, 3-bromo-2-propenyl (meth)acrylate, 2-methyl-3-chloro-2-propenyl (meth)acrylate, 2-methyl-3-bromo-2-propenyl (meth)acrylate, 2-chloro-3-phonyl-2-propenyl (meth)acrylate, 2-bromo-3-phonyl-2-propenyl (meth)acrylate, 2-bromo-3-(4-nitrophenyl)-2-propenyl (meth)acrylate, 2-fluoro-3-phenyl-2-propenyl (meth)acrylate, 2-fluoro-3-(4-methoxyphenyl)-2-propenyl (meth) acrylate, 2-cyano-3-phenyl-2-propenyl (meth)acrylate, 2-chloro-2-propenyl (meth)acrylate, 2-bromo-2-propenyl (meth) acrylate, 2-chloro-3,3-difluoro-2-propenyl (meth)acrylate, 2-fluoro-3-chloro-2-propenyl (meth)acrylate, 2,3-dibromo-2-propenyl (meth)acrylate, 2-chloro-3-methyl-2-propenyl (meth)acrylate, 1,1-dimethyl-2-propenyl (meth)acrylate, 2-pentenyl (meth)acrylate, 2-hexenyl (meth)acrylate, and 2-heptenyl (meth)acrylate. Among these, allyl (meth)acrylate is particularly preferable in terms of hardening property and raw material cost.

Examples of the aforementioned polymerizable monomer having a carboxyl group include (meth)acrylic acid, vinylbenzoic acid, maleic acid, itaconic acid, crotonic acid, sinnamic acid and acrylic acid dimer. There can also be used

an addition product of a monomer having a hydroxyl group such as 2-hydroxyethyl (meth)acrylate with a cyclic anhydride such ae maleic anhydride or phthalic anhydride. There can also be used an anhydride monomer such as maleic anhydride or itaconic anhydride as the precursor of carboxylic acid. Among these, (meth)acrylic acid is particularly preferable in terms of polymerizability and raw material cost.

Examples of the aforementioned (meth)acrylate having at least an aromatic ring and/or an aliphatic ring include cycloalkyl (meth)acrylates (such as cyclohexyl (meth)acrylate, norbornyl (meth)acrylate, or adamantyl (meth)acrylate), aryl (meth)acrylates (such as phenyl (meth)acrylate, chlorophenyl (meth)acrylate, methoxyphenyl (meth)acrylate, or naphthyl (meth)acrylate) and aralkyl esters (such as benzyl (meth)acrylate, or phenethyl (meth)acrylate).

Among these, benzyl (meth)acrylate and cyclohenyl (meth)acrylate are preferable in terms of raw material cost, solubility and pigment dispersibility.

Also examples of another monomer which can copolymerize with these structural units include alkyl (meth) acrylates (for example, $C_1$ to $C_{18}$ alkyl esters of (meth)acrylic acid, such as methyl (meth)acrylate, ethyl (meth)acrylate, propyl (meth)acrylate, isopropyl (meth)acrylate, n-butyl (meth)acrylate, isobutyl (meth)acrylate, t-butyl (meth)acrylate, hexyl (meth)acrylate, octyl (meth)acrylate, 2-ethylhexyl (meth)acrylate, lauryl (meth)acrylate or stearyl (meth)acrylate); aralkyl (meth)acrylates (such as benzyl (meth)acrylate), substituted alkyl (meth)acrylates (such as dimethylaminoethyl (meth)acrylate, diethylaminoethyl (meth)acrylate, dimethylaminopropyl (meth)acrylate or diethylaminopropyl (meth)acrylate), (meth)acrylamides (such as (meth)acrylamide, dimethyl (meth)acrylamide, isopropyl (meth)acrylamide, or t-butyl (meth)acrylamide), substituted (meth)acrylamides (such as (meth)acryloyl morpholine or dimethylaminopropyl (meth) acrylamide), aromatic vinyl compounds (such as styrene, vinyltoluene or .alpha, methylstyrene), heterocyclic vinyl compounds (such as vinylimidazol or vinylpyridine), vinyl esters (such as vinyl acetate, vinyl propionate or vinyl barsatate), N-vinylamides (such as N-vinylpyrrolidone, N-vinylformamide or N-vinylacetoamide), allyl esters (such as allyl acetate), halogen-containing monomers (such as vinylidene chloride or vinyl chloride), vinyl cyanides (such as (meth)acrylonitrile), and olefins (such as ethylene or propylene).

Among these, alkyl (meth)acrylates, such as methyl (meth)acrylate, ethyl (meth)acrylate, propyl (meth)acrylate, isopropyl (meth)acrylate, n-butyl (meth)acrylate, isobutyl (meth)acrylate, t-butyl (meth)acrylate, hexyl (meth)acrylate, octyl (meth)acrylate, or 2-ethylhexyl (meth)acrylate are particularly preferable in terms of the copolymerizing property, the solubility of the resulting polymer in solvents and the film forming property of the obtained polymer.

As for copolymerization composition ratio of these components, "the structural unit represented by General Formula (1)" is preferably 20 mol% to 80 mol%, more preferably 20 mol% to 75 mol%, and particularly preferably 25 mol% to 75 mol%. The structural unit represented by General Formula (1) falling within the above range may be excellent in hardening property and developability. "The structural unit having a carboxyl group" is preferably 10 mol% to 40 mol%, more preferably 15 mol% to 35 mol%, and particularly more preferably 20 mol% to 35 mol%. The structural unit having a carboxyl group falling within the above range may be excellent in develop ability, and in resistant to developer in an image portion. Moreover, "the structural unit having a (meth)acrylate having at least an aromatic ring and/or aliphatic ring" is preferably 10 mol% to 70 mol%, more preferably 10 mol% to 60 mol% and particularly preferably 10 mol% to 50 mol%. The structural unit having a (meth)acrylate having at least an aromatic ring and/or aliphatic ring falling within the above range may be excellent in pigment dispersibility, developability and hardening property.

The copolymer suitably used as the binder preferably has a mass-average molecular weight of 5,000 to 200,000, more preferably 10,000 to 100,000 and particularly preferably 12,000 to 80,000. The copolymer having a mass average molecular weight within the above range is preferable in terms of production adequacy and developability of the copolymer.

Specific examples of the copolymer suitably used as the binder are shown as follows, but the invention is not limited by such example compounds. The following copolymers can be referred to JP-A Nos. 2003-131379 and 2003-207787.

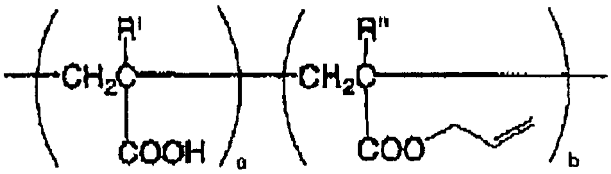

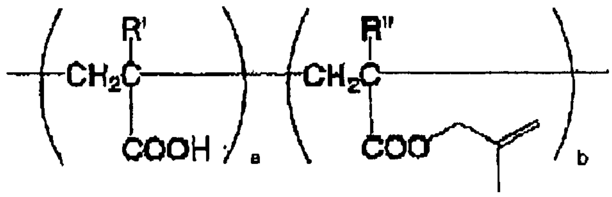

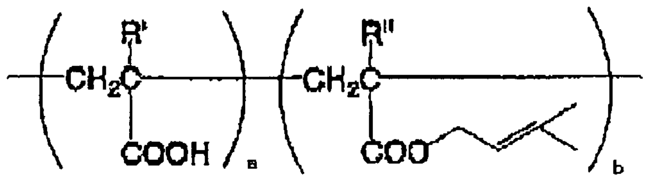

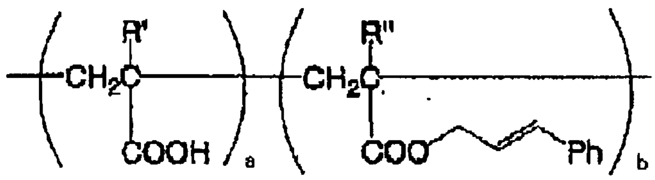

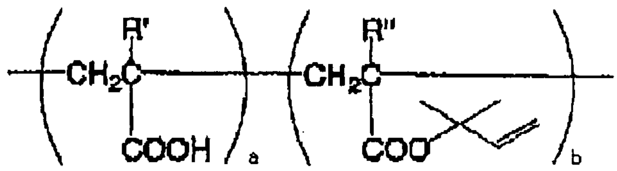

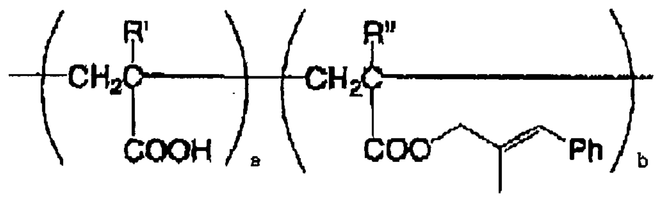

In the above formulas, R' and R" each independently represents a hydrogen atom or a methyl group; a = 10 to 40; and b = 90 to 60.

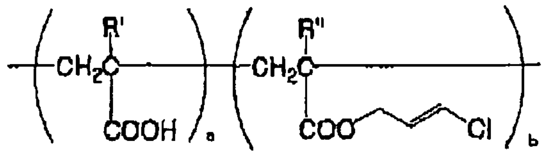

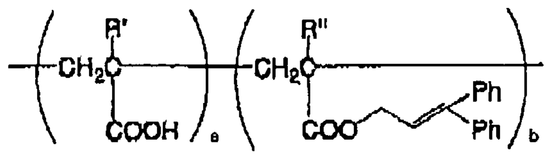

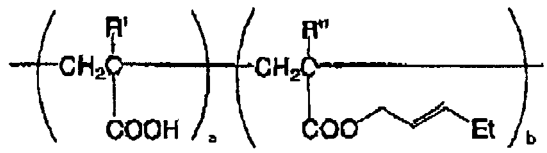

In the above formulas, R' and R" each independently represents a hydrogen atom or a methyl group; a = 10 to 40; and b = 90 to 60.

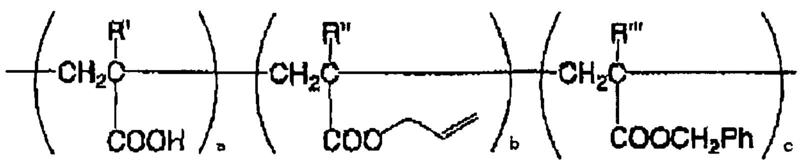

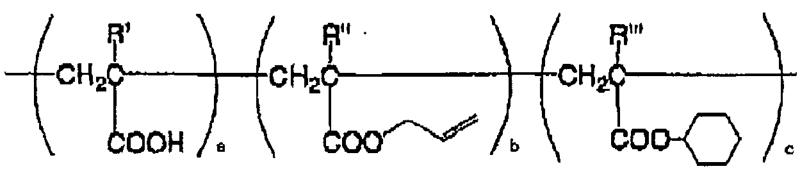

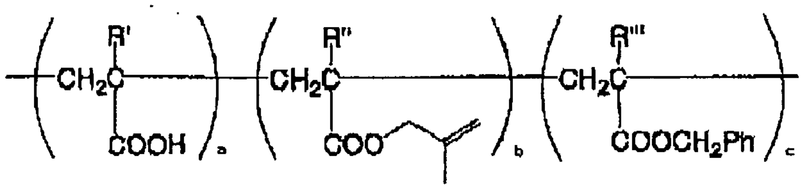

In the above formulas, R', R" and R"' each independently represents a hydrogen atom or a methyl group; a = 10 to 40; b = 80 to 20; and c = 10 to 40.

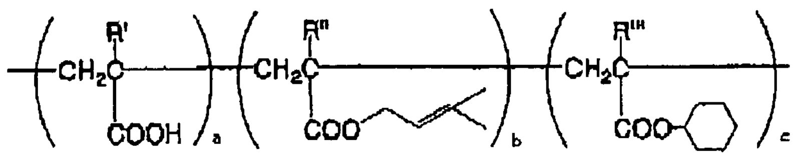

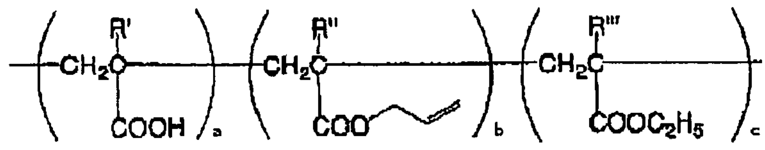

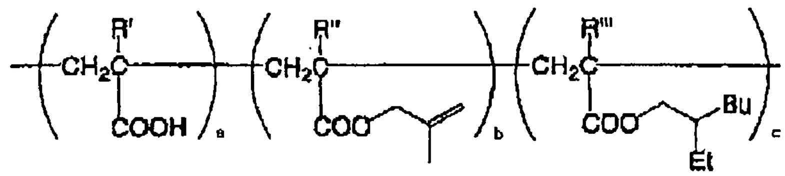

In the above formulas, R', R" and R"' each independently represents a hydrogen atom or a methyl group; a = 10 to 40; b = 80 to 20; and c = 10 to 40.

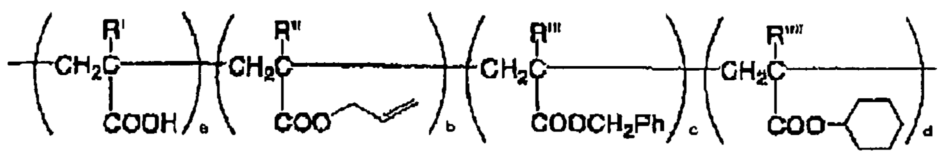

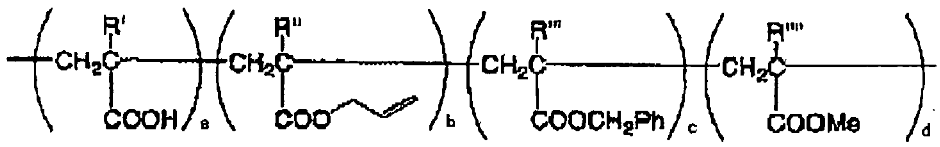

In the above formulas, R', R", R"' and R"" each independently represents a hydrogen atom or a methyl group; a = 10 to 40; b = 80 to 20; c = 5 to 20; and d = 5 to 20.

The copolymer suitably used as the binder can be obtained by copolymerizing the respectively corresponding monomers by an ordinary known method. For example it can be obtained by dissolving these monomers in a suitable solvent, adding a radical polymerization initiator and carrying out a polymerization reaction in liquid.

The suitable solvent for the above-mentioned copolymerization can be arbitrarily selected according to the monomers to be used and the solubility of the resulting copolymer, and examples thereof include methanol, ethanol, propanol, isopropanol, 1-methoxy-2-propanol, acetone, methyl ethyl ketone, methyl isobutyl ketone, methoxypropyl acetate, ethyl lactate, ethyl acetate, acetonitrile, tetrahydrofuran, dimethylformamide, chloroform, toluene and a mixture thereof. Moreover, the polymerization initiator can be used, and examples thereof include azo compounds such as 2,2'-azobis(isobutyronitrile) (AIBN), 2,2'-azobis-(2,4'-dimethylvaleronitrile); peroxides such as benzoyl peroxide; and persulfate salts.

Also a known chain transfer agent may be suitably used in order to adjust the molecular weight. It may be necessary to suitably adjust the polymerization concentration, the amount of the initiator, the chain transfer agent and the polymerization temperature. For example, the polymerization concentration is preferably 5% by mass to 50% by mass, and more preferably 10% by mass to 40% by mass.

The amount of the binder in the photosensitive composition is preferably 30% by mass to 70% by mass, and more preferably 40% by mass to 50% by mass, on solid content conversion.

< (D) Coloring Agent >

Examples of the coloring agent include dyes and a pigment. These are used depending on the needs of a member of the color filter formed from the photosensitive composition.

In the case of formation of a spacer, preferable types and sizes of the pigment may be appropriately selected in accordance with the description of, for instance, JP-A No. 11-149008. When the coloring agent such as the pigment is contained, a colored pixel can be formed. Examples of pigments that can be used include extender pigments and coloring pigments. The extender pigment is not particularly limited, and may be appropriately selected depending on the purpose. For instance, the extender pigments described in paragraphs [0035] to [0041] of JP-A No. 2003-302639 may be preferably cited. As the coloring pigments, pigments described in paragraph [0043] of JP-A No. 2003-302639 may be suitably cited,

< (E) Solvent >

The solvent of the solution is not particularly limited and may be appropriately selected depending on the purpose. Examples thereof include alcohols such as methanol, ethanol, n-propanol, isopropanol, n-butanol, sec-butanol, and n-hexanol; ketones such as acetone, methyl ethyl ketone, methyl isobutyl ketones, cyclohexanon, and diisobutyl ketone; esters such as ethyl acetates, butyl acetates, n-amyl acetate, methyl sulfate, ethyl propionate, dimethyl phthalate, ethyl benzoate, and methoxy propyl acetate; aromatic hydrocarbons such as toluene, xylene, benzene, and ethylbenzene; halogenated hydrocarbons such as carbon tetrachloride, trichloroethylene, chloroform, 1,1,1-trichloroethane, methylene chloride, and monochlorobenzene; ethers such as tetrahydrofuran, diethyl ether, ethylene glycol monomethylether, ethylene glycol monoethylether, and 1-methoxy-2-propanol; and dimethylformamide, dimethylacetoamide, dimethylsulfooxide, and sulfolane. These solvents may be used alone or in combination.

< (F) Sensitizer >

Moreover, a compound represented by the following General Formula (F-1) is preferably contained as a sensitizer.

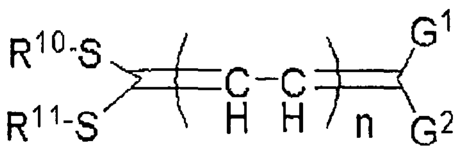

General Formula (F-1)

in General Formula (F-1), $R^{10}$ and $R^{11}$, which may be identical or different, each represent an alkyl group, an aryl group or an alkenyl group, each of which may have a substituent; $R^{10}$ and $R^{11}$ may form, together with sulfur atoms to which $R^{10}$ and $R^{11}$ are bonded, a ring composed of non-metal elements; n is an integer of 0, 1 or 2; $G^1$ and $G^2$, which may be identical or different, each represent an alkoxycarbonyl group, an aryloxylcarbonyl group, an acyl group, an arylcarbonyl group, an alkylthio group, an arylthio group, an alkylsulfonyl group, an arylsulfonyl group, a fluoroalkylsulfonyl group, each of which may have a substituent, or each represent a hydrogen atom or a cyano group; one of $G^1$ and $G^2$ is an alkoxycarbonyl group, an aryloxylcarbonyl group, an acyl group, an arylcarbonyl group, an alkylsulfonyl group, an arylsulfonyl group, a fluoroalkylsulfonyl group, each of which may have a substituent, or is a cyano group; and $G^1$ and $G^2$ may form, together with carbon atoms to which $G^1$ and $G^2$ are bonded, a ring composed of non-metal atoms.

The alkyl group for $R_1$ and $R_2$, preferably has 1 to 10 carbon atoms (for example, methyl, ethyl), and may be substituted by a substituent group such as a hydroxyl group, a cyano group, an alkoxyl group (for example, methoxy, ethoxy), a halogen atom (for example, chlorine, bromine), a carboxyl group, an alkoxycarbonyl group, a sulfonyl group, an aryl group (for example, phenyl, p-nitrophenyl), a vinyl group, a methylvinyl group, a cinnamyl group, etc.

The aryl group for $R_1$ and $R_2$, each is preferably a phenyl group or a naphthyl group. These aryl groups may

be substituted by a substituent group such as an alkyl group having 1 to 10 carbon atoms, a hydroxyl group, a cyano group, an alkoxyl group having 1 to 10 carbon atoms (for example, methoxy, ethoxy), a halogen atom (for example, chlorine, bromine), a carboxyl group, an alkoxycarbonyl group, a sulfonyl group, etc.

The alkenyl group for $R_1$ and $R_2$ is preferably a vinyl group. This alkenyl group may be substituted by a substituent group such as an alkyl group having 1 to 10 carbon atoms or an aryl group having 6 to 18 carbon atoms. These substituent groups may be further substituted by an alkyl group having 1 to 10 carbon atoms (for example, methyl).

$R_1$ and $R_2$ may form, together with sulfur atoms to which $R_1$ and $R_2$ are bonded, a ring composed of non-metal elements. Examples of the ring include 5-and 6-membered rings, and 5- and 6-membered rings condensed by an aromatic ring. These rings may contain substituent groups such as an alkyl group, an aryl group, a substituted alkyl group, a substituted aryl group, a hydroxyl group, a cyano group, an alkoxyl group, a halogen atom, a carboxyl group, an carboalkoxyl group, and a sulfonyl group.

$G_1$ and $G_2$ each represents an alkoxycarbonyl group, an aryloxycarbonyl group, an acyl group, an arylcarbonyl group, an alkylthio group, an arylthio group, an alkylsulfonyl group, an arylsulfonyl group, or a fluoroalkylaufonyl group, the alkyl groups contained in the above groups preferably have 1 to 10 carbon atoms and the aryl groups contained in the above groups are preferably a phenyl group or a naphthyl group. These alkyl and aryl groups may further contain substituent groups. Examples of the substituted groups include an alkyl group having 1 to 10 carbon atoms, a hydroxyl group, an cyano group, an alkoxyl group having 1 to 10 carbon atoms, a halogen atom, a carboxyl group, an carboalkoxyl group, a sulfonyl group, a sulfoalkoxy group, an aryl group having 6 to 18 carbon atoms, an acyl group, a vinyl group, and a cinnamyl group.

When $G_1$ and $G_2$ form a ring containing non-metallic elements together with the carbon atoms to which they are bonded respectively, the rings are generally ones used as acidic nuclei in merocyanine dyes. Examples of the acidic nuclei are as follows:

(a) 1,3-dicarbonyl nuclei, such as 1,3-indandione, 1,3-cyclohexanedione, 5,5-dimethyl-1,3-cyclohexanedione, and 1,3-dioxane-4,6-dione;
(b) pyrazolinone nuclei, such as 3-methyl-1-phenyl-2-pyrazolin-5-one, 1-phenyl-2-pyrazolin-5-on, and 1-(2-benzothiazolyl)-3-methyl-2-pyrazolin-5-one;
(c) isoxazolinone nuclei, such as 3-phenyl-2-isoxasolin-5-one, and 3-methyl-2-isoxazolin-5-one;
(d) oxyindole nuclei, such as 1-alkyl-2,3-dihydro-2-oxyindole;
(e) 2,4,6-triketohexahydropyrimidine nuclei, such as barbituric acid, 2-thiobarbituric acid, and derivatives thereof including 1-alkyl (e.g., 1-methyl, 1-ethyl) derivatives, 1,3-dialkyl (e.g., 1,3-diethyl, 1,3-dibutyl) derivatives, 1,3-diaryl (e.g., 1,3-diphenyl, 1,3-di(p-chlorophenyl), 1,3-di(p-ethoxycarbonylphenyl)) derivatives, and 1-alkyl-2-aryl (e.g., 1-ethyl-3-phenyl) derivatives;
(f) 2-thio-2,4-thiazolidinedione nuclei, such as rhodanine and derivatives thereof including 3-alkylrhodanine (e.g., 3-ethylrhodanine, 3-allylrhodanine), and 3-arylrhodanine (e.g., 3-phenylrhodanine);
(g) 2-thio-2,4-oxazolidinedione (2-thio-2,4-(3H, 5H)-oxazoledione) nuclei, such as 2-ethyl-2-thio-2,4-oxazolidinedione;
(h) thianaphthenone nuclei, such as 3(2H)-thianaphthenone, and 3(2H)-thianaphthenone-1,1-dioxide;
(i) 2-thio-2,5-thiazolidinedione nuclei, such as 3-ethyl-2-thio-2,5-thiazolidinedione;
(j) 2,4-thiazolidinedione nuclei, such as 2,4-thiazolidinedione, 3-ethyl-2,4-thiazolidinedione, and 3-phenyl-2,4-thiazolidinedione;
(k) thiazolidione nuclei, such as 4-thiazolidinone, 3-ethyl-4-thiazolidinone;
(l) 4-thiazolidinone nuclei, such as 2-ethylmercapto-5-thiazolin-4-one, and 2-alkylphenylamino-5-thiazolin4-one;
(m) 2-imino-2-oxozolin-4-one(pseudohydantoin)nuclei;
(n) 2,4-imidazolidinedione(hydantoine)nuclei, such as 2,4-imidazolidinedione, and 3-ethyl-2,4-imidazolidinedione;
(o) 2-thio-2,4-imidazolidinedione(2-thiohydantoine)nuclei, such as 2-thio-2,4-imidazolidinedione, and 3-ethyl-2-thio-2,4-imidazolidinedione;
(p) 2-imidazolin-5-one nuclei, such as 2-n-propylmercapto-2-imidazolin-5-one; and
(q) furan-5-one.
(r) 4-hydroxy-2(1H)-quinolinone nuclei or 4-hydroxy-2(1H)-pyridinone nuclei such as N-methyl-4-hydroxy-2(1H)-quinolinone, N-n-butyl-4-hydroxy-2(1H)-quinolinone and N-methyl-4-hydroxy-2(1H)-pyridinone,
(s) substituted or unsubstituted 4-hydroxy-2H-pyran-2-one and 4-hydroxycoumarin, and
(t) substituted or unsubstituted thioindoxyl such as 5-methylthioindoxyl.

The sensitizer represented by General Formula (F-1) is preferably a compound represented by General Formula (F-2):

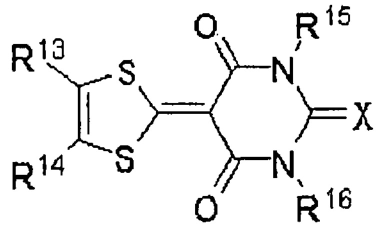

General Formula (F-2)

in General Formula (F-2), $R^{13}$ and $R^{14}$, which may be identical or different, each represent a hydrogen atom, an alkyl group, an aryl group, an acyl group or a halogen atom; $R^{13}$ and $R^{14}$ may be linked together to form an aromatic ring which may have an alkyl group, an alkyloxy group or a halogen atom; $R^{15}$ and $R^{16}$, which may be identical or different, each represent a hydrogen atom, an alkyl group or an aryl group; and X represents an oxygen atom or a sulfur atom.

Specific examples of the sensitizers represented by the above General Formula (F-1) include as follows:

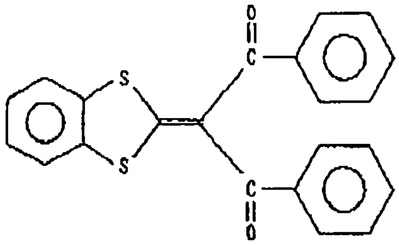

No. 1

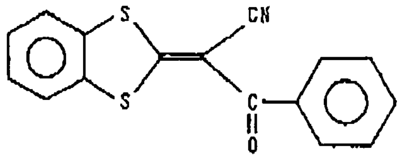

No. 2

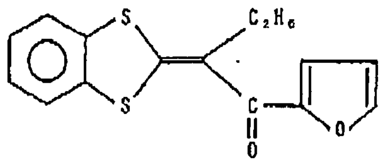

No. 3

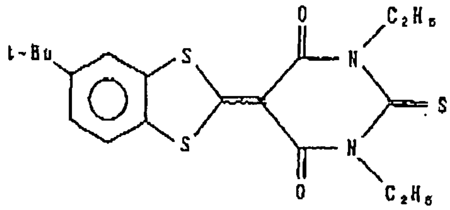

No. 4

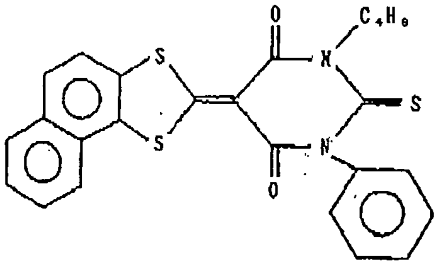

No. 5

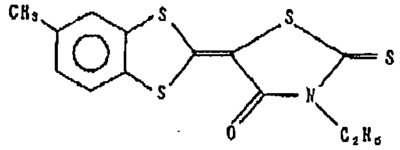

No. 6

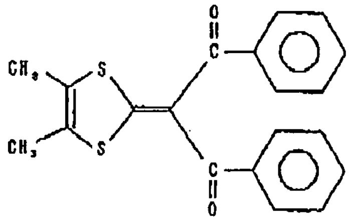

No. 9

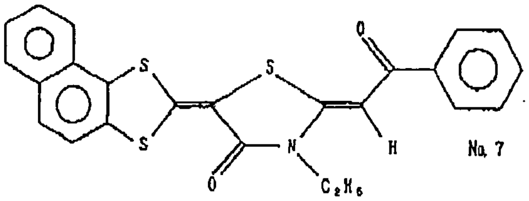

No. 7

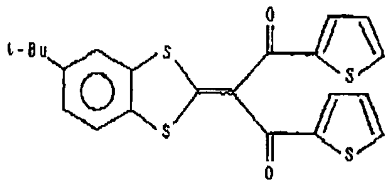

No.10

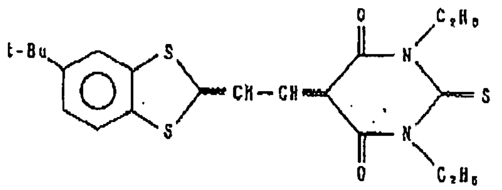

No. 8

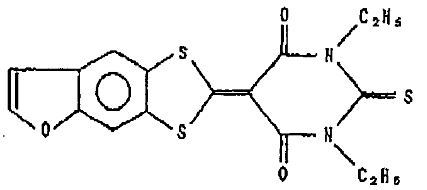

No.11

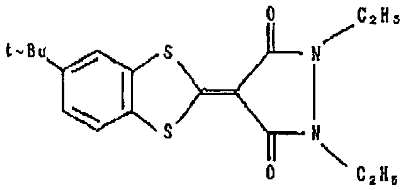

No.14

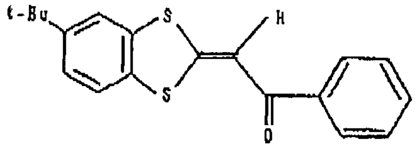

No.12

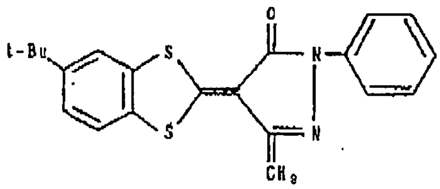

No.15

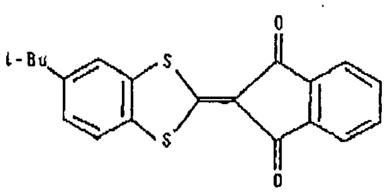

No.13

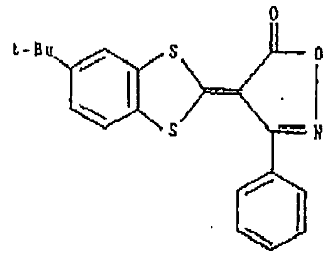
No.16

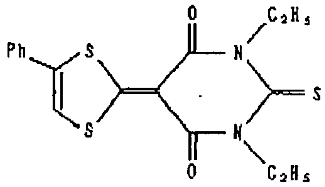
No.19

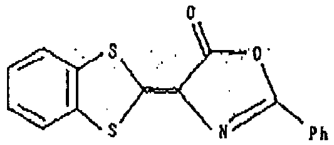
No.17

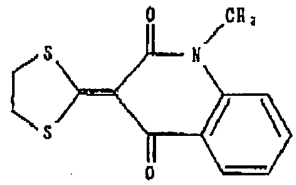
No.20

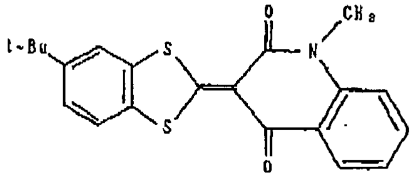
No.18

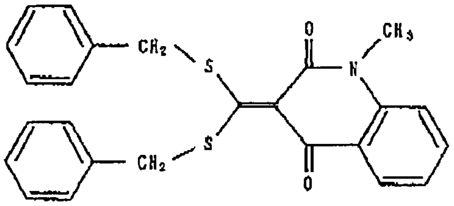
No.21

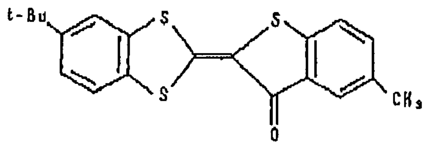
No.24

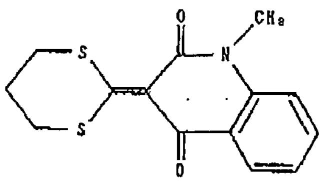
No.22

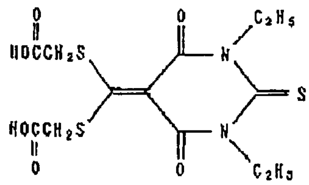
No.25

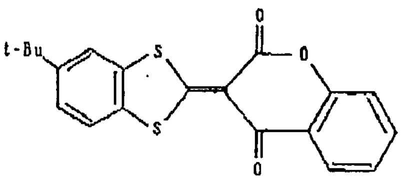
No.23

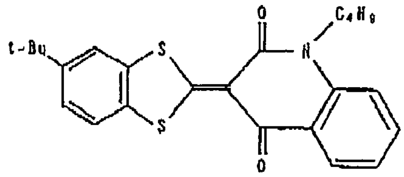

No.26

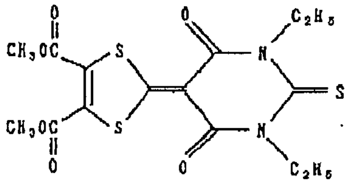

No.27

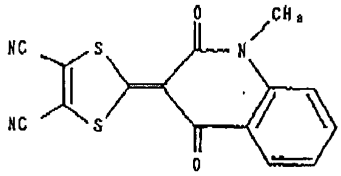

No.28

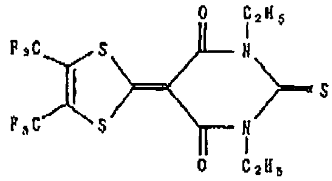

No.29

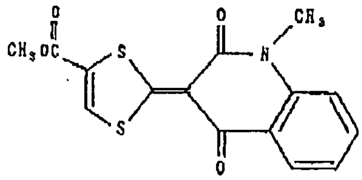

No.30

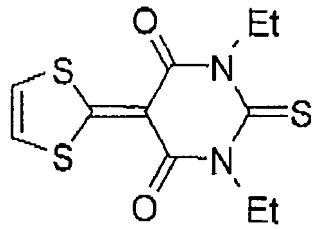

No.31

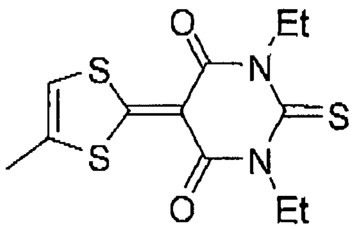

No.32

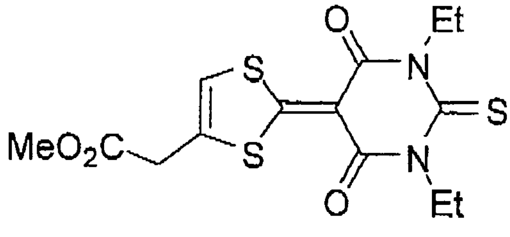

No.33

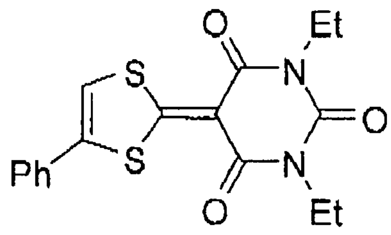

No.34

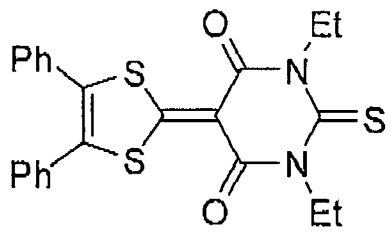

No.35

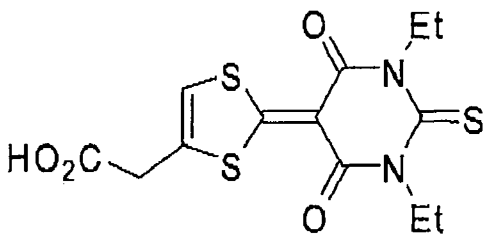

No36

In the above specific examples t-Bu represents a tert-butyl group; and Ph represents a phenyl group.

Moreover, other sensitizers may be contained. In this case, such other sensitizers are selected so that the photopolymerization velocity of the resulting composition is further increased when they are used in combination with the sensitizers represented by Genearal Formula (F-1). Examples of such sensitizers include benzophenone derivatives, benzanthrone derivatives, quinones, anthraquinones, aromatic nitro compounds, naphthothiazoline derivatives, benzothiazoline derivatives, xanthones, naphthothiazole derivatives, ketocoumalin derivatives, benzothiazole derivatives, naphthofuranone compounds, benzoin compounds, acetophenone compounds, fluorenone compounds, pyrylium salts and thiapyrylium salts. Specific examples thereof include Michler's ketone, 4,4'-bis(diethylamino)benzophenone, benzanthrone, (3-methyl-1,3-diaza-1,9-benz)anthrone picramide, 5-nitroacenaphthene, 2-nitrofluorene, 2-dibenzoylmethylene-3-methylnaphthothiazoline, 3,3-carbonyl-bis(7-diethylaminocoumalin), 2,4,6-triphenylthiapyrylium perchlorate, 2-(p-chlorobenzoyl)naphthothiazole, benzoin, benzoin methyl ether, benzoin ethyl ether, 2,2-dimethoxy-2-phenylacetophenone, 9-fluorenone, 2-chloro-9-fluorenone, 2-methyl-9-fluorenone, 9,10-anthraquinone, 2-ethyl-9,10-anthraquinone, 2-t-butyl-9,10-anthraquinone, 2,6-dichloro-9,10-anthraquinone, xanthone, 2-methyl xanthone, 2-methoxy xanthone, dibenzalacetone, p-(dimethylamino)phenyl styryl ketone and p-(dimethylamino)phenyl-p-methyl styryl ketone.

< Other Component >

In addition to the above components (A) to (F), the photosensitive composition of the present invention further contains other known components such as surfactants, polymerization inhibitors, ultraviolet absorbents, as necessary.

- Surfactant -

Any surfactants are applicable as long as they are miscible with the components of the photosensitive composition. Surfactants preferably applicable to the present invention include those disclosed in paragraphs [0015] to [0024] in JP-A No. 2003-337424, paragraphs [0012] to [0017] in JP-A No. 2003-177522, paragraphs [0012] to [0015] in JP-A No. 2003-177523, paragraphs [0010] to [0013] in JP-A No. 2003-177521, paragraphs [0010] to [0013] in JP-A No.

2003-177519, paragraphs [0012] to [0015] in JP-A No. 2003-177520, paragraphs [0034] to [0035] in JP-A No. 11-133600 and those disclosed as the invention in JP-A No. 6-16684.

In order to obtain higher effects, it is preferable to use any of fluorine-containing surfactants and/or silicon surfactants (fluorine-containing surfactant, silicon surfactant, or surfactant containing both of fluorine atom and silicon atom), or two or more surfactants selected therefrom. Of these, the fluorine-containing surfactant is most preferable.

When the fluorine-containing surfactant is used, the number of fluorine atoms contained in the fluorine-containing substituents in one surfactant molecule is preferably 1 to 38, more preferably 5 to 25, most preferably 7 to 20. The number of fluorine atoms within the above range is preferable in terms of excellent solbility and improvement effect of unevenness.

Particularly preferable surfactants can be those containing a copolymer which includes a monomer A represented by Genearl Formula (a) and a monomer B represented by Genearl Formula (b), having a copolymerization ratio of the monomer A to the monomer B [A/B (mass ratio)] is 20/80 to 60/40:

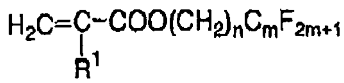

Genearl Formula (a)

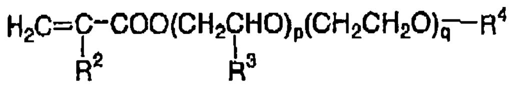

Genearl Formula (b)

In General Formulas (a) and (b), each of $R^1$, $R^2$ and $R^3$ represents a hydrogen atom or a methyl group. Preferably, each of $R^1$ and $R^2$ represents a hydrogen atom, and $R^3$ represents a methyl group.

$R^4$ represents a hydrogen atom or an alkyl group having 1 to 5 carbon atoms. Examples of the alkyl group represented by $R^4$ include methyl, ethyl, propyl and butyl. In particular, alkyl groups each having 1 or 2 carbon atoms are preferred. Particulalry preferably, $R^4$ is a hydrogen atom.

In General Formula (a), n is an integer of 1 to 18, preferably 2 to 10, and m is an integer of 2 to 14, preferably 4 to 12. $C_mF_{2m+1}$ in General Formula (a) may be a linear or branched chain. The ratio of $C_mF_{2m+1}$ to monomer A is preferably 20% by mass to 70% by mass, particularly preferably 40% by mass to 60% by mass.

In General Formula (b), each of p and q is an integer of 0 to 18, preferably 2 to 8, with the proviso that the case where both p and q are 0 is excluded.

In the surfactant suitably used in the present invention, a plurality of monomers A contained in one molecule thereof may have the same structure or different structures. Also, the same applies to monomer B.

The surfactant is a copolymer produced through copolymerization of monomer A, monomer B, and other monomers than polymers A and B preferably at a proportion of 20% by mass to 60% by mass (monomer A): 80% by mass to 40% by mass (monomer B): balance (% by mass) (the other monomers), more preferably at a proportion of 25% by mass to 60% by mass (monomer 10: 75% by mass to 40% by mass (monomer B): balance (% by mass) (the other monomers), still more preferably at a proportion of 25% by mass to 60% by mass (monomer A) : 60% by mass to 40% by mass (monomer B) : balance (% by mass) (the other monomers).

Among them, preferred surfactants are produced by copolymerizing monomer A represented by General Formula (a) where $R^1$ is a hydrogen atom, n = 2 and m = 6 with monomer B represented by General Formula (b) where each of $R^2$ and R is a hydrogen atom, $R^3$ is a methyl group, p = 7 and q = 0 or where each of $R^2$, $R^3$ and $R^4$ is a hydrogen atom, p = 0 and q = 7 at a copolymerization ratio of 20/80 to 60/40 (A/B). Particularly preferred surfactants are produced by copolymerizing these monomers A and B at a copolymerization ratio of 25/60 to 60/40.

Specific examples of the surfactants are given in Table 1 of paragraph [0068] in JP-A No. 2003-337424.

- Polymerization Inhibitor -

The photosensitive composition may further contain a polymerization inhibitor. Examples of the polymerization inhibitor include known polymerization inhibitors such as a phenolic hydroxy group-containing compounds, N-oxide compounds, piperidine-1-oxyl free radical compounds, pyrrolidine-1-oxyl free radical compounds, N-nitrosophenyl hydroxylamines, and cationic dyes.

Examples of phenolic hydroxy group-containing compounds include a phenolic hydroxy group is selected from

the group consisting of hydroquinone, p-methoxyphenol, di-tert-butyl-p-cresol, pyrogallol, tert-butylcatechol, benzoquinone, 4,4-thiobis(3-methyl-6-tert-butylphenol),
2,2'-methylenebis(4-methyl-6-tert-butyl-phenol), phenol resins and cresol resins.

Example of the N-oxide compounds include 5,5-dimethyl-1-pyrrolin-N-oxide, 4-methylmorpholine-N-oxide, pyridine-N-oxide, 4-nitropyridine-N-oxide, 3-hydroxypyridine-N-oxide, picolinic acid-N-oxide, nicotinic acid-N-oxide and isonicotinic acid-N-oxide.

Examples of the piperidine-1-oxyl free radical compounds include piperidine-1-oxyl free radical, 2,2,6,6-tetramethylpiperidine-1-oxyl free radical, 4-oxo-2,2,6,6-tetramethylpiperidine-1-oxyl free radical, 4-hydroxy-2,2,6,6-tetramethylpiperidine-1-oxyl free radical, 4-acetamido-2,2,6,6-tetramethylpiperidine-1-oxyl free radical, 4-maleimido-2,2,6,6-tetramethylpiperidine-1-oxyl free radical and 4-phophonoxy-2,2,6,6-tetramethylpiperidine-1-oxyl free radical.

Examples of the pyrrolidine-1-oxyl free radical compounds include 3-carboxyproxyl free radical (3-carboxy-2,2,5,5-tetramethylpyrrolidine-1-oxyl free radica).

Examples of the N-nitrosophenyl hydroxylamines include N-nitrosophenylhydroxylamine primary cerium salts and N-nitrosophenylhydroxylamine aluminum salts.

Examples of the cationic dyes include Crystal Violet, Methyl Violet, Ethyl Violet and Victoria Pure Blue BOH.

Particularly, piperidine 1-oxyl free radical compounds (including derivatives having these skeletons), such as 4-hydxoxy-2,2,6,6-tetramethylpiperidine-1-oxyl free radical, are preferably used because they are effective in small amount.

Additilly, N-oxide compounds, particularly, compounds having pyridine N-oxide skeleton are also preferably used.

- Ultraviolet Absorbent. -

The photosensitive composition may contain an ultraviolet absorbent, as necessary. Examples of the ultraviolet absorbents include compounds described in JP-A No. 05-72724, salicylates, benzophenones, benzotriazoles, cyanoacrylates, nickel chelates, and hindered amines.

Specific examples thereof include phenyl salicylate, 4-t-butylphenyl salicylate, 2,4-di-t-butylphenyl-3',5'-di-t-butyl-4'-hydroxybenzoate, 4-t-butylphenyl salicylate, 2,4-dihydroxybenzophenone, 2-hydroxy-4-methoxybenzophenone, 2-hydroxy-4-n-octoxybenzophenone, 2-(2'-hydroxy-5'-methylphenyl)-benzotriazole, 2-(2'-hydroxy-3'-t-butyl-5'-methylphenyl)-5-chlorobenzotriazole, ethyl-2-cyano-3,3'-diphenyl acrylate, 2,2'-dihydroxy-4-methoxybenzophenone, nickel dibutyl dithiocarbamate, bis(2,2,6,6-tetramethyl-4-pyridine)sebacate, 4-t-butylphenyl salicylate, phenyl salicylate, 4-hydroxy-2,2,6,6-tetramethylpiperidine condensate, bis(2,2,6,6-tetramethyl-4-piperidinyl) succinate, 2-[2-hydroxy-3,5-bis($\alpha$,$\alpha$-dimethylbenzyl)phenyl]-2H-benzotriazole, and 7-{[4-chloro-6-(diethylamino)-5-triazin-2-yl]amino}-3-phenylcoumarin.

(2) Method for Producing Color Filter

Hereinafter, a method for producing a color filter using the photosensitive composition of the present invention will be specifically explained with a unique exposure system of the present invention.

The method for producing the color filter of the present invention includes a step of forming a photosensitive layer by applying a photosensitive composition for liquid crystal display element onto a substrate surface, and drying the applied photosensitive composition so as to form a photosensitive layer, and an step of patternwise exposing and curing the photosensitive layer so as to have a desired pattern, and a step of developing the exposed photosensitive layer to remove uncured portions. Hereinafter, the method for producing the color filter is referred to as a coating method (liquid resist method).

< Photosensitive Layer Forming Step >

Firstly, the photosensitive layer forming step in the coating method will be explained. In this step, the photosensitive composition is dissolved or dispersed in an appropriate liquid so as to prepare a coating liquid, and the coating liquid is applied to a substrate surface, and then dried to form a photosensitive layer.

The solvent or dispersant is not particularly limited, and may be appropriately selected from known liquids, for example water, or organic solvents depending on the composition of the photosensitive composition.

The method for applying the coating liquid to the substrate surface is not particularly limited and may be appropriately selected from known methods. Examples thereof include spin coating, curtain coating, slit coating, dip coating, air knife coating, roller coating, wire bar coating, gravure coating, or extrusion coating using a hopper described in U.S. Pat. No. 2,681,294. Among these, a method using a slit coater having a slit nozzle having a slit at a portion through which a liquid is discharged, is preferably used.

Examples of the slit coater havaing a slit nozzle include slit nozzles and slit coaters described in JP-A Nos. 2004-89851, 2004-17043, 2003-170098, 2003-164787, 2003-10767, 2002-79163, and 2001-310147.

A method for drying a coated film containing the photosensitive composition on the support may be appropriately selected from known methods in the art. The drying conditions vary depending on, for example, the formulation of a photosensitive composition used and/or on the type of a solvent used. In general, the temperature during drying is preferably 70°C to 150°C.

When the photosensitive layer is formed by a coating method, the thickness of the layer is preferably 0.5 μm to 10.0 μm, more preferably 1 μm to 6 μm. When the thickness falls within the above range, pinholes are prevented from being formed in the coated film during film formation. Thus, unexposed portions can be removed through development within an appropriate time without adversely affecting exposed/cured portions.

- Slit coating rate -

The coating rate at which the support is coated with the photosensitive composition is preferably 100 mm/sec to 300 mm/sec, more preferably 120 mm/sec to 280 mm/sec, particularly preferably 140 mm/see to 250 mm/sec, in order to attain both reduction of uneven coating and increase in production efficiency.

When the coating rate is lower than 100 mm/sec, time required for pre-ejection, etc. must be shortened for maintaining productivity, which may cause increase in uneven coating. Whereas when the coating rate exceeds 300 mm/sec, liquid shortage may often occur.

In the case of a slit coating apparatus in which the support is moved, the coating rate is substantially the same as the rate at which the support is conveyed to the coating apparatus. In the case of a slit coating apparatus whose slit nozzle is moved, the coating rate is substantially the same as the rate at which the slit nozzle is moved.

In the above-described production method, the photosensitive composition is applied onto the support surface to form a photosensitive layer. If necessary, other layers may by formed in combination. Examples of the other layers optionally provided include a protective layer having an oxygen barrier property for preventing inhibition of polymerization by oxygen in the curing reaction of the photosensitive layer, an adhesive layer for improving interlayer adhesiveness, and a light barrier layer which prevents transmittance of light with a wavelength falling within a desired region. These layers may be formed by a coating method similar to the case of the photosensitive layer. Alternatively, they may be pre-formed so as to have a sheet shape and then be laminated.

Examples of the support on which the photosensitive layer is formed include transparent supports (e.g., glass supports and plastic supports), transparent conductive film (e.g., an ITO film)-formed support, color filter-formed supports (which may be referred to as a "color filter support") and drive elements (e.g., thin film transistors (TFTs)-formed drive support. The thickness of the support may be appropriately determined depending on the intended use of the formed color filter. In general, it is preferably 700 μm to 1,200 μm.

The size of the support is not particularly limited and may be appropriately selected depending on the purpose. It is preferably 1 $m^2$ or greater from the viewpoint of realizing a large-scale liquid crystal display device. Coating of the large-scale support with a conventional photosensitive composition causes problematic uneven coating. In contrast, the photosensitive composition of the present invention can be satisfactorily applied onto a support with a size of 1 $m^2$ or greater, and is suitably used for a slit coating method.

Also, when the support is subjected to coupling treatment in advance, preferred adhesiveness between the support and the photosensitive layer can be obtained. The coupling treatment is preferably a method described in JP-A No. 2000-39033.

< Exposing step >

The photosensitive layer formed in the photosensitive layer-forming step is patternwise exposed, whereby exposed portions are cured. A pattern is formed in this exposing step and the subsequent developing step, and pixels of a color filter and members such as a spacer can be formed as desired. The exposing step is preferably carried out with an exposing method in which a two-dimensional image is formed through relative scanning exposure while modulating, based on image data, light having a wavelength of 350 nm to 420 nm by two-dimensionally arranged spatial light modulation devices.

The above exposure is scanning exposure with no use of mask pattern (e.g., a laser direct imaging system) and thus, may be called maskless exposure. Specifically, the maskless exposure used in the present invention is exposure in which a two-dimensional image is formed through relative scanning exposure while modulating light based on image data.

- Light source -

In the exposing method, an ultra-high pressure mercury lamp or a laser is used as a light source.

The ultra-high pressure mercury lamp is a discharge lamp including mercury sealed into a quartz glass tube or the like. In the ultra-high pressure mercury lamp, the vapor pressure of mercury is set to a high value to enhance the luminescence efficiency (in some ultra-high pressure mercury lamps, the vapor pressure of mercury during lighting is about 5MPa (W. Elenbaas: Light Sources, Philips Technical Library 148-150)). Among bright line spectra, i line (365 nm), h line (405 nm) and g line (436 nm) are generally used. In particular, i line (365 nm) is mainly used.

The them "laser" is an acronym for "Light Amplification by Stimulated Emission of Radiation." The laser utilizes an induced emission phenomenon that occurs in a material having a population inversion to provide a single monochromatic light having higher interference and directionality by amplification and oscillation of light waves. Excitation materials include liquid crystals, glass, liquids, dyes, and gases. Conventional lasers such as solid state lasers (YAG lasers), liquid lasers, gas lasers (argon lasers, He-Ne lasers, carbon dioxide lasers, or excimer lasers), and semiconductor lasers may be used.

The semiconductor laser is a laser using a light emitting diode that causes induced emission of coherent light by pn junction when electrons and holes flow out to a junction area, for example, by carrier injection, excitation with electron beams, ionization by collision, or photoexcitation, The wavelength of the emitted coherent light depends on the semiconductor compound.

The wavelength of laser is not particularly limited. From the viewpoints of resolution, cost of a laser device, and availability, in the case of a semiconductor laser, the wavelength preferably falls within a range of 300 nm to 500 nm, more preferably 340 nm to 450 nm, still more preferably 360 nm to 420 nm.

Also, in the case of a solid state laser, for example, a YAG-SHG solid state laser beam of 532 nm is employed. Furthermore, in the case of a semiconductor laser excitation solid state laser, a laser beam of 532 nm, 355 nm or 266 nm is employed. In particular, a laser beam of 355 nm is preferably employed since a conventionally used photopolymerization initiator for resist is sensitive thereto. In the case of a gas laser, a KrF laser beam of 249 nm and an ArF laser beam of 193 nm are employed.

Next will be described a method in which relative scanning is performed by modifying light in the present invention.

One typical method is a method by a spatial modulation device in which micromirrors are two-dimensionally arranged, such as a digital micromirror device (DMD) (a light semiconductor developed in 1987 by Dr. Larry Hornbeck et. al. of Texas Instruments (US)).

In this method, light emitted from a light source is applied through an appropriate optical system to the DMD, where the light is reflected on each of the two-dimensionally arranged micromirrors, The thus-reflected light is applied through another optical system, etc. to the photosensitive layer, to thereby form a two-dimensionally arranged light spot image. In this state, the photosensitive layer is not exposed at points between the light spots. But, by making the light spot image slightly oblique to the direction in which the light spots are two-dimensionally arranged, the points between the light spots which form a first column are exposed to the subsequent light spot column. As a result, the photosensitive layer can be entirely exposed. An image pattern can be formed by controlling the angle of each mirror of the DMD and by being each light spot on or off. DMD-containing exposing heads arranged in different manners can respond to various substrates having different widths.

In the DMD, the brightness of the light spot has only two tones (i.e., on or off). But, when a mirror tone type spatial modulation device is used, exposure of 256 tones can be performed.

Another typical method is a method by a polygon mirror. In this method, light emitted from a light source is applied through reflection to the photosensitive layer, while scanning the photosensitive layer with the light spot of the reflected light through rotation of the flat mirror. By moving the substrate in a perpendicular direction to the scanning direction, the photosensitive layer on the substrate can be entirely exposed. An image pattern can be formed by controlling light from a light source in intensity to be on or off or a middle tone, using an appropriate method. The scanning time can be shortened by emitting a plurality of light beams from the light source.

Further, the following methods are applicable.

Specifically, an imaging method by a polygon mirror described in JP-A No. 05-150175; a method described in JP-A No. 2004-523101 (International Publication No. WO 2002/039793) in which the image of the lower layer is partially visualized, and the upper layer is exposed with a polygon mirror-containing apparatus with being positioned at the lower layer; an exposing method by a DMD-containing apparatus described in JP-A No. 2004-56080; an exposing method by a polygon mirror-containing exposing apparatus described in JP-A No. 2002-523905; an exposing method by a polygon mirror-containing exposing apparatus described in JP-A No. 2001-255661; a method in which DMD, LD and multiple exposure are used in combination described in JP-A No. 2003-50469; an exposing method in which the exposure dose is changed depending on the position on the substrate described in JP-A No. 2003-156853; and an exposing method in which the displacement is adjusted described in JP-A No. 2005-43576.

- Relative scanning exposure -

The above exposing step is carried out using an exposure head which is moved relatively to the photosensitive layer in the scanning direction. The exposure head includes a light irradiation unit and a light modulation unit, and is disposed so that the direction of pixel part columns makes a predetermined set inclination angle $\theta$ with the scanning direction of the exposure head. In the exposure head, pixel parts used in N-fold exposure (where N is a natural number of 2 or more) are specified among usable pixel parts by a usable pixel part specifying unit. For the exposure head, the pixel part control unit controls the pixel parts so that only the pixel parts specified by the usable pixel part specifying unit participate in the exposure.

In the present invention, the term "N-fold exposure" refers to exposure set so that, in substantially all areas in the exposed areas on an exposure surface on the photosensitive layer, straight lines parallel to the scanning direction of the exposure head intersect N light spot columns (pixel columns) applied onto the exposure surface. Here the term "light spot columns (pixel columns)" refer to the sequence of light spots (pixels) as pixel units, which have a smaller angle to the scanning direction of the exposure head, among the light spots (pixels) as pixel units generated by the pixel parts. The arrangement of the pixel parts is not always limited to a rectangular lattice form, and the pixel parts may be disposed, for example, in a parallelogram form.

The description of the "substantially all areas" in the exposed areas is derived from the fact that, due to inclination of the pixel part column in both-side parts of each pixel part, the number of pixel part columns in the pixel parts used that intersect a straight line parallel to the scanning direction of the exposure head is reduced and, thus, in this case, even when a plurality of exposure heads connected to each other is used, due to the occurrence of an error, for example, in mounting angle and arrangement in the exposure head, in some cases, the number of pixel part columns in the pixel parts used that intersect the straight line parallel to the scanning direction is slightly increased or decreased, and that, in a very small part equal to or smaller than the resolution in connections between pixel part columns in each pixel part used, due to the occurrence of an error, for example, in mounting angle and arrangement in the exposure head, the pitch of the pixel part along a direction orthogonal to the scanning direction is not exactly equal to the pitch of the pixel parts in other parts, and, consequently, the number of pixel part columns of the pixel parts used that intersect the straight line parallel to the scanning direction is increased or decreased within $\pm 1$.

N in the N-fold exposure may be any natural number of 2 or more without particular limitation, and the value of N may be properly selected according to the purpose. A natural number of 3 or more is preferred, and a natural number of 3 to 7 is more preferred.

The exposing device may be, for example, that described in JP-A No. 2007-41082.

In the exposing method by the exposing device, the exposing energy is appropriately determined in consideration of the composition of the photosensitive composition. The exposure is preferably performed under such energy conditions that the density of the crosslinking group contained in the cured photosensitive layer is 0.0073 mol/g or greater, the crosslinking reaction rate is 86% to 100%, and high crosslinking rate is obtained in the resin part containing crosslinking components at a high density.

Here, when the "resin part" refers to a resin part excluding solid microparticles such as a pigment and silica.

When such high crosslinking density is realized, the formed pattern does not tend to be deformed by external force. Also, good deformation recovery can be secured after plastic deformation by external force. Thus, even when a spacer or pixels are provided to form a thin cell having a thickness of 2 $\mu$m to 4 $\mu$m, the uniformity of the cell thickness can be secured to effectively prevent unevenness in an image displayed by a liquid crystal display element. Moreover, the formed pattern is excellent in uniformity and surface smoothness and thus, the ITO resistance decreases to advantageously achieve high-speed operability.

The crosslinking group density (mol/g) and crosslinking reaction rate (%) are calculated by the following equations.

Crosslinking group density = mole of crosslinked group / mass of resin component

Crosslinking reaction rate = [(initial amount of crosslinking group − post-reaction amount of crosslinking group) / initial amount of crosslinking group] × 100

Notably, the crosslinking reaction rate is specifically calculated as follows. The pre-reaction initial crosslinking group amount and the post-reaction crosslinking group amount after crosslinking reaction are measured for IR absorbance by an IR method and calculated based on a calibration curve of a known sample between its crosslinking group amount vs. IR absorbance. Then, the crosslinking reaction rate is calculated from the above equation using the obtained values.

< Developing step >

After patternwise exposure, the photosensitive layer is developed to remove their unexposed portions; i.e., uncured portions, whereby a pattern is formed.

The photosensitive layer can be developed with a conventional alkali development method. For example, the photosensitive layer formed on the support is dipped in a development bath containing a developer such as a solvent-based or water-based developer, especially an alkaline aqueous solution (an alkaline developer); or is sprayed with the developer. Then, the layer is rubbed using a rotary brush or a wet sponge, or is ultrasonicated for development. The temperature of the developer is preferably 20°C to 40°C. The pH thereof is preferably 8 to 13, more preferably 10 to 12. Also, the developed layer is preferably washed with water.

Examples of the employable developing method include known developing methods such as a paddle developing method, a shower developing method, a shower & spin developing method and a dip developing method.

When the shower developing method is employed, the exposed photosensitive layer is showered with a developer to remove uncured portions. Notably, prior to development, showering of, for example, an alkali aqueous solution in which the photosensitive layer is dissolved at low level is preferably performed to remove the thermoplastic resin layer, the intermediate layer, etc,

The alkaline aqueous solution is preferably a diluted aqueous solution of an alkaline substance. Preferably, the alkaline aqueous solution further contains a small amount of a water-miscible organic solvent.

The alkaline substance is not particularly limited, so long as the effects of the present invention is not be impeded, and may be appropriately selected depending on the purpose. Examples thereof include sodium hydroxide, potassium hydroxide, and other alkali metal hydroxides; sodium carbonate, potassium carbonate, and other alkali metal carbonates; sodium hydrogencarbonate, potassium hydrogencarbonate, and other alkali metal hydrogencarbonates; sodium silicate, potassium silicate, and other alkali metal silicates; sodium metasilicate, potassium metasilicate, and other alkali metal metasilicates; triethanolamine, diethanolamine, monoethanolamine, morpholine; tetramethylammonium hydroxide and other tetraalkylammonium hydroxides, as well as trisodium phosphate. These may be used singly or in combination.

Preferably, the alkaline aqueous solution has a concentration of the alkaline substance of 0.01% by mass to 30% by mass and has a pH of 8 to 14.

The water-miscible organic solvent may be appropriately selected depending on the purpose. Examples thereof include methanol, ethanol, 2-propanol, 1-propanol, butanol, diacetone alcohol, ethylene glycol monomethyl ether, ethylene glycol monoethyl ether, ethylene glycol mono-n-butyl ether, benzyl alcohol, acetone, methyl ethyl ketone, cyclohexanone, ε-caprolactone, γ-butyrolactone, dimethylformamide, dimethylacetamide, hexamethylphosphoramide, ethyl lactate, methyl lactate, ε-caprolactam and N-methylpyrrolidone. The amount of the water-miscible organic solvent added is preferably 0.1% by mass to 30% by mass.

Notably, the alkaline aqueous solution may further contain any of various known surfactants. When the surfactant is added to the alkaline aqueous solution, the amount thereof is preferably 0.01% by mass to 10% by mass.

In the production method of the present invention for the color filter, not only a spacer but only a pixel itself can be formed by the above pattern-forming method. Specifically, the pixels of three primary colors (RGB) can be arranged on a transparent substrate (e.g., a glass substrate) in a mosaic or stripe shape by the above pattern-forming method.

The dimension of each pixel is not particularly limited and may be appropriately selected depending on the purpose. For example, a lattice-shape pattern each side of which is 40 μm to 200 μm or stripe-shape pattern whose width is 40 μm to 200 μm can be readily formed. Also, a higher definition pattern can be formed by controlling the exposing accuracy.

Also in the production method of the present invention for the color filter, a black matrix is formed on a transparent substrate through exposure and development using a black-colored photosensitive layer, and then a photosensitive layer of any of three primary colors (RGB) is disposed in position on the black matrix, And, the photosensitive layer of each color is subjected to exposure and development, to thereby form a color filter in which pixels of three primary colors (RGB) are arranged on the transparent substrate in a mosaic or stripe shape.

(3) Liquid crystal display element

A liquid crystal display element of the present invention contains, as at least one member, the color filter produced by the above production method; and is, for example, that containing at least a liquid crystal layer and a liquid

crystal driving unit (including passive or active matrix units) provided between a pair of substrates at least one of which has optical transparency.

In this case, the liquid crystal display element may be a color filter substrate having a plurality of RGB pixels which are partitioned one another by the black matrix. This color filter substrate has a photospacer having the same height and being excellent in deformation recovery. Thus, the liquid crystal display element having the color filter substrate is prevented from forming uneven cell gap (variation in cell thickness) between the color filter substrate and the counter substrate. As a result, uneven display (e.g., unevenness in color) can be effectively prevented, enabling the liquid crystal display element to display a sharp image.

In another embodiment, the liquid crystal display element contains at least a liquid crystal layer and a liquid crystal driving unit between a pair of substrates at least one of which has optical transparency, wherein the liquid crystal driving unit contains an active element (e.g., TFT) and the interdistance of the substrates is controlled to a predetermined width by a photospacer having the same height and being excellent in deformation recovery.

Examples of the liquid crystal which can be used in the present invention include nematic liquid crystals, cholesteric liquid crystals, smectic liquid crystals and ferroelectric liquid crystals.

Also, a group of pixels of the color filter substrate may be composed of pixels of two different colors, pixels of three different colors, or pixels of four different colors. For example, in the case of three colors, three hues; i.e., red (R), green (G) and blue (B) are employed. A group of pixels of three colors RGB is preferably arranged in a mosaic or triangle shape. A group of pixels of four or more colors may be arranged in any shape. In production of the color filter, for example, a black matrix may be formed after formation of a group of pixels of two or more different colors, in contast, a group of pixels may be formed after formation of a black matrix. Regarding to formation of RGB pixels, reference may be made to JP-A No. 2004-347831.

The liquid crystal display element of the present invention is preferably used in LCD display devices. Preferred examples of the liquid crystal display mode used in the liquid crystal display device include STN, TN, GH, ECB, ferroelectric liquid crystals, antiferroelectric liquid crystals, VA, IPS, OCB and ASM. Since prevention of uneven display can be effectively attained, the liquid crystal display element of the present invention is satisfactorily used in display modes (e.g., VA, IPS and OCB display modes) using a liquid crystal cell having a thickness of 2 μm to 4 μm which cell easily cause uneven display due to change in cell thickness.

The liquid crystal display device may have the following basic configuration, for example, (a) the configuration where a drive-side substrate is disposed so as to face a counter substrate with the interposition of a spacer, and a liquid crystal material is encapsulated in the space between the two substrates, the drive-side substrate having driving elements such as thin film transistors (TFTs) and pixel electrodes (conductive layer) arrayed thereon, the counter substrate having a counter electrode (conductive layer); and (b) the configuration where a drive-side substrate is disposed so as to face a counter substrate with the interposition of a spacer, and a liquid crystal material is encapsulated in the space between the two substrates, wherein the counter substrate having a counter electrode (conductive layer).

The liquid crystal display device may be configured so as to be liquid crystal display devices of various types, described, for examples, in "Next-Generation Liquid Crystal Display Technology (edited by Tatsuo Uchida, published by Kogyo Chosakai Publishing Inc., 1994)." Among others, the liquid crystal display device of the present invention can be advantageously used to form a color TFT liquid crystal display device. The color TFT liquid crystal display devices are described in, for example, "color TFT Liquid Crystal Displays (published by Kyoritsu Shuppan Co., Ltd., 1996)."

The liquid crystal display device may be configured so as to have a general configuration using various members such as an electrode substrate, a polarizing film, a retardation film, a backlight, a spacer, a viewing angle compensation film, an anti-reflection film, a light diffusing film and an anti-glare films. These members are described in, for example, "Market of Liquid Crystal Display-related Materials and Chemicals in 1994 (by Kentaro Shima, published by CMC Publishing, 1994)," and "Current Status and Future Prospect of Liquid Crystal-related Market in 2003 (2nd vol.) (by Ryokichi Omote, published by Fuji Chimera Research Institute, Inc., 2003)."

Examples

The present invention will next be described by way of examples, which should not be construed as limiting the present invention thereto. Notably, unless otherwise specified, the unit "part(s)" is on a mass basis.

(Example 1)

- Preparation of photosensitive composition -

A photosensitive composition having the following composition was prepared.

[Composition of photosensitive composition]

RIPOXY PR-300 (concentration: 67%, product of SHOWA HIGHPOLYMER CO., LTD.): 50 parts
RIPOXY SPC-2X (concentration: 60%, product of SHOWA HIGHPOLYMER CO., LTD.): 30 parts
Dipentaerythritol hexaacrylate (DPHA, product of UCB Chemicals): 20 parts Methyl ethyl ketone: 100 parts
Oxime compound having the following Structural Formula (5) (photopolymerization initiator): 2 parts
1-Chloro-4-propoxythioxanthone (sensitizer): 1 part

The above-listed components were mixed under stirring with one another to prepare a solution of the photosensitive composition of Example 1. Notably, all the procedure was performed under yellow light.

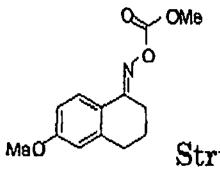

Structural Formula (5)

In Structural Formula (5), Me represents a methyl group.
The oxime compound having Structural Formula (5) was synthesized with a method described in the following Synthesis Example 1. Also, the amount of the oxime compound having Structural Formula (5) contained in the photosensitive composition was 2% by mass.

[Synthesis Example1]

6-Methoxy-1-tetralone (10 g), hydroxylamine hydrochloride (6 g) and potassium acetate (10 g) were added to ethanol (100 mL), and the resultant solution was stirred for 1 hour under heating at 100°C. After consumption of the starting materials had been confirmed through TLC, the mixture was poured into water (200 mL) for precipitation. The precipitated crystals were recovered through filtration and then recrystallized from acetonitrile (80 mL), to thereby yield 10.0 g of an oxime compound raw material.
The thus-obtained oxime compound raw material was measured for $^1$H-NMR spectrum (300 MHz, $CDCl_3$), which was found to be δ: 1.8 (q, 2H), 2.6-2.7 (m, 4H), 3.8 (s, 3H), 6.7-6.8 (m, 2H), 7.8 (d, 1H).
This oxime compound raw material (2.0 g) and pyridine (10 g) were dissolved in tetrahydrofuran (10 mL). The resultant solution was cooled to 0°C and then added dropwise to methyl chloroformate (1.5 g) at the same temperature. The mixture was increased to room temperature, followed by stirring for 2 hours. After consumption of the starting materials has been confirmed through TLC, the mixture was poured into water (60 mL). The precipitated crystals were recovered through filtration and then purified through silica gel chromatography, to thereby yield 1.5 g of the oxime compound having Structural Formula (5).
The $^1$H-NMR spectrum (300 MHz, $CDCl_3$) of the oxime compound was found to be δ: 1.9 (q, 2H), 2.7 (t, 2H), 2.9 (t, 2H), 3.8 (s, 3H), 3.9 (s, 3H), 6.6 (s, 1H), 6.8 (d, 1H), 8.1 (s, 1H).

- Formation of photosensitive layer -

The above-prepared photosensitive composition solution was applied onto a copper clad laminate (thickness of copper: 12 μm) which had been subjected to surface polishing, water washing and drying. The copper clad laminate was heated at 80°C for 15 min in a convection-type oven for solvent removal and drying, to thereby form a photosensitive layer having a thickness of 30 μm,

- Pattern formation -

-- Exposing step --

Using a semiconductor laser (central emission wavelength: 405 nm), the photosensitive layer was partially 2-fold exposed to lights having different light energies falling within a range of 0.1 mJ/cm$^2$ to 100 mJ/cm$^2$ at a $2^{1/2}$-fold rate. After left to stand still at room temperature for 10 min, the thus-exposed photosensitive layer was sprayed for 30

sec at a spraying pressure of 0.15 MPa with a 1% by mass aqueous sodium carbonate solution having a temperature of 30°C, to thereby remove/dissolve uncured portions. Subsequently, the residual cured portions were measured for thickness. Here, the light energy dose at which 90% or higher of the photosensitive layer remained was regarded as a light energy dose required for curing the photosensitive layer.

< Evaluation of sensitivity >

The light energy dose required for curing the photosensitive layer made of the photosensitive composition was evaluated as the sensitivity of the oxime compound of the present invention. The smaller the light energy dose, the higher the sensitivity; i.e., the photosensitive layer can be cured for shorter time. The results are shown in Table 1.

< Evaluation of change over time in sensitivity (storage stability) >

A photosensitive layer was produced in a manner similar to the above, followed by storing at 40°C for 3 days. Thereafter, the photosensitive layer was evaluated for sensitivity similar to the above. The results are shown in Table 1.

(Example 2)

The procedure of Example 1 was repeated, except that the oxime compound having Structural Formula (5) was changed to an oxime compound having the following Structural Formula (2), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

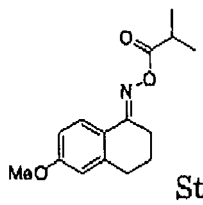

Structural Formula (2)

The oxime compound having Structural Formula (2) was prepared in a manner similar to that employed in Synthesis Example 1, except that methyl chloroformate was changed to 2-methylpropanoyl chloride.

Notably, the $^1H$-NMR spectrum (300 MHz, $CDCl_3$) of the oxime compound having Structural Formula (2) was found to be δ: 1.3 (d, 6H), 1.9 (q, 2H), 2.7-2.8 (m, 3H), 2.9 (t, 2H), 3.8 (s, 1H), 6.6 (s, 1H), 6.8 (d, 1H), 8.1 (d, 1H).

(Example 3)

The procedure of Example 1 was repeated, except that the oxime compound having Structural Formula (5) was changed to an oxime compound having the following Structural Formula (8), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

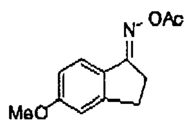

Structural Formula (8)

The oxime compound having Structural Formula (8) was synthesized as follows, Specifically, 5-methoxyindanone oxime (serving as a starting material) was reacted with hydroxylamine to form an oxime compound raw

material, and the thus-obtained oxime compound raw material was acetylated with acetic anhydride in the presence of triethylamine.

Notably, the $^1$H-NMR spectrum (300 MHz, $CDCl_3$) of the oxime compound having Structural Formula (8) was found to be 2.2 (s, 3H), 3.0 (m, 4H), 3.8 (s, 3H), 6.8 (s, 1H), 6.9 (d, 1H), 7.8 (d, 1H).

(Example 4)

The procedure of Example 1 was repeated, except that the oxime compound having Structural Formula (5) was changed to an oxime compound having the following Structural Formula (9), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

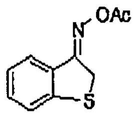

... Structural Formula (9)

The oxime compound having Structural Formula (9) was synthesized in a manner similar to that employed for synthesizing the oxime compound having Structural Formula (8) in Example 3, except that benzothiophenone was used as a starting material.

Notably, the $^1$H-NMR spectrum (300 MHz, $CDCl_3$) of the oxime compound having Structural Formula (9) was found to be δ: 2.1 (s, 3H), 4.2 (s, 2H), 7.1 (t, 1H), 7.3 (d, 1H), 7.4 (t, 1H), 7,9 (d, 1H).

(Example 5)

The procedure of Example 1 was repeated, except that the oxime compound having Structural Formula (5) was changed to an oxime compound having the following Structural Formula (10), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

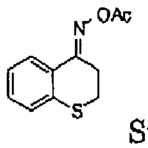

Structural Formula (10)

The oxime compound having Structural Formula (10) was synthesized in a manner similar to that employed in Example 3, except that thiochromanone was used as a starting material.

The $^1$H-NMR spectrum (300 MHz, $CDCl_3$) of the oxime compound having Structural Formula (10) was found to be δ: 2.2 (s, 3H), 3.0 (t, 2H), 3.2 (t, 2H), 7.1-7.3 (m, 3H, 8.2 (d, 1H).

(Example 6)

The procedure of Example 1 was repeated, except that the oxime compound having Structural Formula (5) was changed to an oxime compound having the following Structural Formula (16), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

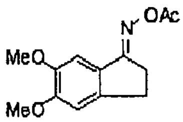

Structural Formula (16)

The oxime compound having Structural Formula (16) was synthesized in a manner similar to that employed for synthesizing the oxime compound having Structural Formula (8) in Example 3, except that dimethoxyindanone was used as a starting material.

Notably, the $^1$H-NMR spectrum (300 MHz, $CDCl_3$) of the oxime compound having Structural Formula (16) was found to be δ: 2.2 (s, 3H), 3.0 (m, 4H), 3.9 (s, 3H), 6,8 (s, 1H), 7.3 (s, 1H).

(Example 7)

The procedure of Example 1 was repeated, except that the oxime compound having Structural Formula (5) was changed to an oxime compound having the following Structural Formula (24), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

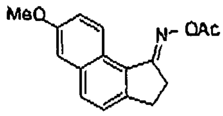

Structural Formula (24)

The oxime compound having Structural Formula (24) was synthesized as follows. Specifically, methoxynaphthaldehyde serving as a starting material was converted with malonic acid to naphthocinnamic acid, and further converted to hydroxycinnamic acid through catalytic hydrogenation. Thereafter, the hydroxycinnamic acid was further converted to methoxynaphthoindanone in polyphosphoric acid, and then oximated and acylated similar to Example 3, to thereby yield the oxime compound having Structural Formula (24).

The $^1$H-NMR spectrum (300 MHz, $CDCl_3$) of the oxime compound having Structural Formula (24) was found to be δ: 2.3 (s, 3H), 3,2 (m, 4H), 3.9 (s, 3H), 7.2 (s, 1H), 7.3 (d, 1H), 7.4 (d, 1H), 7.8 (d, 1H), 9.0 (d, 1H).

(Example 8)

The procedure of Example 1 was repeated, except that the oxime compound having Structural Formula (5) was changed to an oxime compound having the following Structural Formula (25), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

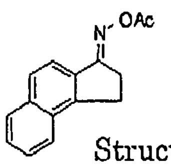

Structural Formula (25)

The oxime compound having Structural Formula (25) was synthesized in a manner similar to that employed in Example 7, except that naphthaldehyde was used as a starting material.

The $^1$H-NMR spectrum (300 MHz, $CDCl_3$) of the oxime compound having Structural Formula (25) was found

to be δ: 2.2 (s, 3H), 3.1 (t, 2H), 3.3 (t, 2H), 7.3 (d, 1H), 7.2 (t, 1H), 7.3 (t, 1H), 7.7 (d, 1H), 7.9 (d, 1H), 8.3 (d, 1H).

(Example 9)

The procedure of Example 1 was repeated, except that the oxime compound having Structural Formula (5) was changed to an oxime compound having the following Structural Formula (27), to thereby prepare a photosensitive composition. Also, similar to Example 1, a photosensitive layer and a laminate were formed using the photosensitive composition, followed by exposing and developing.
Further, in a manner similar to that employed in Example 1, the photosensitive layer was evaluated for exposure sensitivity and change in sensitivity over time. The results are shown in Table 1.

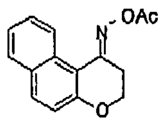

Structural Formula (27)

The oxime compound having Structural Formula (27) was synthesized in a manner similar to that employed for synthesizing the oxime compound having Structural Formula (8) in Example 3, except that naphthochromanone was used as a starting material.
Notably, the $^1$H-NMR spectrum (300 MHz, $CDCl_3$) of the oxime compound having Structural Formula (27) was found to be δ: 1.6 (m, 2H), 1.8 (m, 2H), 2.1 (s, 3H), 2.8 (m, 4H), 7.1 (d, 1H), 7.2 (t, 1H), 7.4 (t, 1H), 7.5 (d, 1H).

(Example 10)

The procedure of Example 7 was repeated, except that the amount of the oxime compound having Structural Formula (24) added to the photosensitive composition was changed from 2 parts by mass to 5 parts by mass, to thereby prepare a photosensitive composition. Similar to Example 7, a photosensitive layer and a laminate were formed using the photosensitive composition, followed by exposing and developing.
Further, in a manner similar to that employed in Example 7, the photosensitive layer was evaluated for exposure sensitivity and change in sensitivity over time. The results are shown in Table 1.

(Example 11)

The procedure of Example 7 was repeated, except that the amount of the oxime compound having Structural Formula (24) added to the photosensitive composition was changed from 2 parts by mass to 0.01 parts by mass, to thererby prepare a photosensitive composition. Similar to Example 7, a photosensitive layer and a laminate were formed using the photosensitive composition, followed by exposing and developing.
Further, in a manner similar to that employed in Example 7, the photosensitive layer was evaluated for exposure sensitivity and change in sensitivity over time. The results are shown in Table 1.

(Example 12)

The procedure of Example 6 was repeated, except that camphorquinone (2 parts by mass) was additionally used as a photopolymerization initiator, to thererby prepare a photosensitive composition. Similar to Example 6, a photosensitive layer and a laminate were formed using the photosensitive composition, followed by exposing and developing.
Further, in a manner similar to that employed in Example 6, the photosensitive layer was evaluated for exposure sensitivity and change in sensitivity over time. The results are shown in Table 1.

(Example 13)

The procedure of Example 1 was repeated, except that 1-chloro-4-propoxythioxanthone was changed to N-butyl-2-chloroacrydone, to thererby prepare a photosensitive composition. Similar to Example 1, a photosensitive layer and a laminate were formed using the photosensitive composition, followed by exposing and developing.
Further, in a manner similar to that employed in Example 1, the photosensitive layer was evaluated for exposure

sensitivity and change in sensitivity over time. The results are shown in Table 1.

(Example 14)

The procedure of Example 1 was repeated, except that the oxime compound having Structural Formula (5) was changed to an oxime compound having the following Structural Formula (26), to thereby prepare a photosensitive composition. Also, similar to Example 1, a photosensitive layer and a laminate were formed using the photosensitive composition, followed by exposing and developing.
Further, in a manner similar to that employed in Example 1, the photosensitive layer was evaluated for exposure sensitivity and change in sensitivity over time. The results are shown in Table 1.

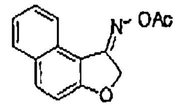

Structural Formula (26)

The oxime compound having Structural Formula (26) was synthesized as follows. Specifically, aluminum chloride (1.02 kg) was added to a suspension of 2-naphthol (1 kg) in chlorobenzene (3 L). Subsequently, chloroacetyl chloride (0.86 kg) was added thereto, and the resultant mixture was heated to 40°C and was allowed to react for 1 hour. The reaction mixture was poured into a 4N aqueous HCl solution (3 L), and then was extracted with ethyl acetate (3 L). The organic layer was washed with saturated brine, followed by addition of potassium carbonate (1.92 kg) and water (3 L). The resultant mixture was stirred at 40°C for 30 min. The organic layer was separated and then concentrated, followed by addition of a methanol (3 L) solution containing hydroxyl amine hydrochloride (1.5 kg) and acetic acid (1.4 kg). The resultant mixture was refluxed for 3 hours. The reaction mixture was poured into water (1 L) to precipitate crystals of interest. The thus-precipitated crystals were recovered through filtration and then washed with water (2 L) and methanol (1 L), to thereby yield 1.07 kg of naphtho[2,1-b]furan-1-one oxime (yield: 99%).
The thus-obtained oxime compound was reacted with acetic anhydride in the presence of triethylamin to synthesize the oxime compound having Structural Formula (26),
**[0568]** The $^1H$-NMR spectrum (300 MHz, $CDCl_3$) of the oxime compound having Structural Formula (26) was found to be δ: 2.3 (s, 3H), 5.4 (s, 2H), 7.2 (d, 1H), 7.5 (t, 1H), 7.7 (t, 1H), 7.9 (d, 1H), 8.0 (d, 1H), 8.6 (d, 1H).

(Example 15)

The procedure of Example 1 was repeated, except that the oxime compound having Structural Formula (5) was changed to an oxime compound having the following Structural Formula (11), to thereby prepare a photosensitive composition. Also, similar to Example 1, a photosensitive layer and a laminate were formed using the photosensitive composition, followed by exposing and developing.
Further, in a manner similar to that employed in Example 1, the photosensitive layer was evaluated for exposure sensitivity and change in sensitivity over time. The results are shown in Table 1.

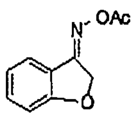

Structural Formula (11)

The oxime compound having Structural Formula (11) was synthesized in a manner similar to that employed for synthesizing the oxime compound having Structural Formula (8) in Example 3, except that benzofranone was used as a starting material.
The $^1H$-NMR spetrum (300 MHz, $CDCl_3$) of the oxime compound having Structural Formula (11) was found to δ: 2.2 (s, 3H), 5.2 (s, 2H), 7.0-7.1 (m, 2H), 7.5 (t, 1H), 7.8 (d, 1H).

(Example 16)

The procedure of Example 1 was repeated, except that the oxime compound having Structural Formula (5) was changed to an oxime compound having the following Structural Formula (29), to thereby prepare a photosensitive composition. Also, similar to Example 1, a photosensitive layer and a laminate were formed using the photosensitive composition, followed by exposing and developing.
Further, in a manner similar to that employed in Example 1, the photosensitive layer was evaluated for exposure sensitivity and change in sensitivity over time. The results are shown in Table 1.

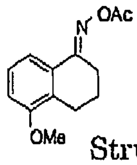

Structural Formula (29)

The $^1$H-NMR spectrum (300 MHz, $CDCl_3$) of the oxime compound having Structural Formula (29) was found to δ: 1.9 (q, 2H), 2,3 (s, 3H), 2.8 (t, 2H), 2.9 (t, 2H), 3.9 (s, 3H), 6.9 (d, 1H), 7.2 (t, 1H), 7.8 (d, 1H).

(Example 17)

The procedure of Example 1 was repeated, except that the oxime compound having Structural Formula (5) was changed to an oxime compound having the following Structural Formula (30), to thereby prepare a photosensitive composition. Also, similar to Example 1, a photosensitive layer and a laminate were formed using the photosensitive composition, followed by exposing and developing.
Further, in a manner similar to that employed in Example 1, the photosensitive layer was evaluated for exposure sensitivity and change in sensitivity over time. The results are shown in Table 1.

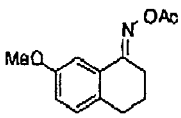

Structural Formula (30)

The $^1$H-NMR, spectrum (300 MHz, $CDCl_3$) of the oxime compound having Structural Formula (30) was found to δ: 1.9 (q, 2H), 2.3 (s, 3H), 2.8 (t, 2H), 2.9 (t, 2H), 3.8 (s, 3H), 6.9 (d, 1H), 7.1 (d, 1H), 7.6 (s, 1H).

(Example 18)

The procedure of Example 1 was repeated, except that the oxime compound having Structural Formula (5) was changed to an oxime compound having the following Structural Formula (31), to thereby prepare a photosensitive composition. Also, similar to Example 1, a photosensitive layer and a laminate were formed using the photosensitive composition, followed by exposing and developing.
Further, in a manner similar to that employed in Example 1, the photosensitive layer was evaluated for exposure sensitivity and change in sensitivity over time. The results are shown in Table 1.

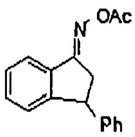

Structural Formula (31)

The $^1$H-NMR spectrum (300 MHz, $CDCl_3$) of the oxime compound having Structural Formula (31) was found

to be δ: 2.2 (s, 3H), 3.0 (dd, 1H), 3.6 (dd, 1H), 4.5-4.6 (m, 1H), 7.1 (t, 3H), 7.2-7.5 (m, 5H), 8.0 (d, 1H).

(Example 19)

The procedure of Example 1 was repeated, except that the oxime compound having Structural Formula (5) was changed to an oxime compound having the following Structural Formula (1), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

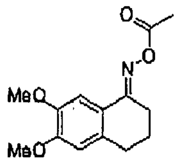

Structural Formula (1)

The $^1H$-NMR spectrum (300 MHz, $CDCl_3$) of the oxime compound having Structural Formula (1) was found to be δ: 1.9 (q, 2H), 2.3 (s, 3H), 2.7 (t, 3H), 3.8 (t, 3H), 2.9 (s, 3H), 3.92 (s, 3H), 6,6 (s, 1H), 7.6 (s, 1H).

(Example 20)

The procedure of Example 1 was repeated, except that the oxime compound having Structural Formula (5) was changed to an oxime compound having the following Structural Formula (34), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

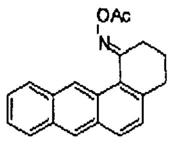

Structural Formula (34)

The $^1H$-NMR spectrum (300 MHz, $CDCl_3$) of the oxime compound having Structural Formula (34) was found to be δ: 2.0 (q, 2H), 2.4 (s, 3H), 3.0 (t, 2H), 3.1 (t, 2H), 7.3 (d, 1H), 7.5 (dd, 2H), 8.0 (d, 2H), 8.1 (d, 1H), 8.4 (s, 1H), 9.9 (s,1H).

(Example 21)

The procedure of Example 1 was repeated, except that the oxime compound having Structural Formula (5) was changed to an oxime compound having the following Structural Formula (35), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

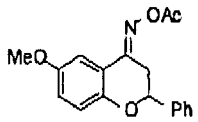

Structural Formula (35)

The $^{1}H$-NMR spectrum (300 MHz, $CDCl_3$) of the oxime compound having Structural Formula (35) was found to be δ: 2.2 (s, 3H), 2.9 (dd, 1H), 3.5 (d, 1H), 3.8 (s, 3H), 5.1 (d, 1H) 6.9-7.1 (m, 2H), 7.4-7.5 (m, 6H).

(Example 22)

The procedure of Example 1 was repeated, except that the oxime compound having Structural Formula (5) was changed to an oxime compound having the following Structural Formula (36), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

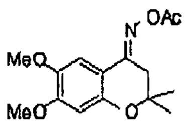

Structural Formula (36)

The $^{1}H$-NMR spectrum (300 MHz, $CDCl_3$) of the oxime compound having Structural Formula (36) was found to be δ: 1.4 (s, 6H), 2.3 (s, 3H), 2.9 (s, 2H), 3.87 (s, 3H), 3.89 (s, 3H), 6.4 (s, 1H), 7.4 (s, 1H)

(Example 23)

The procedure of Example 1 was repeated, except that the oxime compound having Structural Formula (5) was changed to an oxime compound having the following Structural Formula (37), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

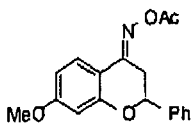

Structural Formula (37)

The $^{1}H$-NMR spectrum (300 MHz, $CDCl_3$) of the oxime compound having Structural Formula (37) was found to be δ: 2.2 (s, 3H), 2.8 (dd, 1H), 3.5 (d, 1H), 5.1 (d, 1H), 6.5 (s, 1H), 6.6 (d, 1H), 7.4-7.5 (m, 5H), 8.0 (d, 1H).

(Example 24)

The procedure of Example 1 was repeated, except that the oxime compound having Structural Formula (5) was changed to an oxime compound having the following Structural Formula (38), to thereby prepare a photosensitive composition. Also, similar to Example 1, a photosensitive layer and a laminate were formed using the photosensitive

composition, followed by exposing and developing.

Further, in a manner similar to that employed in Example 1, the photosensitive layer was evaluated for exposure sensitivity and change in sensitivity over time. The results are shown in Table 1.

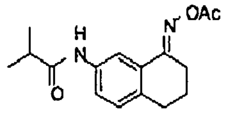

Structural Formula (38)

The $^1$H-NMR spectrum (300 MHz, $CDCl_3$) of the oxime compound having Structural Formula (38) was found to be δ: 1.3 (d, 6H), 1.9 (q, 2H), 2.3 (s, 3H), 2.5 (m, 1H), 2.8 (t, 2H), 2.9 (t, 2H), 7.2 (d, 1H), 7,4 (s, 1H), 7.8 (s, 1H), 8.1 (d, 1H).

(Example 25)

The procedure of Example 1 was repeated, except that the oxime compound having Structural Formula (5) was changed to an oxime compound having the following Structural Formula (39), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

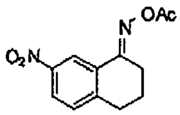

Structural Formula (39)

The $^1$H-NMR spectrum (300 MHz, $CDCl_3$) of the oxime compound having Structural Formula (39) was found to be δ: 2,0 (q, 2H), 2.3 (s, 3H), 2.9-3.0 (m, 4H), 7.4 (d, 1H), 8.2 (d, 1H), 9.0 (s, 1H).

(Example 26)

The procedure of Example 1 was repeated, except that the oxime compound having Structural Formula (5) was changed to an oxime compound having the following Structural Formula (40), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

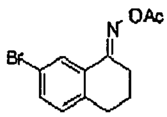

Structural Formula (40)

The $^1$H-NMR spectrum (300 MHz, $CDCl_3$) of the oxime compound having Structural Formula (40) was found to be δ: 1.9 (q, 2H), 2.3 (s, 3H), 2.7 (t, 2H), 2.9 (t, 2H), 7.1 (d, 1H), 7.5 (d, 1H), 8,3 (s, 1H).

(Example 27)

The procedure of Example 1 was repeated, except that the oxime compound having Structural Formula (5)

was changed to an oxime compound having Structural Formula (52), and that 1-chloro-4-propoxythioxanthone (sensitizer) was changed to N-butylchloroacrydone (sensitizer), to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

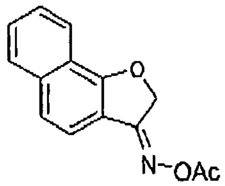

Structural Formula (52)

The oxime compound having Structural Formula (52) was synthesized in a manner similar to that employed for synthesizing the oxime compound having Structural Formula (26) in Example 14, except that 1-naphthol was used as a starting material.

The $^1H$-NMR spectrum (300 MHz, $CDCl_3$) of the oxime compound having Structural Formula (52) was found to be δ: 2,2 (s, 3H), 5.4 (s, 2H), 7.45 (d, 1H), 7.55 (t, 1H), 7.60 (t, 1H), 7.7 (d, 1H), 7.8 (d, 1H), 8.1 (d, 1H).

(Example 28)

The procedure of Example 14 was repeated, except that the amount of 1-chloro-4-propoxythioxanthone (sensitizer) added was changed to 1.6 parts by mass, to thereby prepare a photosensitive composition. Also, similar to Example 1, a photosensitive layer and a laminate were formed using the photosensitive composition, followed by exposing and developing.

Further, in a manner similar to that employed in Example 1, the photosensitive layer was evaluated for exposure sensitivity and change in sensitivity over time. The results are shown in Table 1.

(Comparative Example 1)

The procedure of Example 1 was repeated, except that the oxime compound having Structural Formula (5) (2 parts) was changed to Irgcure. OXE-01 (product of Ciba Specialty Chemicals Inc.) having the following Structural Formula (53) (5 parts by mass), to thereby prepare a photosensitive composition. Also, similar to Example 1, a photosensitive layer was formed using the photosensitive composition, followed by exposing and developing.

Further, in a manner similar to that employed in Example 1, the photosensitive layer was evaluated for exposure sensitivity and change in sensitivity over time. The results are shown in Table 1.

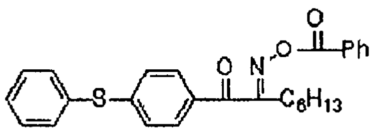

Structural Formula (53)

(Comparative Example 2)

The procedure of Example 1 was repeated, except that the oxime compound having Structural Formula (5) (2 parts) was changed to Irgcure. 907 having the following Structural Formula (54) (product of Ciba Specialty Chemicals Inc.) (5 parts by mass), to thereby prepare a photosensitive composition. Also, similar to Example 1, a photosensitive layer was formed using the photosensitive composition, followed by exposing and developing.

Further, in a manner similar to that employed in Example 1, the photosensitive layer was evaluated for exposure

sensitivity and change in sensitivity over time. The results are shown in Table 1.

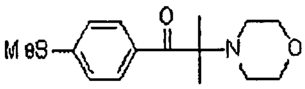

Structural Formula (54)

(Comparative Example 3)

The procedure of Example 1 was repeated, except that the oxime compound having Structural Formula (5) (2 parts) was changed to an oxime compound having Structural Formula (55) described in JP-A No. 2006-36750 (2 parts by mass), to thereby prepare a photosensitive composition. Also, similar to Example 1, a photosensitive layer was formed using the photosensitive composition, followed by exposing and developing.
Further, in a manner similar to that employed in Example 1, the photosensitive layer was evaluated for exposure sensitivity and change in sensitivity over time. The results are shown in Table 1.

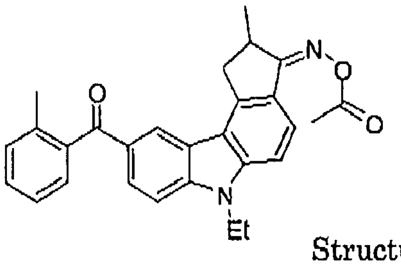

Structural Formula (55)

Table 1

| | Exposure sensitivity ($mJ/cm^2$) | Exposure sensitivity after storage ($mJ/cm^2$) |
|---|---|---|
| Ex. 1 | 30 | 35 |
| Ex. 2 | 20 | 25 |
| Ex. 3 | 15 | 25 |
| Ex. 4 | 40 | 45 |
| Ex. 5 | 45 | 45 |
| Ex. 6 | 12 | 15 |
| Ex. 7 | 10 | 15 |
| Ex. 8 | 20 | 25 |
| Ex. 9 | 40 | 45 |
| Ex. 10 | 8 | 12 |
| Ex. 11 | 40 | 40 |
| Ex. 12 | 10 | 12 |
| Ex. 13 | 20 | 25 |
| Ex. 14 | 20 | 25 |
| Ex. 15 | 15 | 25 |
| Ex. 16 | 30 | 35 |

(continued)

| | Exposure sensitivity (mJ/cm$^2$) | Exposure sensitivity after storage (mJ/cm$^2$) |
|---|---|---|
| Ex. 17 | 12 | 20 |
| Ex. 18 | 18 | 26 |
| Ex. 19 | 20 | 25 |
| Ex. 20 | 40 | 45 |
| Ex. 21 | 30 | 35 |
| Ex. 22 | 30 | 35 |
| Ex. 23 | 25 | 30 |
| Ex. 24 | 30 | 35 |
| Ex. 25 | 40 | 40 |
| Ex. 26 | 40 | 45 |
| Ex. 27 | 10 | 12 |
| Ex. 28 | 8 | 10 |
| Comp. Ex. 1 | 60 | *-- |
| Comp. Ex. 2 | 50 | *-- |
| Comp. Ex. 3 | 80 | 110 |
| *: the filme of Comp. Exe. 1 and 2 in Table 1 were completely cured before exposure and could not be evaluated | | |

In the case where the exposing system (semiconductor laser) employed in the Examples is used, image scanning must be performed and thus, it is necessary that curing can be performed at low energy (high sensitivity) in order to shorten exposure time (reduce the cost for the treatment). The sensitivity of a sensitized material can be increased by increasing the amount of a photopolymerization initiator added. But, in this case, the material cost for the sensitized material disadvantageously increases.

Also, when the sensitivity is considerably changed over time, it is difficult to control the conditions required for stably obtaining an image. Furthermore, the required exposure time is extended due to a drop in sensitivity, which problematically elevates the cost for the treatment.

For this reason, demand has arisen for a sensitized material which exhibits high sensitivity even in a small amount and which is changed in sensitivity over time to less extent (i.e., is excellent in storage stability).

As is clear from Table 1, the photosensitive layers of Examples 1 to 28, being formed from the photosensitive compositions of the present invention containing the oxime compounds of the present invention represented by General Formula (1), were found to be remarkably excellent in exposure sensitivity and storage stability (i.e., exposure sensitivity after storage), even when the oxime compound was incorporated in a small amount (2 parts by mass).

In contrast, the photosensitive layers of Comparative Examples 1 to 2, being formed from the photosensitive composition containing no oxime compound of the present invention, were not found to exhibit an increase in exposure sensitivity and were found to show a change in the quality of the films over time (i.e., to be cured before exposure), even when the amount of the sensitized agent was increased from 2 parts by mass to 5 parts by mass. Also, the photosensitive layer of Comparative Example 3 was found to exhibit poor exposure sensitivity and poor storage stability; i.e., to exhibit change in sensitivity.

(Example 29)

< Preparation of photosensitive composition K1 >

K pigment dispersion 1 and propylene glycol monomethyl ether acetate were weighed respectively in corresponding amounts shown in Table 2, and were mixed under stirring with each other at 24°C (±2°C) and 150 r.p.m. for 10 min.

Subsequently, methyl ethyl ketone, binder 1, DPHA liquid, photopolymerization initiator 1, sensitizer 1, hydroquinone monomethyl ether and surfactant 1 were weighed respectively in corresponding amounts shown in Table 2,

and were sequentially added to the resultant mixture in this order at 25°C (±2°C), followed by stirring/mixing at 40°C (±2°C) and 150 r.p.m. for 30 min, to thereby prepare photosensitive composition K1.

The concentration of solid matter in the photosensitive composition K1 was found to be 9% by mass.

Table 2

| Photosensitive resin composition K1 | |
|---|---|
| K Pigment dispersion 1 (carbon black) | 19 parts |
| Propylene glycol monomethyl ether acetate | 15 parts |
| Methyl ethyl ketone | 54 parts |
| Binder 1 | 8.1 parts |
| DPHA liquid | 3.5 parts |
| Photopolymerization initiator 1 | 0.18 parts |
| Sensitizer 1 | 0.18 parts |
| Hydroquinone monomethyl ether | 0.002 parts |
| Surfactant 1 | 0.036 parts |

Each component listed in Table 2 will next be described in detail.

K Pigment dispersion 1

Carbon black: 13.1 parts (Nipex 35, product of Degussa Japan Co.)
Dispersing agent (compound 1): 0.65 parts
Polymer (benzyl methacrylate/methacrylic acid (= 72/28 [mole ratio]) random copolymer, molecular weight: 37,000): 6.72 parts
Propylene glycol monomethyl ether acetate: 79.53 parts

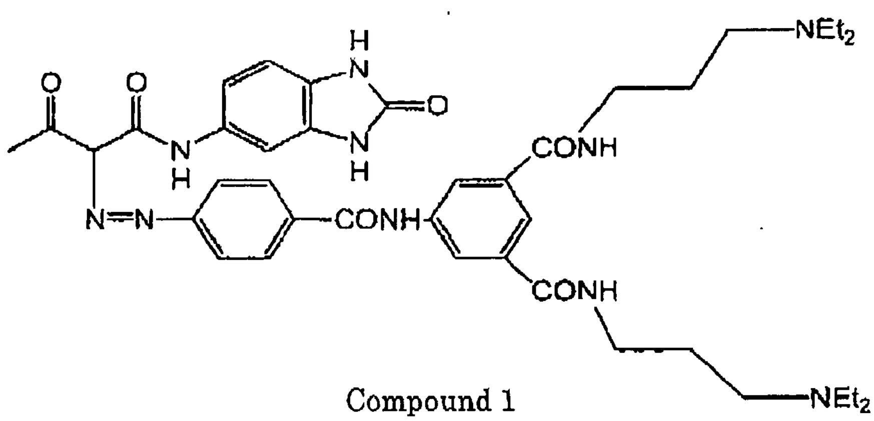

Compound 1

Binder 1

Polymer (benzyl methacrylate/methacrylic acid (= 78/22 [mole ratio]) random copolymer, molecular weight: 38,000): 27 parts
Propylene glycol monomethyl ether acetate: 73 parts

DPHA liquid

Dipentaerythritol hexaacrylate: 76 parts (containing polymerization inhibitor MEHQ (500 ppm), KAYARAD DPHA (trade name), product of NIPPON KAYAKU CO., Ltd.)
Propylene glycol monomethyl ether acetate: 24 parts

Photopolymerization initiator 1

CGI325 (trade name) (product of Ciba Specialty Chemicals Inc.) (compound 2)

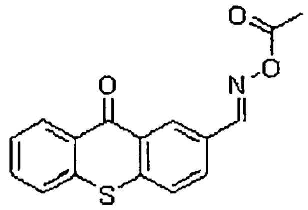

Compound 2

Sensitizer 1

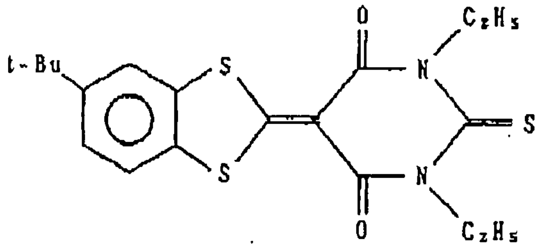

No. 4

Surfactant 1

Compound having the following Structural Formula 1: 30 parts
Methyl ethyl ketone: 70 parts

Structural Formula 1

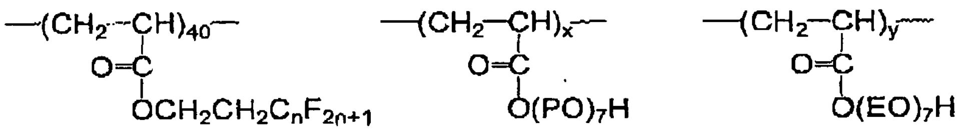

(n = 6、 x = 55、 y = 5、 Mw = 33940、 Mw/Mn = 2.55
(PO: Propylene oxide, EO: Ethylene oxide)

< Formation of black matrix >

A 1,900 mm × 2,100 mm alkali-free glass substrate (hereinafter referred to as a "glass substrate") was washed with a UV washing apparatus, was washed with a detergent and a brush, and was washed through ultrasonication using ultrapure water. The thus-washed glass substrate was thermally treated at 120°C for 3 min to stabilize its surface conditions. Thereafter, the glass substrate was cooled to 23°C, and slit coating was performed thereon using a slit coating apparatus having a rotatable roller-type pre-ejection device and having a slit head with a slit space (i.e., the width of the slit head's ejection hole as viewed from the front) of 50 μm and an application width (i.e., the length of the slit head's ejection hole) of 1,880 mm. Here, this slit coating apparatus was fabricated so as to have the same configuration as a coater described in JP-A No. 2001-310147 except the slit space and the slit head.

First, the slit head was moved above the rotatable roller-type pre-ejection device and located at a height of 200 μm from the rotatable roller, Subsequently, a coating liquid (the above-prepared photosensitive composition K1) was fed by a pump and then applied on the rotatable roller for 4 sec for pre-ejection, to thereby stabilize ejection of the coating

liquid from the ejection hole.

Operation of the pump was stopped and then the slit head was rapidly made to approach one short side of the rectangular glass substrate (width: 1,900 mm, length: 2,100 mm, thickness: 0.7 mm). While the clearance (i.e., the distance between the substrate and the ejection hole of the slit head) was being maintained to be 200 μm, the coating liquid was fed again by the pump to resume coating, and the head was moved at 150 mm/sec. At the time when the slit head approached the other short side of the substrate, operation of the pump was stopped and the slit head was moved again above the rotatable roller-type pro-ejection device for the subsequent coating. The thus-coated glass substrate was dried under reduced pressure using a vacuum drying apparatus, followed by pre-baking on a hot plate at 90°C for 60 sec, to thereby form a black (K)-colored layer. The film thickness was found to be 2.3 μm.

The thus-obtained K-colored layer-formed glass substrate was evaluated for uneven coating by the below-described method.

Using an ultra-high pressure mercury lamp-containing proximity exposing apparatus (product of Hitachi High-Tech Electronics Engineering Co. Ltd.), the K-colored layer was patternwise exposed at an exposure dose of 100 $mJ/cm^2$ in a state where the glass substrate and a mask (quartz exposure mask having an image pattern) were disposed so as to be perpendicular to the horizontal surface. In this exposure, the distance between the mask surface and the K-colored layer was adjusted to 200 μm.

The mask is for use in formation of a black matrix with a line width of 30 μm, and has striped openings each having a size of 100 μm × 300 μm.

Next, the exposed K-colored layer was shower-developed at 30°C and a flat nozzle pressure of 0.04 MPa for 50 sec using a triethanol amine-based developer (containing triethanol amine (30% by mass); which was prepared by 12-fold diluting TPD2 (trade name) (product of FUJIFILM Corporation) with pure water (by mixing TPD2 (1 part) with pure water (11 parts)) to remove a thermoplastic resin layer and an intermediate layer. Subsequently, the glass substrate surface was subjected to air blow to remove the remaining liquid and then was showered with pure water for 10 sec for washing. Again, the resultant glass substrate was subjected to air blow to reduce liquid pools.

Thereafter, the photosensitive resin layer was shower-developed at 29°C and a cone-shape nozzle pressure of 0.15 MPa for 30 sec using an Na carbonate-based developer (sodium hydrogencarbonate (0.38 mol/L); which was prepared by 5-fold diluting T-CD1 (trade name) (product of FUJIFILM Corporation) with pure water), to thereby form a patterned image.

Furthermore, the thus-formed patterned image was showered at 33°C and a cone-shape nozzle pressure of 0.02 MPa for 20 sec with a diluted detergent (containing a phosphate, a silicate, a nonionic surfactant, a defoamer and a stabilizer; T-SD1 (trade name) (product of FUJIFILM Corporation), which was 10-fold diluted with pure water). And, the thus-treated patterned image was rubbed by a rotary brush having nylon fibers to remove residues, whereby a black matrix was obtained. Notably, T-SD2 (product of FUJIFILM Corporation) can be used instead of T-SD1 used above as a detergent.

Thereafter, using no mask, both surfaces of the glass substrate was post-exposed at an exposure dose of 500 $mJ/cm^2$ with the exposing apparatus used in the above exposure, followed by thermal treatment at 220°C for 15 min, to thereby form a black matrix. The black matrix-formed substrate was evaluated for development latitude by the below-described method.

< Formation of red (R), green (G) blue (B) pixels >

The black matrix-formed glass substrate was cooled, and then the following R, G and B photosensitive compositions were applied thereon in a manner similar to that employed for forming the K-colored layer, to thereby form R-, G- and B-colored layers. The mask used has striped openings each having a size of 103 μm × 303 μm.

The thickness of a photosensitive resin layer R1 was 1.6 μm, and the coating amounts of C. I. Pigment Red 254 and C. I. Pigment Red 177 were 0.88 $g/m^2$ and 0.22 $g/m^2$, respectively.

The thickness of a photosensitive resin layer G1 was 1.6 μm, and the coating amounts of C. I. Pigment Green 36 and C. I. Pigment Yellow 150 were 1.12 $g/m^2$ and 0.48 $g/m^2$, respectively.

The thickness of a photosensitive resin layer B1 was 1.6 μm, and the coating amounts of C. I. Pigment Blue 15:6 and C. I. Pigment Violet 23 were 0.63 $g/m^2$ and 0.07 $g/m^2$, respectively.

Through the above procedure, a color filter (hereinafter may be referred as a "color filter substrate" was formed in which a black matrix (BM) and RGB pixels were formed on the glass substrate.

< Photosensitive compositions R1, G1 and B1 >

Table 3

| Photosensitive resin composition | R1 | G1 | B1 |
|---|---|---|---|
| R pigment dispersion 1 (C.I.P.R.254) | 44.0 parts | - | - |
| R pigment dispersion 2 (C.I.P.R.177) | 5.0 parts | - | - |
| G pigment dispersion **I** (C.I.P.G.36) | - | 24.0 parts | - |
| Y pigment dispersion 1 (C.I.P.Y.150) | - | 13.0 parts | - |
| B pigment dispersion 1 (C.I.P.B.15:6) | - | - | 8.0 parts |
| B pigment dispersion 2 (C.I.P.B.15:6 + C.I.P.V.23) | - | - | 14.0 parts |
| Propylene glycol monomethyl ether acetate | 7.6 parts | 29.0 parts | 28.0 parts |
| Methyl ethyl ketone | 37.0 parts | 26.0 parts | 26.0 parts |
| Cyclohexanone | - | 1.3 parts | - |
| Binder 1 | - | 2.5 parts | - |
| Binder 2 | 0.7 parts | - | - |
| Binder 3 | - | - | 9.0 parts |
| DPHA liquid | 3.8 parts | 3.5 parts | 4.2 parts |
| Photopolymerization initiator 1 | 0.18 parts | 0.15 parts | 0.17 parts |
| Sensitizer 1 | 0.18 parts | 0.15 parts | 0.17 parts |
| Phenothiazine | 0.01 parts | 0.005 parts | 0.02 parts |
| Surfactant 1 | 0.06 parts | 0.07 parts | 0.06 parts |

Next will be described each of the photosensitive compositions R1, G1 and B1 shown in Table 3.

< Preparation of photosensitive composition R1 >

R pigment dispersions 1 and 2 and propylene glycol monomethyl ether acetate were weighed respectively in corresponding amounts shown in Table 3, and were mixed under stirring with one another at 24°C (±2°C) and 150 r.p.m. for 10 min.

Subsequently, methyl ethyl ketone, binder 2, DPHA liquid, photopolymerization initiator 1, sensitizer 1 and phenothiazine were weighed respectively in corresponding amounts shown in Table 3, and were sequentially added to the resultant mixture at 24°C (±2°C) in this order, followed by stirring at 150 r.p.m. for 30 min. Thereafter, surfactant 1 was weighed in an amount shown in Table 2, and added to the resultant mixture at 24°C (±2°C), followed by stirring at 30 r.p.m. for 5 min. The thus-obtained mixture was filtrated with a nylon mesh (#200) to prepare photosensitive composition R1.

< Preparation of photosensitive composition G1 >

G pigment dispersion 1, Y pigment dispersion 1 and propylene glycol monomethyl ether acetate were weighed respectively in corresponding amounts shown in Table 3, and were mixed under stirring with one another at 24°C (±2°C) and 150 r.p.m. for 10 min.

Subsequently, methyl ethyl ketone, cyclohexanone, binder 1, DPHA liquid, photopolymerization initiator 1, sensitizer 1 and phenothiazine were weighed respectively in corresponding amounts shown in Table 3, and were sequentially added to the resultant mixture at 24°C (±2°C) in this order, followed by stirring at 150 r.p.m. for 30 min. Thereafter, surfactant 1 was weighed in an amount shown in Table 3, and was added to the resultant mixture at 24°C (±2°C), followed by stirring at 30 r.p.m. for 5 min. The thus-obtained mixture was filtrated with a nylon mesh (#200) to prepare photosensitive composition G1.

< Preparation of photosensitive composition B1 >

B pigment dispersions 1 and 2 and propylene glycol monomethyl ether acetate were weighed respectively in corresponding amounts shown in Table 3, were mixed under stirring at 24°C (±2°C) and 150 r.p.m. for 10 min.

Subsequently, methyl ethyl ketone, binder 3, DPHA liquid, photopolymerization initiator 1, sensitizer 1 and phenothiazine were weighed respectively in corresponding amounts shown in Table 3, and were added to the resultant mixture at 25°C (±2°C) in this order, followed by stirring at 40°C (±2°C) and 150 r.p.m. for 30 min. Thereafter, surfactant 1 was weighed in an amount shown in Table 3, and was added to the resultant mixture at 24°C (±2°C), followed by stirring at 30 r.p.m. for 5 min. The thus-obtained mixture was filtrated with a nylon mesh (#200) to prepare photosensitive composition B1.

Each component listed in Table 3 will next be described below in detail.

R pigment dispersion 1

C.I. Pigment Red 254 (Irgaphor Red B-CF (trade name), product of Ciba

Specialty Chemicals Inc.): 8.0 parts

Compound 1 (dispersing agent): 0.8 parts
Polymer (benzyl methacrylate/methacrylic acid (= 72/28 [mole ratio]) random copolymer, molecular weight: 30,000): 8 parts
Propylene glycol monomethyl ether acetate: 83 parts

R pigment dispersion 2

C.I. Pigment Red 177 (Cromophtal Red A2B (trade name), product of Ciba Specialty Chemicals Inc.): 18 parts
Polymer (benzyl methacrylatelmethacrylic acid (= 72/28 [mole ratio]) random copolymer (mass average molecular weight: 37,000)): 12 parts
Propylene glycol monomethyl ether acetate: 70 parts

G pigment dispersion 1

GT-2 (trade name) (product of FUJIFILM Electronic Materials Co., Ltd.) Y pigment dispersion 1
CF yellow EX3393 (trade name) (product of Mikuni Color Ltd.) B pigment dispersion 1
CF blue EX3357 (trade name) (product of Mikuni Color Ltd.) B pigment dispersion 2
CF blue EX3383 (trade name) (product of Mikuni Color Ltd.) Binder 1: as described above

Binder 2

Polymer (benzyl methacrylate/mathacrylic acid/methyl methacrylate (= 38/25/37 [mole ratio]) random copolymer, molecular weight: 40,000): 27 parts

Propylene glycol monomethyl ether acetate: 73 parts

Binder 3

Polymer (benzyl methacrylatelmathacrylic acid/methyl methacrylate (= 36/22/42 [mole ratio]) random copolymer (mass average molecular weight: 38,000)): 27 parts
Propylene glycol monomethyl ether acetate: 73 parts
DPHA liquid: as described above
Photopolymerization initiator 1: as described above
Sensitizer 1: as described above
Surfactant 1: as described above

An overcoat layer was provided on the R, G and B pixels and the black matrix of the above-formed color filter substrate, followed by forming of a transparent electrode of ITO (Indium Tin Oxide) through sputtering.

< Formation of photospacer >

A photospacer was formed on this ITO electrode-laid color filter substrate in a manner similar to that employed

for forming the K-colored layer, except that the photosensitive composition K1 was changed to a photospacer forming resin composition having the following composition. The resin layer dried was found to have a thickness of 4.1 μm.

The composition of the photospacer forming resin composition is as follows.

1-Methoxy-2-propyl acetate: 452 parts
Methyl ethyl ketone: 327 parts
Methanol: 0.035 parts
Binder 4:101 parts
(methacrylic acid/allyl methacrylate copolymer (= 20/80 [mole ratio]), mass average molecular weight: 36,000; high-molecular-weight compound)
DPHA liquid (as described above): 99 parts 2,4-Bis(trichloroethyl)-6-[4'(N,N-bisethoxycarbonylmethyl)amino-3'-bromophenyl]-s -triazine: 2.5 parts
Hydroquinone monomethyl ether: 0.039 parts
Surfactant 1 (as described above): 0.86 parts
Decoloring agent: 17 parts
(VICTRIA PURE BLUE BOH-M (trade name), product of HODOGAYA CHEMICAL CO., LTD.)

< Fabrication of liquid crystal display device >

Polyimide was applied on the spacer-provided color filter substrate, followed by rubbing, to thereby form an oriented film. Subsequently, a liquid crystal material was sandwiched between this color filter substrate and a drive-side substrate to fabricate a liquid crystal display element. Specifically, first, a TFT substrate for IPS having TFTs and comb-shaped pixel electrodes (conductive layer) was provided as the drive-side substrate. The TFT substrate and the color filter substrate were disposed so that a TFT substrate's surface having the pixel electrodes, etc. faced a color filter's surface having the oriented film, and were fixed the spacer's gap apart. The liquid crystal material was charged into the gap to form a liquid crystal layer responsible for image display, whereby a liquid crystal cell was obtained. Both surfaces of the thus-obtained liquid crystal cell were provided with polarizing plates HLC2-2518 (product of SANRITZ Co.). Next, a cold-cathode tube backlight was assembled and disposed on the back surface of the liquid crystal cell having the polarizing plates, whereby a liquid crystal display device was obtained,

Notably, in this Example, a liquid crystal display device for an IPS mode was described in detail, but the present invention is not limited thereto. Similar effects can be obtained even when the present invention is applied to other liquid crystal display modes such as AFFS. The same applies to Examples 30 to 34 given below.

(Example 30)

The procedure of Example 29 was repeated, except that photosensitive composition K1 was changed to photosensitive composition K2 (solid matter concentration: 6.7% by mass), to thereby form RGB pixels and a spacer as well as fabricate a liquid crystal display device.

(Example 31)

The procedure of Example 29 was repeated, except that photosensitive composition K1 was changed to photosensitive composition K3 (solid matter concentration: 11.6% by mass), to thereby form RGB pixels and a spacer as well as fabricate a liquid crystal display device.

(Example 32)

The procedure of Example 29 was repeated, except that photosensitive composition K1 was changed to photosensitive composition K4 (solid matter concentration: 4.2% by mass), to thereby form RGB pixels and a spacer as well as fabricate a liquid crystal display device.

(Example 33)

The procedure of Example 29 was repeated, except that photosensitive composition K1 was changed to photosensitive composition K5 (solid matter concentration: 14.1% by mass), to thereby form RGB pixels and a spacer as well as fabricate a liquid crystal display device.

(Example 34)

There was prepared a photosensitive composition which was the same as photosensitive composition K1 except that no sensitizer was contained. The procedure of Example 29 was repeated, except that the thus-prepared photosensitive composition was used, to thereby form RGB pixels and a spacer as well as fabricate a liquid crystal display device.

(Comparative Example 4)

The procedure of Example 29 was repeated, except that photosensitive composition K1 was changed to photosensitive composition K6 (solid matter concentration: 2.4% by mass), to thereby form RGB pixels and a spacer as well as fabricate a liquid crystal display device.

(Comparative Example 5)

The procedure of Example 29 was repeated, except that photosensitive composition K1 was changed to photosensitive composition K7 (solid matter concentration: 15.3% by mass), to thereby form RGB pixels and a spacer as well as fabricate a liquid crystal display device.

(Comparative Example 6)

The procedure of Comparative Example 5 was repeated, except that the glass substrate was changed to that having a size of 1,000 mm × 900 mm, to thereby form RGB pixels and a spacer as well as fabricate a liquid crystal display device.

The components/amounts of photosensitive compositions K1 to K7 are collectively shown in Table 4.

Table 4

| Photosensitive resin composition | K1 | K2 | K3 | K4 | K5 | K6 | K7 |
|---|---|---|---|---|---|---|---|
| K pigment dispersion 1 (carbon black) | 19 parts | 14 parts | 24 parts | 8.7 parts | 29 parts | 5 parts | 32 parts |
| Propylene glycol monomethyl ether acetate | 15 parts | 21 parts | 7.8 parts | 28 parts | 0.79 parts | 34 parts | - |
| Methyl ethyl ketone | 54 parts | 56 parts | 58 parts | 57 parts | 51 parts | 58 parts | 53 parts |
| Binder 1 | 8.1 parts | 6 parts | 10 parts | 3.8 parts | 13 parts | 2.2 parts | 14 parts |
| DPHA liquid | 3.5 parts | 2.6 parts | 4.5 parts | 1.6 parts | 5.4 parts | 0.93 parts | 5.9 parts |
| Photopolymerization initiator 1 | 0.18 parts | 0.13 parts | 0.23 parts | 0.084 parte | 0.28 parts | 0.048 parts | 0.31 parts |
| Sensitizer 1 | 0.18 parts | 0.13 parts | 0.23 parts | 0.084 parts | 0.28 parts | 0.048 parts | 0.31 parts |
| Hydroquinone monomethyl ether | 0.002 parts | 0.002 parts | 0.003 parts | 0.001 parts | 0.003 parts | 0.001 parts | 0.004 parts |
| Surfactant 1 | 0.036 parts | 0.027 parts | 0.046 parts | 0.017 parts | 0.056 parts | 0.01 parts | 0.061 parts |

Each of the black matrixes, color filters and liquid crystal display devices of Examples and Comparative Examples was subjected to measurement and evaluation as follows. The results are shown in Table 5.

< Calculation method for solid matter concentration >

One gram of the coating liquid (photosensitive composition) is weighed and mixed with 4 g of sea sand. The resultant mixture is dried in a vacuum oven under reduced pressure at 150°C for 2 hours. The mass of the dried product

is measured, and the mass (4 g) of the sea sand is subtracted from the measured mass to determine the solid content by mass of the coating liquid. The solid content is divided by the initial mass (1 g) of the coating liquid and then is multiplied by 100 to determine the solid matter concentration.

< Uneven coating >

Randomly selected 100 points in each K-colored layer-formed glass substrate were measured for optical density using a Macbeth densitometer. The difference (ΔOD) between the highest and lowest densities was calculated and evaluated according to the following evaluation criteria:

$\Delta OD \leq 0.05$: A,
$0.06 < \Delta OD \leq 0.10$: B,
$0.10 < \Delta OD \leq 0.30$: C, and
$0.30 < \Delta OD$: D.

Note that the K-colored layer-formed glass substrates evaluated as "A," "B" or "C" are regarded practically applicable.

< Development latitude >

Under a metal microscope VANOX AHMT3 (product of OLYMPUS CORPORATION) containing OLYMPUS Ikegami ITC-370M (product of OLYMPUS CORPORATION), randomly selected 100 points in each black matrix substrate were directly observed and measured for line width with FLOVEL VIDEO MICRO METER MODEL VM-40 (product of OLYMPUS CORPORATION). The difference (d) between the largest and smallest line widths was calculated and evaluated according to the following evaluation criteria:

$d \leq 1,5\ \mu m$: A,
$1.5\ \mu m < d \leq 2.0\ \mu m$: B,
$2.0\ \mu m < d \leq 2.5\ \mu m$: C, and
$2.5\ \mu m < d$: D.

Notably, the black matrix substrates evaluated as "A," "B" and "C" are regarded practically applicable. Also, "latitude" refers to a range in which light and dark of an object can be reproduced as a contrasting density.

[Uneven display performance]

Each liquid crystal display device was visually observed for presence or absence of display unevenness when being input by gray test signal, and was evaluated according to the following evaluation criteria. The liquid crystal display device evaluated as A or B is regarded as being practically applicable.

[Evaluation criteria]

A: No display unevenness observed
B: Display unevenness slightly observed
C: Display unevenness considerably observed

Table 5

| | Photosensitive resin composition | Conc. of solid matter (% by mass) | Size of glass (mm) | Uneven coating | Development latitude | Uneven display performance |
|---|---|---|---|---|---|---|
| Ex.29 | K1 | 9.0 | 1,900×2,100 | A | A | A |
| Ex.30 | K2 | 6.7 | 1,900×2,100 | B | B | A |
| Ex.31 | K3 | 11.6 | 1,900×2,100 | B | B | A |

(continued)

| | Photosensitive resin composition | Conc. of solid matter (% by mass) | Size of glass (mm) | Uneven coating | Development latitude | Uneven display performance |
|---|---|---|---|---|---|---|
| Ex.32 | K4 | 4.2 | 1,900×2,100 | C | C | B |
| Ex.33 | K5 | 14.1 | 1,900×2,100 | C | C | B |
| Ex.34 | ± | 9.0 | 1,900×2,100 | B | C | B |
| Comp.Ex.1 | K6 | 2.4 | 1,900×2,100 | D | D | C |
| Comp.Ex.2 | K7 | 15.3 | 1,900×2,100 | D | D | C |
| Comp.Ex.2 | K7 | 15.3 | 1,000×900 | B | B | B |
| *: photosensitive resin composition which is the same as K1 except that no sensitizer is contained | | | | | | |

As is clear from Table 5, in Examples 29 to 34, uneven coating could be remarkably reduced and high-quality image display could be observed without uneven display. Also, as an unexpected effect, the line width of the black matrix was found to be stable against development; i.e., the development latitude was considerably widened.

Further, it was found that the solid matter concentration of the photosensitive composition was preferably 2.5% by mass to 15% by mass, more preferably 5% by mass to 12.5% by mass, most preferably 7.5% by mass to 10% by mass.

The liquid crystal display device of Example 29, being formed from the composition containing the sensitizer, was found to be superior to that of Example 34 being formed from the composition containing no sensitizer.

In contrast, when the solid matter concentration of the photosensitive composition was lower than 2.5% by mass (Comparative Example 4) or greater than 15% by mass (Comparative Example 5), uneven coating, development latitude and uneven display were found to be poor.

In Comparative Example 6, the substrate used therein had a smaller size and thus, uneven coating and development latitude were found to be non-problematic, while uneven display was found to occur.

Industrial Applicability

The oxime compound of the present invention is excellent in storage stability and sensitivity and thus, is contained in a polymerizable composition to enable it to be excellent in storage stability without causing polymerization reaction during storage and to be a highly sensitive polymerizable composition by generating active radicals upon irradiation of energy beam, especially, light, to thereby efficiently and quickly allow the polymerizable compounds to be polymerized

The oxime compound of the present invention can be used as a radical generator in printing inks, clear finish materials, powder coating materials, marking materials for roads and buildings, photographic reproduction techniques, holographic recording materials, image recording techniques, production of printing plates which can be developed with organic solvents or with aqueous alkali, daylight-curable coating materials for production of masks for screen printing, dental filling compositions, adhesives, pressure-sensitive adhesives, laminating resins, liquid etching resists (film), solder resists, electroplating resists, permanent resists, and photostructurable dielectric for printed circuit boards and/or electronic circuits. Also, the oxime compound of the present invention can be used in various materials applied to display, constituent materials in the production process of plasma-display panels and electroluminescence displays, color filters, optical switches, optical lattices (interference lattices), materials for optical circuits, materials for production of three-dimensional articles by mass curing (UV curing in transparent molds) or by the stereolithographic technique, composite materials (e.g., styrene polyesters), other thick-layered compositions, resists for coating or sealing electronic components and integrated circuits, materials for optical fibers, and coating materials for production of optical lenses. Further, the oxime compound of the present invention can be used in production of medical devices, medical aids, implants, gels with thermotropic properties, and used, as an initiator, in emulsion polymerization, pearl polymerization and suspension polymerization. Furthermore, the oxime compound of the present invention can be used as a polymerization initiator for orderly arranging liquid crystal monomers or oligomers, and used as an initiator for binding dyes to organic materials.

Also, the photosensitive composition according to a first embodiment of the present invention is remarkably excellent in storage stability and sensitivity, allows easy handling, and enables a polymerizable compound to be efficiently polymerized in short time and thus, can be used in printing inks, clear finish materials, powder coating materials, marking materials for roads and buildings, photographic reproduction techniques, holographic recording materials, image recording techniques, production of printing plates which can be developed with organic solvents or with aqueous alkali, daylight-curable coating materials for production of masks for screen printing, dental filling compositions, adhesives,

pressure-sensitive adhesives, laminating resins, liquid etching resists (film), solder resists, electroplating resists, permanent resists, and photostructurable dielectric for printed circuit boards and/or electronic circuits. Also, the photosensitive composition of the present invention can be used in various materials applied to display, constituent materials in the production process of plasma-display panels and electroluminescence displays, color filters, optical switches, optical lattices (interference lattices), materials for optical circuits, materials for production of three-dimensional articles by mass curing (UV curing in transparent molds) or by the stereolithographic technique, composite materials (e.g., styrene polyesters), other thick-layered compositions, resists for coating or sealing electronic components and integrated circuits, materials for optical fibers, and coating materials for production of optical lenses. Further, the photosensitive composition of the present invention can be used in production of medical devices, medical aids, implants, gels with thermotropic properties.

Further, the photosensitive composition according to a second embodiment of the present invention has a wide development latitude, can be suitably used as a coating liquid for slit coating attaining easy coating on a large substrate, is excellent in coating properties such as uniformity of a coated product, and can be suitably used for producing a color filter.

The production method of the present invention for a color filter is suitably used for producing the color filter of the present invention The color filter of the present invention can be suitably used in the liquid crystal display element of the present invention. The liquid crystal display element can be suitably used in LCD devices.

## Claims

1. An oxime compound represented by General Formula (1):

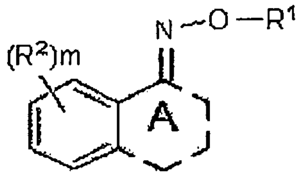

General Formula (1)

in General Formula (1), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group; m is an integer of 0 to 4; when m is an integer of 2 or more, $R^2$s may be linked together to form a ring; and A represents a 4-, 5-, 6- or 7-membered ring.

2. The oxime compound according to claim 1, wherein the oxime compound is represented by General Formula (2):

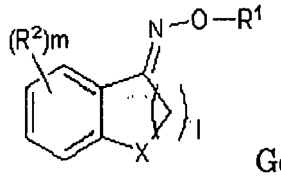

General Formula (2)

in General Formula (2), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group; m is an integer of 0 to 4; X represents $CH_2$, O or S; and 1 is an integer of 1 to 3,

3. The oxime compound according to claim 1, wherein the oxime compound is represented by General Formulas (3) or (4):

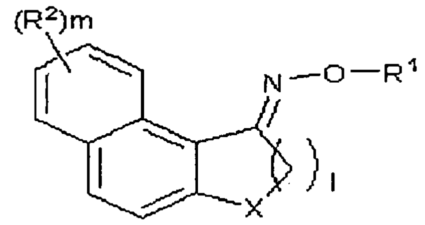

General Formula (3)

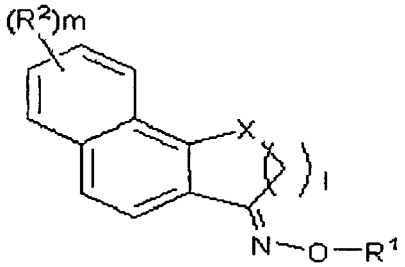

General Formula (4)

in General Formula (3) or (4), $R^1$ represents a hydrogen atom, an acyl group, an alkoxycarbonyl group or an aryloxylcarbonyl group, each of which may have a substituent; $R^2$ represents or $R^2$s each represent a halogen atom, an alkyl group, an aryl group, an alkyloxy group, an aryloxy group, an alkylthio group, an arylthio group or an amino group; m is an integer of 0 to 4; X represents $CH_2$, O or S; and 1 is an integer of 1 to 3.

**4.** The oxime compound according to any one of claims 2 and 3, wherein X is O or S, 1 is an integer of 1 or 2, and $R^1$ is an acyl group or an alkoxycarbonyl group, each of which may have a substituent.

**5.** A photosensitive composition comprising:

at least one ethylenically unsaturated photopolymerizable compound, and
at least one of the oxime compounds according to any one of claims 1 to 4, each serving as a photopolymerization initiator.

**6.** A photosensitive composition comprising:

(A) a photopolymerization initiator,
(B) an ethylenically unsaturated compound,
(C) a binder,
(D) a coloring material, and
(E) a solvent,

wherein the photopolymerization initiator is an oxime compound represented by General Formula (A-1), and wherein the concentration of solid matter contained in the photosensitive composition is 2.5% by mass to 15% by mass:

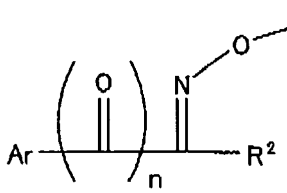

General Formula (A-1)

in General Formula (A-1), $R^1$ represents an acyl group, an alkyloxycarbonyl group or an aryloxylcarbonyl group; $R^2$ represents a hydrogen atom, an alkyl group or a cyano group; Ar represents an aromatic or heteroaromatic ring; Ar and $R^2$ may be linked together to form a ring; and n is an integer of 0 or 1.

**7.** A photosensitive composition comprising

(A) a photopolymerization initiator,
(B) an ethylenically unsaturated compound,
(C) a binder,
(D) a coloring material, and
(E) a solvent,

wherein the photopolymerization initiator is the oxime compound according to any one of claims 1 to 4, and wherein the concentration of solid matter contained in the photosensitive composition is 2.5% by mass to 15% by mass.

**8.** The photosensitive composition according to claim 6, wherein the oxime compound represented by General Formula (A-1) is a compound represented by

General Formula (A-2):

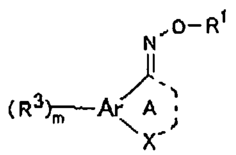

General Formula (A-2)

in General Formula (A-2), $R^1$ represents an acyl group, an alkyloxycarbonyl group or an aryloxylcarbonyl group, each of which may have a substituent; $R^3$ represents an alkyl group, an alkyloxy group, an aryloxy group, an alkylthio group, an arylthio group, a halogen atom, an alkyloxycarbonyl group, a nitro group or an acylamino group; m is an integer of 0 or more; when m is an integer of 2 or more, $R^3$s may be identical or different and may be linked together to form a ring; Ar represents an aromatic or heteroaromatic ring; A represents a 4-, 5- or 6-membered ring, each of which may have a substituent; and X represents an oxygen atom or a sulfur atom.

**9.** The photosensitive composition according to claim 8, wherein the oxime compound represented by General Formula (A-2) is a compound represented by General Formula (A-3) or (A-4):

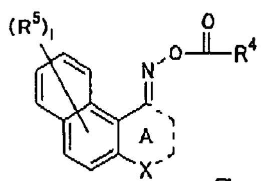

General Formula (A-3)

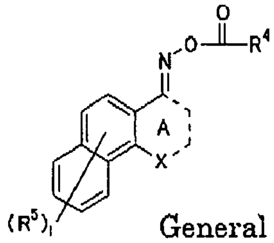

General Formula (A-4)

in General Formula (A-3) or (A-4), $R^4$ represents an alkyl group or an alkyloxy group, each of which may have a substituent; $R^5$ represents an alkyl group, an alkyloxy group, an aryloxy group, an alkylthio group, an arylthio group, a halogen atom, an alkyloxycarbonyl group, a nitro group or an acylamino group; l s an integer of 0 to 6; when 1 is an integer of 2 or more, $R^6$s may be identical or different and may be linked together to form a ring; A represents a 5- or 6-membered ring, each of which may have a substituent; and X represents an oxygen atom or a sulfur atom.

**10.** The photosensitive composition according to any one of claims 6 to 9, further comprising a sensitizer represented by General Formula (F-1):

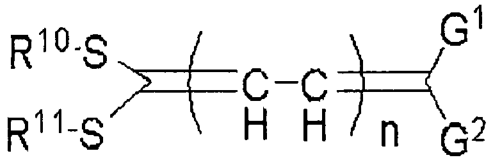

General Formula (F-1)

in General Formula (F-1), $R^{10}$ and $R^{11}$, which may be identical or different, each represent an alkyl group, an aryl group or an alkenyl group, each of which may have a substituent; $R^{10}$ and $R^{11}$ may form, together with sulfur atoms to which $R^{10}$ and $R^{11}$ are bonded, a ring composed of non-metal elements; n is an integer of 0, 1 or 2; $G^1$ and $G^2$, which may be identical or different, each represent an alkoxycarbonyl group, an aryloxylcarbonyl group, an acyl group, an arylcarbonyl group, an alkylthio group, an arylthio group, an alkylsulfonyl group, an arylsulfonyl group, a fluoroalkylsulfonyl group, each of which may have a substituent, or each represent a hydrogen atom or a cyano group; one of $G^1$ and $G^2$ is an alkoxycarbonyl group, an aryloxylcarbonyl group, an acyl group, an arylcarbonyl group, an alkylsulfonyl group, an arylsulfonyl group, a fluoroalkylsulfonyl group, each of which may have a substituent, or is a cyano group; and $G^1$ and $G^2$ may form, together with carbon atoms to which $G^1$ and $G^2$ are bonded, a ring composed of non-metal atoms.

**11.** The photosensitive composition according to claim 10, wherein the sensitizer represented by General Formula (F-1) is a compound represented by General Formula (F-2):

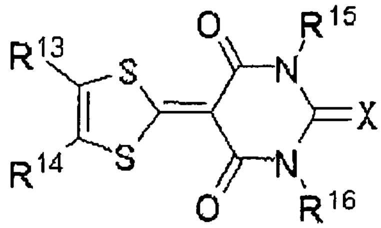

General Formula (F-2)

in General Formula (F-2), $R^{13}$ and $R^{14}$, which may be identical or different, each represent a hydrogen atom, an alkyl group, an aryl group, an acyl group or a halogen atom; $R^{13}$ and $R^{14}$ may be linked together to form an aromatic ring which may have an alkyl group, an alkyloxy group or a halogen atom; $R^{15}$ and $R^{16}$, which may be identical or different, each represent a hydrogen atom, an alkyl group or an aryl group; and X represents an oxygen atom or a sulfur atom.

**12.** A production method for a color filter, the method comprising:

forming a photosensitive layer by applying the photosensitive composition according to any one of claims 6 to 11 onto a substrate and by drying the applied photosensitive composition,
patternwise exposing and curing the photosensitive layer so as to have a desired pattern, and
developing the exposed photosensitive layer to remove uncured portions.

**13.** The production method according to claim 12, wherein the patternwise exposing and curing is forming a two-dimensional image through scanning of the photosensitive layer by moving light having a wavelength of 350 nm to 420 nm relatively to the photosensitive layer while modifying the light based on image data, using two-dimensionally arranged spatial light modulation devices.

**14.** The production method according to any one of claims 12 and 13, wherein the substrate has a size of 1 $m^2$ or greater.

**15.** A color filter obtained by the production method according to any one of claims 12 to 14.

**16.** A liquid crystal display element comprising:

the color filter according to claim 15 as at least one member.

**INTERNATIONAL SEARCH REPORT**

International application No.
PCT/JP2007/065103

A. CLASSIFICATION OF SUBJECT MATTER
*C07C251/66*(2006.01)i, *C07D307/82*(2006.01)i, *C07D307/92*(2006.01)i, *C07D311/92*(2006.01)i, *C07D333/66*(2006.01)i, *C07D335/06*(2006.01)i, *G03F7/031*(2006.01)i

According to International Patent Classification (IPC) or to both national classification and IPC

B. FIELDS SEARCHED

Minimum documentation searched (classification system followed by classification symbols)
C07C251/66, C07D307/82, C07D307/92, C07D311/92, C07D333/66, C07D335/06, G03F7/031

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | US 3287368 A (McNeil Laboratories, Inc.),<br>22 November, 1966 (22.11.66),<br>Claims; examples I, II, VI<br>(Family: none) | 1,2<br>5,7-9 |
| X<br>A | WO 2005/082838 A1 (PPG-SIPSY),<br>09 September, 2005 (09.09.05),<br>Claims; pages 10 to 11; example 1<br>& EP 1574498 A1 | 1,2<br>5,7-9 |
| X<br>A | WO 1996/036596 A1 (CHIROSCIENCE LTD.),<br>21 November, 1996 (21.11.96),<br>Claims; Intermediate 3, 4, 5<br>& AU 9657723 A | 1,2<br>5,7-9 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 September, 2007 (12.09.07) | 06 November, 2007 (06.11.07) |
| Name and mailing address of the ISA/<br>Japanese Patent Office<br><br>Facsimile No. | Authorized officer<br><br>Telephone No. |

Form PCT/ISA/210 (second sheet) (April 2005)

**INTERNATIONAL SEARCH REPORT**

International application No. PCT/JP2007/065103

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 9-48770 A (Sankyo Co., Ltd.),<br>18 February, 1997 (18.02.97),<br>Claims; referential examples<br>& WO 1996/038427 A1 | 1,2<br>5,7-9 |
| X<br>A | JP 2003-514809 A (Bayer AG.),<br>22 April, 2003 (22.04.03),<br>Claims; Par. Nos. [0010] to [0012]; preparation examples 2, 9 to 13; tables 1, 2<br>& WO 2001/036405 A1 & US 6603025 B1<br>& DE 19954936 A1 | 1,2<br>5,7-9 |
| X<br>A | Scapini, G., Substances with antiviral activity. III. Strucures of monosubstituted derivatives of 1H-benzo[e]indene-1, 3-dione, Farmaco, Edizione Scientifica, 30, (7), 1975, p.568-580 | 1,3<br>5,7-9 |
| X<br>A | JP 7-70033 A (EGIS Gyogyszergyar Rt.),<br>14 March, 1995 (14.03.95),<br>Claims; examples<br>& US 5439940 A & EP 595364 A1 | 1,3<br>5,7-9 |
| X<br>A | WO 1992/019589 A2 (SHITHKLINE BEECHAM CORP.),<br>12 November, 1992 (12.11.92),<br>Claims; examples<br>& US 5140047 A | 1,3<br>5,7-9 |
| X<br>A | WO 2002/102798 A1 (Idemitsu Kosan Co., Ltd.),<br>27 December, 2002 (27.12.02),<br>Claims; examples<br>& JP 2005-22974 A | 1,2,4<br>5,7-9 |
| Y<br>A | JP 2002-323762 A (Nippon Kayaku Co., Ltd.),<br>08 November, 2002 (08.11.02),<br>Claims; Par. Nos. [0012] to [0041]<br>(Family: none) | 6,10-16<br>5,7-9 |
| Y<br>A | JP 2001-235858 A (Ciba Specialty Chemicals Holding Inc.),<br>31 August, 2001 (31.08.01),<br>Claims; Par. Nos. [0004] to [0244]<br>& US 2002/20832 A1 & DE 10061948 A1 | 6,10-16<br>5,7-9 |
| Y<br>A | JP 2005-319758 A (Fuji Photo Film Co., Ltd.),<br>17 November, 2005 (17.11.05),<br>Claims; Par. Nos. [0021] to [0163]<br>(Family: none) | 6,10-16<br>5,7-9 |
| Y<br>A | JP 3-164722 A (Fuji Photo Film Co., Ltd.),<br>16 July, 1991 (16.07.91),<br>Claims; synthesis examples<br>(Family: none) | 6,10-16<br>5,7-9 |

Form PCT/ISA/210 (continuation of second sheet) (April 2005)

**INTERNATIONAL SEARCH REPORT**

International application No.
PCT/JP2007/065103

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The inventions of claims 1-4 relate to an oxime compound represented by the general formula (1), (2), (3) or (4).

The invention of claim 6 relates to a photosensitive composition comprising an oxime compound represented by the general formula (A-1).

When $R^2$ represents a hydrogen atom, an alkyl group or a cyano group, when Ar represents a heterocyclic aromatic group, when Ar and $R^2$ do not together form a ring, and when n is 1 in the invention of claim 6, there is no relationship involving one or more of the same technical feature between the invention of claim 6 and the inventions of claim 1-4, and therefore these inventions do not comply with the requirement of unity of invention.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 60166306 A
- JP 2001233842 A
- JP 2000080068 A
- JP 2005182004 A
- JP 61243869 A
- JP 7050473 A
- JP 7017737 B
- JP 2006036750 A
- JP 2665696 B
- JP 2000194132 A
- JP 8179120 A
- JP 7294726 A
- WO 9001512 A
- EP 624826 A
- EP 12339 A
- EP 33896 A
- EP 41125 A
- DE 2936039
- EP 126541 A
- EP 022788 A
- US 4371606 A
- US 4371607 A
- US 4950581 A
- EP 780729 A
- EP 497531 A
- EP 441232 A
- EP 339841 A
- EP 438123 A
- GB 2180358 A
- JP 6068309 A
- JP 9179299 A
- JP 9325209 A
- JP 8305019 A
- EP 245639 A
- EP 445624 A
- US 5013768 A
- JP 10171119 A
- US 5853446 A
- EP 863534 A
- JP 9244230 A
- JP 10062980 A
- JP 8171863 A
- US 5840465 A
- EP 855731 A
- JP 5271576 A
- JP 5067405 A
- US 4575330 A
- DE 19700064
- EP 678534 A
- JP 5173320 A
- DE 2308830
- DE 4228514
- EP 636669 A
- EP 320264 A
- US 5368976 A
- JP 59044615 B
- JP 54034327 A
- JP 6812577 A
- JP 54025957 A
- JP 59053836 A
- JP 59071048 A
- JP 60159743 A
- JP 60258689 A
- JP 1152449 A
- JP 21999403 A
- JP 2199404 A
- US 5650263 A
- US 4139391 A
- JP 60258539 A
- JP 50034443 B
- JP 50034444 B
- US 5153095 A
- US 5677385 A
- US 5650233 A
- JP 10221843 A
- US 5879855 A
- US 5800952 A
- US 5882843 A
- US 5866298 A
- US 5863678 A
- JP 6230212 A
- JP 9269410 A
- JP 1090516 A
- US 5821016 A
- US 5847015 A
- US 6719008 B
- EP 881541 A
- EP 902327 A
- US 5626796 A
- JP 57042009 A
- JP 1130103 A
- JP 1134306 A
- JP 2000081701 A
- JP 11174459 A
- JP 11174464 A
- JP 60038989 A
- JP 60165623 A
- JP 61067003 A

- JP 2000039503 A
- WO 9938035 A
- JP 61163602 A
- JP 60072927 A
- JP 60166946 A
- EP 0932256 A2
- US 5969867 A
- EP 7086 A
- WO 9641240 A
- EP 706091 A
- EP 511403 A
- US 3579339 A
- US 4622286 A
- DE 4013358
- JP 48041708 B
- JP 50006034 B
- JP 51037193 A
- JP 48064183 A
- JP 49043191 B
- JP 52030490 B
- JP 2003131379 A
- JP 2003207787 A
- JP 11149008 A
- JP 2003302639 A
- JP 2003337424 A
- JP 2003177522 A
- JP 2003177523 A
- JP 2003177521 A
- JP 2003177519 A
- JP 2003177520 A
- JP 11133600 A
- JP 6016684 A
- JP 5072724 A
- US 2681294 A
- JP 2004089851 A
- JP 2004017043 A
- JP 2003170098 A
- JP 2003164787 A
- JP 2003010767 A
- JP 2002079163 A
- JP 2001310147 A
- JP 2000039033 A
- JP 5150175 A
- JP 2004523101 A
- WO 2002039793 A
- JP 2004056080 A
- JP 2002523905 A
- JP 2001255661 A
- JP 2003050469 A
- JP 2003156853 A
- JP 2005043576 A
- JP 2007041082 A
- JP 2004347831 A

**Non-patent literature cited in the description**


- **J. March.** Advanced Organic Chemistry. Wiley Interscience, 1992
- **S. R. Sandler ; W. Karo.** Organic functional group preparations. Academic Press, vol. 3
- Organic Syntheses. J. Wily & Sons, 1988, vol. VI, 199, 840
- Organic Synthesis. vol. V, 32, 373
- ORGANIC SYNTHESIS. vol. III, 191, 513
- ORGANIC SYNTHESIS. vol. II, 202, 204, 363
- **G. Buhr ; R. Dammel ; C. Lindley.** *Polym. Mater. Sci. Eng.,* 1989, vol. 61, 269
- **E. A, Bartmann.** *Synthesis,* 1993, vol. 5, 490
- **M. Wittig ; Th. Gohmann.** Radiation Curing of Powder Coating. *Conference Proceedings, Radtech Europe,* 1993
- **T. Kudo et al.** *J. Appl. Phys.,* 1998, vol. 37, 3594-3603
- **T. Kudo et al.** *Jpn. J. Appl. Phys.,* 1998, vol. 37, 3594
- **T. Kudo et al.** *J. Photopolym. Sci. Technol.,* 1996, vol. 9, 109
- **K. Kobayashi.** *Solid State Techno,* November 1992, S15-S18
- *Journal of the Research Group in Microoptics Japanese Society of Applied Physics, Colloquium in Optics,* 1987, vol. 5 (2), 118-123
- *JOURNAL OF THE RESEARCH GROUP IN MICROOPTICS JAPANESE SOCIETY OF APPLIED PHYSICS, COLLOQUIUM IN OPTICS,* 1988, vol. 6 (2), 87-92
- **Popovic et al.** *SPIE,* 1988, vol. 898, 23-25
- **K. -P. Mieck.** *T. Reussmann in Kunststoffe,* 1995, vol. 85, 366-370
- **P. H. Selden.** Glasfaserveretaerkte Kunststoffe. Springer Verlag, 1967, 610
- **A. Bertsch ; J. Y. Jezequel ; J. C. Andre.** *Journal of Photochemistry and Photobiology A: Chemistry,* 1997, vol. 107, 275-281
- **K. -P, Nicolay.** *Offset Printing,* 1997, vol. 6, 34-37
- *Journal of the Adhesion Society of Japan,* vol. 20 (7), 300-308
- **W. Elenbaas.** Light Sources. Philips Technical Library, 148-150
- Next-Generation Liquid Crystal Display Technology. Kogyo Chosakai Publishing Inc, 1994
- color TFT Liquid Crystal Displays. Kyoritsu Shuppan Co., Ltd, 1996
- **Kentaro Shima.** Market of Liquid Crystal Display-related Materials and Chemicals. CMC Publishing, 1994

- **Ryokichi Omote.** Current Status and Future Prospect of Liquid Crystal-related Market. Fuji Chimera Research Institute, Inc, 2003